# EUROPEAN PATENT APPLICATION

(11) **EP 4 397 758 A1**
(43) Date of publication of application: **10.07.2024**
(21) Application number: 22858494.2
(22) Date of filing: 17.08.2022
(51) Int. Cl.: C12N 15/11, A61K 48/00, A61P 35/00, C12N 15/63, C12Q 1/6897, G01N 33/15, G01N 33/50

(54) **NUCLEIC ACID CONSTRUCT CAPABLE OF MEASURING HOMOLOGOUS RECOMBINATION ACTIVITY AND UTILIZATION THEREOF**

(30) Priority: 18.08.2021 JP 2021133207
(71) Applicant: Public University Corporation Yokohama City University, Yokohama-shi, Kanagawa 236-0027 (JP)
(72) Inventor: ADACHI, Noritaka, Yokohama-shi, Kanagawa 236-0027 (JP)
(74) Representative: Mewburn Ellis LLP
(86) International application number: PCT/JP2022/031085
(87) International publication number: WO 2023/022176

(57) **Abstract**

Disclosed are a means which enables simple and rapid detection of the presence or absence, and of the degree, of HR activity in an individual, and a means which is useful for detection and treatment of homologous recombination-restored cancer for which no treatment means is available at present. The nucleic acid construct of the present invention is a nucleic acid construct comprising a set of a first nucleic acid molecule and a second nucleic acid molecule, the first nucleic acid molecule comprising: a promoter region; and a mutant gene sequence operably linked downstream thereof, the mutant gene sequence being constituted by a gene sequence encoding a protein and a cleavage site arranged inside thereof; the second nucleic acid molecule comprising a complementation region capable of replacing, by homologous recombination, a partial region comprising the cleavage site in the mutant gene sequence, the complementation region being constituted by a first homologous region and a second homologous region. The construct is useful as a measurement reagent or kit for homologous recombination activity, a diagnostic agent for homologous recombination-deficient cancer, a therapeutic agent for homologous recombination-restored cancer, or the like.

## Description

### TECHNICAL FIELD

The present invention relates to a nucleic acid construct capable of measuring homologous recombination activity, and use of the nucleic acid construct as a measurement reagent and measurement kit for homologous recombination activity, a detection agent for a homologous recombination-deficient cancer, and the like.

### BACKGROUND ART

Double-strand breaks of genomic DNA are dangerous DNA lesions with the highest mutagenicity. Therefore, homologous recombination (HR), which is capable of accurately repairing double-strand breaks, is indispensable for maintaining stability of the genome (Non-Patent Document 1). HR occurs only in the S to G2 phases, during which sister chromatids as templates are present, whereas, non-homologous end joining (NHEJ) functions as a double-strand break repair mechanism besides HR throughout the cell cycle (Non-Patent Document 2). In addition to these, there are minor repair routes such as alternative end-joining, in which PolQ is involved, and single-strand annealing, but, in any of these routes, induction of mutations inevitably occurs after the repair (Non-Patent Document 3).

HR is a highly complex reaction involving a number of proteins. In particular, RAD51, which is involved in strand exchange reaction, plays a central role (Non-Patent Document 4). Further, factors that assist the function of RAD51, such as BRCA1 and BRCA2, are indispensable for HR, and human cells deficient in BRCA1 or BRCA2 exhibit abnormality in the HR repair of double-strand breaks (Non-Patent Document 5).

HR deficiency causes various cancers. Hereditary breast ovarian cancer (HBOC) is the hereditary tumor that is most frequently found. Its main cause is a genetic abnormality of BRCA1 or BRCA2 (Non-Patent Documents 6 and 7). Also for sporadic (non-genetic) cancers, mutations in HR-related genes have been reported in various types of cancers (Non-Patent Documents 8 and 9).

Diagnosis of HR-deficient cancers is carried out by genetic diagnosis on BRCA1 and BRCA2 (Non-Patent Document 10). However, from the viewpoint of the fact that several ten kinds of proteins are involved in the HR reaction, the current genetic diagnosis cannot be considered to be a sufficient examination method. Based on the idea that detection of the HR activity itself is more desirable, several methods have been proposed therefor (Non-Patent Documents 11 and 12). However, all of these are methods for relative evaluation of the HR activity. It is conceivable that the most effective and reliable method is evaluation of the HR activities of the individual cells as absolute values rather than relative evaluation. However, such a method has not been developed.

For treatment of HR-deficient cancers, platinum formulations or poly(ADP-ribose) polymerase (PARP) inhibitors are used (Non-Patent Documents 13 and 14). In particular, the latter, PARP inhibitors, are attracting attention since they have a "synthetic lethal" relationship with HR. However, frequent occurrence of resistant cancers in PARP inhibitor treatment has been a serious problem, and there is no method for treatment of such resistant cancers at present (Non-Patent Documents 15 and 16).

### PRIOR ART DOCUMENT(S)

### NON-PATENT DOCUMENT(S)

Non-Patent Document 1: Moynahan ME and Jasin M. Nat Rev Mol Cell Biol. 2010 Mar; 11(3): 196-207.
Non-Patent Document 2: Chang HHY et al. Nat Rev Mol Cell Biol. 2017 Aug; 18(8): 495-506.
Non-Patent Document 3: Saito S et al. Nat Commun. 2017 Jul 11; 8: 16112
Non-Patent Document 4: Morrical SW. Cold Spring Harb Perspect Biol. 2015 Feb 2; 7(2): a016444.
Non-Patent Document 5: Prakash R et al. Cold Spring Harb Perspect Biol. 2015 Apr 1; 7(4): a016600.
Non-Patent Document 6: King MC et al. Science. 2003 Oct 24; 302(5645): 643-646.
Non-Patent Document 7: Cancer Genome Atlas Research Network. Nature. 2011 Jun 29; 474(7353): 609-615.
Non-Patent Document 8: Heeke AL et al. JCO Precis Oncol. 2018; 2018.
Non-Patent Document 9: Lord CJ and Ashworth A. Nat Rev Cancer. 2016 Feb; 16(2): 110-120.
Non-Patent Document 10: Gourley C et al. J Clin Oncol. 2019 Sep 1; 37(25): 2257-2269.
Non-Patent Document 11: Telli ML et al. Clin Cancer Res. 2016 Aug 1; 22(15): 3764-3773.
Non-Patent Document 12: Ransburgh DJ et al. Cancer Res. 2010 Feb 1; 70(3): 988-995.
Non-Patent Document 13: Bryant BE et al. Nature. 2005 Apr 14; 434(7035): 913-917.
Non-Patent Document 14: Farmer H et al. Nature. 2005 Apr 14; 434(7035): 917-921.
Non-Patent Document 15: Lord CJ and Ashworth A. Nat Med. 2013 Nov; 19(11): 1381-1388.
Non-Patent Document 16: Noordermeer SM and van Attikum H. Trends Cell Biol. 2019 Oct; 29(10): 820-834.
Non-Patent Document 17: Norquist B et al. J Clin Oncol. 2011 Aug 1; 29(22): 3008-3015.
Non-Patent Document 18: Bouwman P and Jonkers J. Clin Cancer Res. 2014 Feb 1; 20(3): 540-547.
Non-Patent Document 19: Pierce AJ et al. Genes Dev. 1999 Oct 15; 13(20): 2633-2638.
Non-Patent Document 20: Liang F et al. Proc Natl Acad Sci USA. 1996 Aug 20; 93(17): 8929-8933.

### SUMMARY OF THE INVENTION

### PROBLEMS TO BE SOLVED BY THE INVENTION

If there is a method that enables simple and rapid detection of the presence or absence, or the degree, of HR activity in cells, tissues, or individuals, the method is expected to be extremely useful for diagnosis and treatment of HR-deficient cancers including HBOC irrespective of the types of the cancers. Further, since restoration (reverse mutation) of BRCA1 is a cause of acquisition of PARP inhibitor resistance in BRCA1-deficient cancers (Non-Patent Documents 17 and 18), it would be a great advantage in the treatment of resistant cancers if detection and killing of cancer cells showing the restoration of HR activity become possible. However, at present, there is no known means of simply and rapidly detecting the presence or absence, or the degree, of the HR activity in an individual, or means of selectively detecting and killing cancer cells showing restoration of the HR activity.

An object of the present invention is to provide a means that enables simple and rapid detection of the presence or absence, or the degree, of the HR activity in an individual. Another object of the present invention is to provide a means that is useful for detection and treatment of homologous recombination-restored cancers for which no treatment means is available at present.

### MEANS FOR SOLVING THE PROBLEMS

As a result of intensive study, the present inventor developed a divided-type nucleic acid construct constituted by a set of two nucleic acid molecules as a nucleic acid construct having a structure designed such that a protein-encoding gene sequence is reconstructed in a cell having homologous recombination activity to cause expression of the protein while neither the reconstruction of the gene sequence nor the expression of the protein occurs in a cell whose homologous recombination activity is lost. The present inventor then discovered that the nucleic acid construct enables detection of the presence or absence of homologous recombination activity using a transient expression system in a very short time, and enables evaluation/measurement of homologous recombination activity as an absolute value rather than relative evaluation, and that, by preparing the nucleic acid construct of the present invention using a gene sequence encoding a protein having an action to decrease cell survival rate, such as a suicide gene, homologous recombination-restored cancer cells can be killed to allow production of a therapeutic effect, thereby completing the present invention that includes the following modes.

[1] A nucleic acid construct comprising the set of a first nucleic acid molecule and a second nucleic acid molecule,
   the first nucleic acid molecule comprising: a promoter region; and a mutant gene sequence placed downstream thereof, said mutant gene sequence being constituted by a gene sequence encoding a protein and a cleavage site arranged inside thereof;
   the second nucleic acid molecule comprising a complementation region capable of replacing, by homologous recombination, a partial region comprising the cleavage site in the mutant gene sequence, the complementation region being constituted by a first homologous region and a second homologous region and composed of a base sequence selected from the following (i) to (iii):
      (i) a continuous partial sequence in the gene sequence encoding the protein, the continuous partial sequence containing upstream and downstream regions adjacent to the cleavage site in the mutant gene sequence;
      (ii) a base sequence which is homologous to the partial sequence of (i) and encodes the same amino acid sequence as that encoded by the partial sequence; and
      (iii) a continuous partial sequence in a gene sequence encoding a protein whose sequence is 80% or more identical to that of the previously-mentioned protein and which has the same activity as the previously-mentioned protein, the continuous partial sequence being a base sequence homologous to the partial sequence of (i).
[2] The nucleic acid construct according to [1], wherein the mutant gene sequence contains a stop codon upstream of the cleavage site.
[3] The nucleic acid construct according to [1] or [2], wherein the base sequences of (ii) and (iii) are 90% or more homologous to the partial sequence of (i).
[4] The nucleic acid construct according to [1], wherein the second nucleic acid molecule comprises a continuous partial sequence in the gene sequence encoding the protein, the continuous partial sequence containing upstream and downstream regions adjacent to the cleavage site in the mutant gene sequence.
[5] The nucleic acid construct according to [2], wherein the second nucleic acid molecule comprises a continuous partial sequence in the gene sequence encoding the protein, the continuous partial sequence containing upstream and downstream regions adjacent to the stop codon and the cleavage site in the mutant gene sequence.
[6] The nucleic acid construct according to any one of [1] to [5], wherein each of the first homologous region and the second homologous region has a strand length of at least 20 bases.
[7] The nucleic acid construct according to any one of [1] to [6], wherein the first nucleic acid molecule comprises a poly-A addition signal downstream of the mutant gene sequence.
[8] The nucleic acid construct according to any one of [1] to [7], wherein the cleavage site is a restriction enzyme recognition site.
[9] The nucleic acid construct according to any one of [1] to [8], wherein the second nucleic acid molecule is a circular nucleic acid molecule further containing an additional sequence of 1 to 20,000 bases linked to the complementation region, or is a linear nucleic acid molecule containing an additional sequence of 1 to 10,000 bases linked to at least one of the 5'-end and the 3'-end of the complementation region.
[10] The nucleic acid construct according to any one of [1] to [9], wherein the first nucleic acid molecule is a circular nucleic acid molecule, or a linear nucleic acid molecule prepared by cleaving said circular nucleic acid molecule at the cleavage site.
[11] The nucleic acid construct according to any one of [1] to [10], wherein the gene sequence is a gene sequence encoding a protein that acts to decrease cell survival rate, or a protein whose intracellular expression is detectable.
[12] The nucleic acid construct according to [11], wherein the gene sequence is a sequence of a suicide gene, a DNA damage-inducing gene, a DNA repair-inhibiting gene, a luminescent enzyme gene, a fluorescent protein gene, a cell surface antigen gene, a secretory protein gene, or a membrane protein gene.
[13] A measurement reagent for homologous recombination activity, the measurement reagent comprising the nucleic acid construct according to any one of [1] to [12], wherein the gene sequence is a gene sequence encoding a protein whose intracellular expression is detectable.
[14] A measurement kit for homologous recombination activity, the kit comprising the measurement reagent according to [13].
[15] A screening system for an agent that affects homologous recombination activity, the screening system comprising the nucleic acid construct according to any one of [1] to [12].
[16] A diagnostic agent for a homologous recombination-deficient cancer, the agent comprising the nucleic acid construct according to any one of [1] to [12].
[17] A detection agent for homologous recombination-restored cancer cells, the agent comprising the nucleic acid construct according to any one of [1] to [12], wherein the gene sequence is a gene sequence encoding a protein whose intracellular expression is detectable.
[18] The detection agent according to [17], wherein the homologous recombination-restored cancer cells are PARP inhibitor-resistant cancer cells.
[19] A companion diagnostic agent for predicting an effect of an anticancer drug on homologous recombination-deficient cancer, the agent comprising the nucleic acid construct according to any one of [1] to [12].
[20] The companion diagnostic agent according to [19], wherein the anticancer drug is a DNA-damaging anticancer drug.
[21] The companion diagnostic agent according to [20], wherein the DNA-damaging anticancer drug is a PARP inhibitor.
[22] A therapeutic agent for homologous recombination-restored cancer, the agent comprising the nucleic acid construct according to any one of [1] to [12], wherein the gene sequence is a gene sequence encoding a protein that acts to decrease cell survival rate.
[23] The therapeutic agent according to [22], wherein the homologous recombination-restored cancer is PARP inhibitor-resistant cancer.
[24] A method of measuring homologous recombination activity in test cells, the method comprising:
   introducing a nucleic acid construct according to any one of [1] to [12], wherein the gene sequence is a gene sequence encoding a protein whose intracellular expression is detectable, into test cells whose homologous recombination activity is to be measured, and into homologous recombination-proficient control cells having normal homologous recombination activity;
   measuring the expression levels of the protein in the test cells and the homologous recombination-proficient control cells; and
   comparing the expression level in the test cells with the expression level in the homologous recombination-proficient control cells;
   wherein a protein expression in the test cells that is lower than the protein expression in the homologous recombination-proficient control cells indicates that the test cells have a lower homologous recombination activity.
[25] A method of identifying a candidate of an agent that affects homologous recombination activity, the method comprising:
   introducing the nucleic acid construct according to any one of [1] to [12] into cells having normal homologous recombination activity, and then treating the cells with each compound in a compound group; or treating cells having normal homologous recombination activity with each compound in a compound group, and then introducing the nucleic acid construct according to any one of [1] to [12] into the cells; and
   measuring expression of the protein.
[26] The method according to [25], wherein the protein is a protein whose intracellular expression is detectable as a signal, the method comprising selecting a compound as a candidate of an inhibitor that inhibits homologous recombination activity when the signal of the protein detected is lower in the cells treated with the compound than in the cells not treated with the compound, or selecting a compound as a candidate of an enhancer that promotes homologous recombination activity when the signal of the protein rises more rapidly or a higher signal is detected in the cells treated with the compound compared to the cells not treated with the compound.
[27] The method according to [25], wherein the protein is a protein that acts to decrease cell survival rate, the method comprising selecting a compound as a candidate of an inhibitor that inhibits homologous recombination activity when the decrease in the cell survival rate is suppressed in the cells treated with the compound compared to the cells not treated with the compound, or selecting a compound as a candidate of an enhancer that promotes homologous recombination activity when the decrease in the cell survival rate occurs earlier in the cells treated with the compound than in the cells not treated with the compound.
[28] A diagnostic method for homologous recombination-deficient cancer, the method comprising:
   introducing the nucleic acid construct according to any one of [1] to [12] into cancer cells of a cancer patient; and
   measuring expression of the protein.
[29] The method according to [28], wherein the cancer cells are cells separated from the cancer patient, and the introduction of the nucleic acid construct into the cancer cells is carried out in vitro.
[30] The method according to [28], wherein: the protein is a protein whose intracellular expression is detectable as a signal; the introduction of the nucleic acid construct into the cancer cells is carried out by administering the nucleic acid construct to the cancer patient; and whether or not the signal of the protein is detected from a cancer lesion is investigated.
[31] The method according to [28], wherein: the protein is a secretory protein whose intracellular expression is detectable; the introduction of the nucleic acid construct into the cancer cells is carried out by administering the nucleic acid construct to the cancer patient; and the activity of the protein in blood separated from the patient after the administration of the nucleic acid construct is measured.
[32] A detection method for homologous recombination-restored cancer cells, the method comprising:
   introducing the nucleic acid construct according to any one of [1] to [12] into cancer cells of a cancer patient that is a patient under treatment with a PARP inhibitor or that is a patient who was once diagnosed with homologous recombination-deficient cancer; and
   measuring expression of the protein.
[33] The method according to [32], wherein the cancer cells are cells separated from the patient, and the introduction of the nucleic acid construct into the cancer cells is carried out in vitro.
[34] The method according to [32], wherein: the protein is a protein whose intracellular expression is detectable as a signal; the introduction of the nucleic acid construct into the cancer cells is carried out by administering the nucleic acid construct to the patient; and whether or not the signal of the protein is detected from a cancer lesion is investigated.
[35] A method of predicting an effect of an anticancer drug on homologous recombination-deficient cancer, the method comprising:
   introducing the nucleic acid construct according to any one of [1] to [12] into cancer cells of a cancer patient; and
   measuring expression of the protein.
[36] The method according to [35], wherein the anticancer drug is a DNA-damaging anticancer drug.
[37] The method according to [36], wherein the DNA-damaging anticancer drug is a PARP inhibitor.
[38] The method according to any one of [35] to [37], wherein the cancer cells are cells separated from the cancer patient, and the introduction of the nucleic acid construct into the cancer cells is carried out in vitro.
[39] The method according to any one of [35] to [37], wherein: the protein is a protein whose intracellular expression is detectable as a signal; the introduction of the nucleic acid construct into the cancer cells is carried out by administering the nucleic acid construct to the cancer patient; and whether or not the signal of the protein is detected from a cancer lesion is investigated.
[40] The method according to any one of [35] to [37], wherein: the protein is a secretory protein whose intracellular expression is detectable; the introduction of the nucleic acid construct into the cancer cells is carried out by administering the nucleic acid construct to the cancer patient; and activity of the protein in blood separated from the patient after the administration of the nucleic acid construct is measured.
[41] A therapeutic method for homologous recombination-restored cancer, the method comprising administering a nucleic acid construct according to any one of [1] to [12], wherein the gene sequence is a gene sequence encoding a protein that acts to decrease cell survival rate, to a patient having the homologous recombination-restored cancer.
[42] The method according to [41], wherein the nucleic acid construct is topically administered into a tumor or into the vicinity of a tumor of the patient.
[43] The method according to [41] or [42], wherein the homologous recombination-restored cancer is PARP inhibitor-resistant cancer.
[44] A method of predicting whether or not a gene mutation identified in a cancer patient is a pathogenic mutation that deteriorates homologous recombination activity, the method comprising:
   constructing an expression vector that expresses a mutant gene having the same mutation as the previously-mentioned mutation;
   providing an expression vector that expresses a wild-type gene not having the mutation;
   introducing each of the mutant-gene expression vector and the wild-type-gene expression vector, and also introducing the nucleic acid construct according to any one of [1] to [12], into cells deficient in the gene; and
   measuring expression of the protein in the cells in which each expression vector and the nucleic acid construct are introduced;
   wherein the mutation is indicated to be a pathogenic mutation that deteriorates homologous recombination activity when the expression level of the protein in the cells in which the mutant-gene expression vector is introduced is lower than the expression level of the protein in the cells in which the wild-type-gene expression vector is introduced.

### EFFECT OF THE INVENTION

By the present invention, a novel means that enables evaluation of the homologous recombination activity itself of a cell as an absolute value rather than relative evaluation is provided. A nucleic acid construct of the present invention is a divided-type nucleic acid construct constituted by a set of a first nucleic acid molecule comprising a mutant gene sequence, and a second nucleic acid molecule comprising a complementation region capable of complementing the mutated portion of the mutant gene sequence by homologous recombination to reconstruct a gene sequence capable of expressing active protein. By the nucleic acid construct of the present invention, the presence or absence of homologous recombination activity can be detected using a transient expression system in a very short time. The nucleic acid construct of the present invention is useful as a measurement reagent or measurement kit for homologous recombination activity, and can also be used, for example, as a screening system for an agent that affects homologous recombination activity, as a diagnostic agent for a homologous recombination-deficient cancer, as a detection agent for homologous recombination-restored cancer cells, or as a companion diagnostic agent for predicting an effect of an anticancer drug on a homologous recombination-deficient cancer. Further, by utilizing a sequence of a gene encoding a protein that acts to decrease the cell survival rate such as a suicide gene as the gene sequence, a nucleic acid construct that selectively exerts a cytocidal effect on cells having homologous recombination activity can be obtained. Anticancer-drug-resistant cancers with restored homologous recombination activity have been a serious problem in the treatment with a PARP inhibitor, and no therapeutic method for such cancers exists at present. However, the nucleic acid construct of the present invention enables treatment of even such resistant cancers. Further, by utilization of the construct of the present invention, whether or not a mutation identified in a cancer patient is a pathogenic mutation can be determined or predicted.

### BRIEF DESCRIPTION OF THE DRAWINGS

[Fig. 1] A schematic diagram illustrating the structures of the integrated-type and the divided-type nucleic acid constructs carrying the *Nluc* gene, which were prepared in Example A.
[Fig. 2] A structural drawing of pIRES, used for the preparation of plasmid-vector-type nucleic acid constructs in Examples (the structural drawing in pIRES Vector Information of Clontech was partially modified).
[Fig. 3] A structural drawing of pUC19, used for the preparation of one of the molecules in each divided-type nucleic acid construct in Examples (from the pUC19 DNA datasheet of Takara Bio Inc.).
[Fig. 4] An example of the result of a luciferase assay using nucleic acid constructs prepared using the *Nluc* gene. The luciferase activity after introduction of each construct was measured over time for a wild line and a *RAD54*/*RAD54B-double-*deficient line of Nalm-6 cells.
[Fig. 5] An example of the result of a luciferase assay using nucleic acid constructs prepared using the *Nluc* gene. The luciferase activity 4 hours after the introduction of each construct was compared between a wild line and a *RAD54*/*RAD54B-double-*deficient line of Nalm-6 cells.
[Fig. 6] An example of the result of a luciferase assay using nucleic acid constructs prepared using the *Nluc* gene. The luciferase activity 4 hours after introduction of each construct was compared between HT1080 cells and MDA-MB-436 cells.
[Fig. 7] A result of investigation of the effect of *RAD51* gene knockdown on the luciferase activity detected after introduction of each construct into Nalm-6 cells.
[Fig. 8] A schematic diagram illustrating the structures of the integrated-type and the divided-type nucleic acid constructs carrying the suicide gene *DT-A*, which were prepared in Example B.
[Fig. 9] The cytocidal effects of nucleic acid constructs prepared using the *DT-A* gene. (A) A result of comparison of the survival rate 96 hours after the introduction of the construct, between a wild line and a *RAD54*/*RAD54B*-double-deficient line of Nalm-6 cells. (B) A result of comparison of the survival rate 96 hours after introduction of each construct, between the human tumor-derived cell line HT1080, which is proficient in homologous recombination, and the human tumor-derived cell line MDA-MB-436, which is deficient in homologous recombination activity due to BRCA1 deficiency.
[Fig. 10] A schematic diagram illustrating the structures of the integrated-type and the divided-type nucleic acid constructs carrying the suicide gene *HSV-TK*, which were prepared in Example C.
[Fig. 11] A result of comparison of the cytocidal effect of each construct prepared using the *HSV-TK* gene, between MDA-MB-436 and HT1080 cells.
[Fig. 12] A schematic diagram illustrating the structures of the integrated-type and the divided-type nucleic acid constructs carrying the suicide gene *TK30,* which were prepared in Example D. The top row shows a TK30 expression construct, which is a positive control; the middle row shows the integrated-type construct; and the bottom row shows the divided-type construct.
[Fig. 13] A diagram illustrating a mutation introduction site of TK30.
[Fig. 14] The cytocidal effects of nucleic acid constructs prepared using the *TK30* gene. The survival rate 72 hours after introduction of each construct was compared between Nalm-6 cells and HR-deficient cells thereof (*RAD54*/*RAD54B-double-*deficient line).
[Fig. 15] A result of comparison of the survival rate after introduction of each construct prepared using the *TK30* gene, between MDA-MB-436 cells (*BRCA1-*deficient cells) and Olap^{R} cells (MDA-MB-436 cells with restored homologous recombination activity).
[Fig. 16] A schematic diagram illustrating the structures of the integrated-type and the divided-type nucleic acid constructs carrying the suicide gene *CD::UPRT* gene, which were prepared in Example E. The top row shows constructs expressing CD::UPRT fusion protein, CD, and UPRT, respectively ; the middle row shows the integrated-type construct; and the bottom row shows the divided-type construct.
[Fig. 17] A result of comparison of the survival rate after introduction of each construct shown in the top row of Fig. 16 into a wild line of Nalm-6 cells, for investigation of whether or not the cytotoxicity by 5-FC is enhanced by co-expression of CD and UPRT.
[Fig. 18] A result of comparison of the survival rate after introduction of each construct prepared using the *CD::UPRT* gene, between Nalm-6 cells and HR-deficient cells thereof (*RAD54*/*RAD54B*-double-deficient line).
[Fig. 19] A result of comparison of the survival rate after introduction of each construct prepared using the *CD::UPRT* gene, between MDA-MB-436 cells and Olap^{R} cells.
[Fig. 20] A schematic diagram illustrating the structures of the integrated-type and the divided-type nucleic acid constructs carrying the *CeNL* gene, which were prepared in Example F. The top row shows a CeNL expression construct, which is a positive control; the middle row shows the integrated-type construct; and the bottom row shows the divided-type construct.
[Fig. 21] A result of measurement of the luciferase activity over time after introduction of each construct prepared using the *CeNL* gene in a wild line and a *RAD54*/*RAD54B*-double-deficient line (homologous recombination-deficient cells) of Nalm-6 cells.
[Fig. 22] A result obtained by introducing the constructs of Example A, which utilize Nluc, or the constructs of Example F, which utilize CeNL, into Nalm-6 cells, and performing a luciferase assay 2 hours after the introduction.
[Fig. 23] A result obtained by introducing the constructs of Example A, which utilize Nluc, or the constructs of Example F, which utilize CeNL, into Nalm-6 cells, and performing a luciferase assay 4 hours after the introduction.
[Fig. 24] A result of comparison of the luciferase activity after introduction of each construct which utilizes CeNL, between MDA-MB-436 cells (cells deficient in homologous recombination activity due to *BRCAl* deficiency) and Olap^{R} cells (MDA-MB-436 cells with restored homologous recombination activity).
[Fig. 25] A result obtained by introducing the constructs of Example A, which utilize Nluc, or the constructs of Example F, which utilize CeNL, into MDA-MB-436 cells and Olap^{R} cells, and performing a luciferase assay 2 hours after the introduction.
[Fig. 26] A result obtained by introducing the constructs of Example A, which utilize Nluc, or the constructs of Example F, which utilize CeNL, into MDA-MB-436 cells and Olap^{R} cells, and performing a luciferase assay 4 hours after the introduction.

### MODE FOR CARRYING OUT THE INVENTION

The nucleic acid construct of the present invention is a construct constituted by a set of two nucleic acid molecules. In the present description, this nucleic acid construct may be referred to as divided-type nucleic acid construct.

The first nucleic acid molecule comprises: a promoter region; and a mutant gene sequence placed downstream thereof, which mutant gene sequence is constituted by a gene sequence encoding a protein and a cleavage site arranged inside thereof. The second nucleic acid molecule comprises a complementation region capable of replacing, by homologous recombination (HR), a partial region comprising the cleavage site in the mutant gene sequence, the complementation region being constituted by a first homologous region and a second homologous region. This complementation region is a region composed of a base sequence selected from the following (i) to (iii):
(i) a continuous partial sequence in the gene sequence encoding the protein, the continuous partial sequence containing upstream and downstream regions adjacent to the cleavage site in the mutant gene sequence;
(ii) a base sequence which is homologous to the partial sequence of (i) and encodes the same amino acid sequence as that encoded by the partial sequence; and
(iii) a continuous partial sequence in a gene sequence encoding a protein whose sequence is 80% or more identical to that of the previously-mentioned protein and which has the same activity as the previously-mentioned protein, the continuous partial sequence being a base sequence homologous to the partial sequence of (i).

In the present invention, homologous recombination (HR) is a recombination reaction that occurs due to RAD51-dependent HR (which may be referred to as "standard HR"). It is widely known in the art that, in general, HR occurs when the homology between the homologous region and the substrate sequence (the target sequence of the recombination reaction) is 100%, and that the RAD51-dependent standard HR can occur even in cases where mismatched bases are partially present.

The gene sequence employed for the nucleic acid construct of the present invention is not limited as long as it is a gene sequence encoding a protein. In the present description, the protein encoded by this gene sequence may be referred to as protein A. In the nucleic acid construct of the present invention, in a cell having HR activity, a partial region containing the cleavage site (the stop codon and the cleavage site in cases where the mutant gene sequence contains a stop codon upstream of the cleavage site) in the mutant gene sequence in the first nucleic acid molecule is replaced by the complementation region contained in the second nucleic acid molecule by HR to reconstruct the gene sequence encoding protein A, resulting in production of protein A. On the other hand, in a cell having no HR activity, the reaction to replace the cleavage site (the stop codon and the cleavage site, in the cases where the stop codon is contained) in the mutant gene sequence with the complementation region does not occur, so that protein A is not expressed. Thus, whether or not the cell to which the nucleic acid construct has been introduced has HR activity can be detected based on the amount of expression of protein A, preferably based on the expression level of the activity of protein A, or HR activity-restored cancer cells can be selectively killed utilizing the activity of protein A.

The gene sequence encoding protein A reconstructed by the homologous recombination may be a sequence containing the entire coding region of a naturally occurring gene, or may be a sequence composed of part thereof or a modified sequence thereof. For example, a sequence encoding a region obtained by deleting a small number of residues (e.g. about several residues) not important for the activity of the protein from one or both of the C-terminus and the N-terminus, or only a region encoding a domain required for the exertion of the activity of the protein (for example, a sequence encoding a protein fragment obtained by removal of domains that are not necessary for the protein activity itself such as a domain required for membrane localization), in a naturally occurring gene sequence may be used as the gene sequence in the present invention. Further, for fluorescent proteins and luminescent proteins, genes with various modifications have been widely commercially utilized. Such gene sequences encoding non-naturally occurring proteins may also be used.

Further, a gene sequence capable of expressing two or more kinds of proteins as a fusion protein, or a gene sequence capable of polycistronic expression of such proteins, may be used as the gene sequence encoding protein A. Thus, the term protein A encompasses a fusion protein, and a set of two or more kinds of proteins expressed in a polycistronic manner. Typical examples of such modes include: a gene sequence encoding a fusion protein of protein α whose activity can be measured directly, or indirectly using as an index, for example, a phenotypic change that occurs in the cell due to the protein activity, and protein β that acts to enhance the activity of protein α; and a gene sequence capable of polycistronic expression of such proteins. Also in such modes, the sequence encoding each constituent protein (for example, the protein α or β) may be a sequence containing the entire coding region of a naturally occurring gene, or may be a sequence composed of part thereof or a modified sequence thereof (whose specific examples are as described above). Techniques for polycistronic expression of a plurality of proteins are well known. For example, a plurality of protein-coding sequences may be linked together through an IRES sequence(s) or a 2A peptide-coding sequence(s). Specific examples of the IRES sequences and the 2A peptide-coding sequences include: known IRES sequences such as the IRES sequence shown in SEQ ID NO:88 and the IRES2 sequence shown in SEQ ID NO: 89 (both of these are sequences derived from the internal ribosome entry site of encephalomyocarditis virus (ECMV)); and 2A peptide-coding sequences such as the P2A-coding sequence (SEQ ID NOs:90 and 91), the E2A-coding sequence (SEQ ID NO:92 and 93), and the F2A-coding sequence (SEQ ID NOs:94 and 95). As is well known, in cases where an IRES sequence is used, a stop codon needs to be added to the upstream gene sequence, and, in cases where a 2A peptide-coding sequence is used, no stop codon must be added to the upstream gene.

Protein A is preferably a protein whose expression level can be rapidly and simply measured. For example, the protein used as protein A is preferably a protein whose activity can be measured directly, or indirectly using as an index, for example, a phenotypic change that occurs in the cells due to the activity of the protein, rather than a protein requiring measurement of the amount of production or accumulation of the protein itself. Examples of such a protein include proteins whose intracellular expression is detectable based on a signal such as luminescence due to reaction with a substrate, or fluorescence of the protein itself. Another example is a protein that acts to decrease the cell survival rate. In this case, the activity of the protein can be indirectly measured as a decrease in the cell survival rate.

The term "measurement of expression (the expression level) of protein A" includes measurement of the amount of production or accumulation of protein A, and measurement of the activity of protein A. As described above, in the present invention, it is preferred to measure the expression of protein A by directly or indirectly measuring the activity of protein A. In other words, it is preferred to employ, as protein A, a protein whose activity can be directly or indirectly measured.

Preferred examples of protein A include a protein that acts to decrease the cell survival rate, and a protein whose intracellular expression is detectable.

Specific examples of the gene encoding a protein that acts to decrease the cell survival rate include, but are not limited to, suicide genes, DNA damage-inducing genes, and DNA repair-inhibiting genes.

Examples of the suicide genes include genes encoding a protein which by itself is toxic or injurious to cells, genes encoding a protein which acts on another compound to produce a toxic substance, and genes encoding a naturally occurring or non-naturally occurring protein that inhibits an endogenous protein to exert cytotoxicity by the dominant negative effect.

Specific examples of the suicide genes include the diphtheria toxin A fragment gene (*DT-A*) (the DT-A protein itself has toxicity to cells, and kills the cells), the herpesvirus-derived thymidine kinase gene (*HSV-TK*) (HSV-TK protein acts on ganciclovir, 5-iodo-2'-fluoro-2'deoxy-1-beta-D-arabino-furanosyl-uracil (FIAU), or the like to convert it to a toxic substance having a DNA synthesis-inhibiting activity; use of this gene as a suicide gene requires treatment of the cells with ganciclovir, FIAU, or the like), the *p53* gene (its overexpression induces cell cycle arrest or apoptosis to cause cell death), and the cytosine deaminase (CD) gene (CD converts 5-fluorocytosine (5-FC) to 5-fluorouracil by deamination. 5-Fluorouracil is metabolized in the cells to become a substance that inhibits DNA synthesis and RNA synthesis, to induce cell death (Austin EA and Huber BE. Mol Pharmacol. 1993 Mar; 43(3): 380-387.).); and modified genes thereof prepared such that the actions of these genes are enhanced.

Specific examples of the modified suicide genes include the *TK30* gene, which is a mutant of the *HSV-TK* gene (see Example D below), and the *CD::UPRT* gene, which is a fusion gene of the *CD* gene and the uracil phosphoribosyl transferase *(UPRT)* gene (see Example E below).

The *TK30* gene is a mutant of the *HSV-TK* gene, prepared by introducing mutations in amino acids near the active center of the *HSV-TK* gene (more specifically, mutations that substitute the alanine at position 152 with valine, the leucine at position 159 with isoleucine, the isoleucine at position 160 with leucine, the phenylalanine at position 161 with alanine, the alanine at position 168 with tyrosine, and the leucine at position 169 with phenylalanine in HSV-TK protein) so as to enhance the action on ganciclovir. The *TK30* gene is known to exhibit a stronger cytotoxicity than that of the wild-type HSV-TK (Kokoris MS et al. Gene Ther. 1999, Aug; 6(8): 1415-1426.).

The *CD::UPRT* gene is a fusion gene for utilizing UPRT, which is known to catalyze a metabolic pathway that converts 5-fluorouracil to a toxic substance in the cell, in combination with CD. By the combination, a cytotoxicity-enhancing effect and a bystander effect due to 5-fluorocytosine are obtained (Tiraby M et al. FEMS Microbiol Lett. 1998 Oct 1; 167(1): 41-49., Bourbeau D et al. J Gene Med. 2004 Dec; 6(12): 1320-1332.). In the Example E below, CD (NP_015387, SEQ ID NO:53) and UPRT (NP_011996, SEQ ID NO:55) derived from budding yeast were utilized, and the full length of a base sequence (SEQ ID NO:52) obtained by codon optimization of the budding yeast *CD* gene *(Fcyl* gene, NM_001184159, SEQ ID NO:82) and almost the full length of a base sequence (SEQ ID NO:54) obtained by codon optimization of the budding yeast *UPRT* gene *(Furl* gene, NM_001179258, SEQ ID NO:83) were linked to each other through an alanine codon, to provide, as the *CD::UPRT* gene, a fusion gene encoding a fusion protein (SEQ ID NO:51) having a structure in which the budding yeast-derived full-length CD and the portion from the third residue to the C-terminal residue of the budding yeast-derived UPRT are linked to each other through one alanine residue. However, the organism species from which the genes are derived, and the fusion method, are not limited to those in this specific example. The *CD::UPRT* gene may be freely designed by subjecting known gene sequences encoding CD and UPRT (each of which may be the full length of a naturally-occurring gene sequence, or may be a sequence composed of part thereof or a modified sequence thereof, as defined above for the gene sequence) to codon optimization in a manner suitable for the animal species of the cells into which the construct is to be introduced, and linking these to each other through, if desired, a linker residue(s) of one or more residues. Further, as mentioned above, the CD and the UPRT can be expressed in a polycistronic manner. Known examples of the *CD* gene and the *UPRT* gene include not only the *Fcyl* gene and the *Furl* gene derived from budding yeast, but also the *codA* gene, which is the *CD* gene of *E. coli* (the protein ID of the CD encoded by this gene is NP_414871.1; SEQ ID NOs:84 and 85), and the *upp* gene, which is the *UPRT* gene of *E. coli* (the protein ID of the UPRT encoded by this gene is NP_416993.2; SEQ ID NOs:86 and 87).

Further specific examples of the suicide genes include naturally occurring nucleases represented by restriction enzymes and meganucleases; and artificial nucleases represented by ZFN and TALEN, and the CRISPR system (these are also included in specific examples of the DNA damage-inducing genes). However, suicide genes are not limited to these specific examples.

Examples of the gene encoding a protein whose intracellular expression is detectable include, but are not limited to, luminescent enzyme genes such as luciferase genes (including genes encoding a secretory luciferase); fluorescent protein genes (genes encoding a naturally occurring or an artificial fluorescent protein including GFP, CFP, OFP, RFP, and YFP, and modified types thereof); cell surface antigen genes; secretory protein genes; and membrane protein genes. Luminescent enzyme genes and fluorescent protein genes are genes encoding a protein whose intracellular expression is detectable as a signal such as luminescence or fluorescence, and such genes are included in examples of genes that may be preferably used in the present invention. As is the case with the suicide genes, modified genes obtained by enhancing the actions of the above-described genes can also be used. Specific examples of the modified genes include the *CeNL* (cyan enhanced nano-lantern) gene (see Example F below), which encodes a fusion protein of Nluc, which is a chemiluminescent protein, and mTurquoise2 (Goedhart J et al. Nat Commun. 2012, Mar 20; 3: 751.), which is a cyan fluorescent protein. The *CeNL* gene is known to exhibit enhanced luminescence intensity by forester resonance energy transfer (FRET) between Nluc and mTurquoise2 (Suzuki K et al. Nat Commun 2016, Dec 14; 7: 13718.).

The promoter contained in the first nucleic acid molecule is not limited, and may be any promoter as long as a promoter activity can be exerted in a cell (typically a mammalian cell such as a human cell). In general, a promoter that constitutively exerts a strong promoter activity in the cell into which the nucleic acid construct of the present invention is introduced may be preferably used. Or, an inducible promoter that exerts the promoter activity under certain conditions may be used. In the following Examples, a human cytomegalovirus promoter that constitutively exerts a strong promoter activity in a cell was used. However, the promoter is not limited thereto. Further, the promoter is not limited to a virus-derived promoter, and the origin of the promoter is not limited as long as it is a sequence having a promoter activity in a cell, preferably in a human cell. The term "a mutant gene sequence is functionally linked downstream of a promoter region" means that a promoter region and a mutant gene sequence are placed such that a gene sequence formed on the first nucleic acid molecule after homologous recombination reaction is capable of expressing protein A by regulation by the promoter. In the nucleic acid construct of the present invention, the mutant gene sequence may be placed downstream of the promoter region on the first nucleic acid molecule.

The mutant gene sequence contained in the first nucleic acid molecule is a base sequence having a structure in which a mutation (more specifically, a cleavage site) is introduced within a gene sequence encoding protein A. In cases where a gene sequence derived from a eukaryote is used as the gene sequence encoding protein A, the mutant gene sequence may be designed using the base sequence of mRNA after exclusion of introns. The source gene sequence, as long as it encodes protein A, may be a naturally occurring gene sequence, or may be a sequence comprising, at a site(s) other than the site where the mutation is introduced, one or more bases that are different from those in the naturally occurring gene sequence (typically a silent mutation(s)).

A stop codon may be contained upstream of the cleavage site. In this case, the stop codon and the cleavage site are introduced into the gene sequence encoding protein A such that they are arranged in the order of [stop codon] - [cleavage site] from the 5'-side. The inclusion of the stop codon upstream of the cleavage site is not indispensable. However, in cases where the first nucleic acid molecule is prepared as a circular molecule and used (introduced into cells, or administered to a patient) as it is in the circular form without linearization by cleavage at the cleavage site, a stop codon is preferably placed upstream of the cleavage site in order to securely stop expression of active protein A from the mutant gene sequence in the first nucleic acid molecule in a cell deficient in homologous recombination activity. The stop codon may be directly linked to the cleavage site, or a certain base sequence may be present therebetween. The strand length of [stop codon] - [cleavage site] is preferably not more than about 50 bases, for example, not more than about 30 bases.

Atypical example of the cleavage site is a restriction enzyme recognition site. However, the cleavage site is not limited thereto. The cleavage may be carried out not only by cleavage by a restriction enzyme, but also by, for example, use of a genome editing technique that causes a DNA strand break. Specific examples of such a cleavage include cleavage by a CRISPR/Cas system, i.e., cleavage by a complex of a guide RNA and a Cas protein such as Cas9. In cases where the complex is used, a PAM sequence is introduced into an appropriate position such that the cleavage by the complex occurs at an appropriate position downstream of the stop codon. In cases of *Streptococcus pyogenes* type II-derived Cas9, which is the most commonly used system among the known CRISPR/Cas systems, the PAM sequence is 5'-NGG (N is A, T, G, or C). By the complex, the nucleic acid construct is cleaved several bases upstream of the PAM sequence. In the case of the cleavage by the CRISPR/Cas system, the PAM sequence and the actual position of the cleavage located several bases upstream thereof constitute the cleavage site in the present invention.

In the present invention, the strand length of a nucleic acid is expressed using the unit "bases". The "bases" means "base pairs" in cases where the nucleic acid is a double-stranded nucleic acid. [Cleavage site], or [stop codon] - [cleavage site] may be inserted within the gene sequence encoding protein A (in this case, the first nucleic acid molecule comprises the entire gene sequence encoding protein A), or part of the gene sequence encoding protein A may be replaced by [cleavage site] or [stop codon] - [cleavage site] (in this case, the first nucleic acid molecule comprises a partially deleted sequence of the gene sequence encoding protein A).

In the present invention, "homology" of a base sequence has the same meaning as "sequence identity" of a base sequence, and is determined by aligning two base sequences to be compared such that their bases match as many as possible, dividing the number of matched bases by the total number of bases, and expressing the resulting value of the division as a percentage. In the alignment, a gap(s) is/are inserted into one or both of the two sequences to be compared, when necessary. Such alignment of sequences can be carried out by using a well-known program such as BLAST, FASTA, or CLUSTAL W. When a gap(s) is/are inserted, the above-mentioned total number of bases is the number of bases determined such that one gap is counted as one base. When the thus counted total number of bases is different between the two sequences to be compared, the homology (%) is calculated by dividing the number of matched bases by the total number of bases of the longer sequence. "Sequence identity" of an amino acid sequence is calculated in the same manner.

In the mutant gene sequence, the upstream side and downstream side of [cleavage site] (when the stop codon is contained, [stop codon] - [cleavage site]) are hereinafter referred to as 5'-side region and 3'-side region, respectively. In cases where the construct is cleaved at the cleavage site and used in the linearized state, in a cell in which HR does not occur, a protein A fragment which lacks the region positioned downstream of the cleavage site is produced. In cases where the circular construct is used as it is, in a cell in which HR does not occur, translation stops by the introduced stop codon to produce a protein A fragment composed of the 5'-side region. Thus, the position of [cleavage site] or [stop codon] - [cleavage site] in the mutant gene sequence should be determined taking into account the size of the 5'-side region such that the activity of protein A is not exerted by the 5'-side-region fragment. The size of the 5'-side region is not limited, and may be appropriately set depending on the type of protein A. In general, the size of the 5'-side region may be not more than about 60/100, for example, not more than 50/100, not more than 40/100, not more than 30/100, or not more than 25/100 of the full length of protein A. In cases where protein A is a fusion protein of protein α that acts to decrease the cell survival rate or that acts to emit a signal and protein β that functions to enhance the action of protein α, and where protein β is fused to the C-terminal side of protein α, the size of the 5'-side region may be not more than about 60/100, for example, not more than 50/100, not more than 40/100, not more than 30/100, or not more than 25/100 of the protein α portion. Or, exertion of the activity by the protein A fragment composed of the 5'-side region can be avoided by placing the cleavage site in the upstream side of the base sequence encoding the amino acids of the active center of protein A (when protein A is a fusion protein prepared by fusing protein β to the C-terminal side of protein α, the active center of protein α) so that a region in the N-terminal side of the active center is the 5'-side region. Although there is no lower limit of the size of the 5'-side region, the size is usually set to not less than 50 bases.

The complementation region contained in the second nucleic acid molecule is a region which is constituted by a first homologous region and a second homologous region and capable of replacing, by homologous recombination, a partial region in the mutant gene sequence, the partial region comprising the cleavage site, and is composed of a base sequence selected from the following (i) to (iii).
(i) A continuous partial sequence in the gene sequence encoding protein A, the continuous partial sequence containing upstream and downstream regions adjacent to the cleavage site in the mutant gene sequence.
(ii) A base sequence which is homologous to the partial sequence of (i) and encodes the same amino acid sequence as that encoded by the partial sequence of (i).
(iii) A continuous partial sequence in a gene sequence encoding a protein whose sequence is 80% or more identical to that of protein A and which has the same activity as protein A, the continuous partial sequence being a base sequence homologous to the partial sequence of (i).

The entire 5'-side region in the mutant gene sequence contained in the first nucleic acid molecule, or a 3'-end-side portion of this region (that is, at least a 3'-end-side portion of the 5'-side region) corresponds to the upstream region adjacent to the cleavage site (or the stop codon and the cleavage site) in the mutant gene sequence (hereinafter referred to as "adjacent upstream region" for convenience) in the sequence of (i). In the sequence of (i), the adjacent upstream region constitutes the first homologous region. The at least a 3 '-end-side portion of the 5'-side region in the mutant gene sequence, and the adjacent upstream region in (i), are referred to as "first homologous region set" for convenience. The entire 3'-side region or a 5'-end-side portion of this region (that is, at least a 5'-end-side portion of the 3'-side region) corresponds to the downstream region adjacent to the cleavage site (or the stop codon and the cleavage site) in the mutant gene sequence ("adjacent downstream region") in the sequence of (i). In the sequence of (i), the adjacent downstream region constitutes the second homologous region. The at least a 5'-end-side portion of the 3'-side region in the mutant gene sequence, and the adjacent downstream region in (i), are referred to as "second homologous region set" for convenience. The strand length of each homologous region may be at least 20 bases, or may be, for example, not less than 50 bases, not less than 60 bases, not less than 70 bases, or not less than 80 bases. Although there is no upper limit, the strand length is usually not more than 10,000 bases, or may be, for example, not more than 5000 bases, not more than 1000 bases, not more than 500 bases, or not more than 300 bases.

A mode in which the complementation region is the base sequence of (i) is a mode in which the homology in the first homologous region set and the homology in the second homologous region set are 100%. In this mode, the corresponding sequences are completely the same.

A mode in which the complementation region is the base sequence of (ii) is a mode in which the encoded amino acid sequence is the same as (a partial sequence of) the amino acid sequence of protein A, but in which at least one of the homology in the first homologous region set and the homology in the second homologous region set is less than 100%. It is widely known in the art that, even in cases where corresponding sequences are not completely the same, RAD51-dependent HR occurs as long as there is not less than a certain level of homology. The sequence of (ii) is preferably 90% or more homologous, more preferably 95% or more, 96% or more, 97% or more, 98% or more, or 99% or more homologous, to the partial sequence of (i). For example, the homology in the first homologous region set is preferably 90% or more, 95% or more, 96% or more, 97% or more, 98% or more, or 99% or more, and the homology in the second homologous region set is preferably 90% or more, 95% or more, 96% or more, 97% or more, 98% or more, or 99% or more. In the sequence of (ii), of course, the regions corresponding to the adjacent upstream region and the adjacent downstream region in (i) constitute the first homologous region and the second homologous region, respectively.

The base sequence of (iii) is a continuous partial sequence in a gene sequence encoding a protein whose sequence is 80% or more, for example, 85% or more, 90% or more, 95% or more, or 98% or more, identical to that of protein A, and which has the same activity as protein A, the continuous partial sequence being a base sequence homologous to the partial sequence of (i). This is a mode in which at least one of the homology in the first homologous region set and the homology in the second homologous region set is less than 100%, and in which the encoded amino acid sequence is not the same as the amino acid sequence of protein A. Even if the encoded amino acid sequence is partially different from the amino acid sequence of protein A, the base sequence can be employed as the sequence of the complementation region as long as the encoded protein has the activity of protein A. Preferably, (iii) is 90% or more, for example, 95% or more, 96% or more, 97% or more, 98% or more, or 99% or more, homologous to the partial sequence of (i), and the encoded amino acid sequence is 80% or more, for example, 85% or more, 90% or more, 95% or more, or 98% or more, identical to the amino acid sequence encoded by the partial sequence of (i).

Conservative substitution, that is, substitution with an amino acid having similar chemical properties, is unlikely to cause deterioration of properties and activity of a protein. Amino acids having similar side chains have similar chemical properties. Based on the similarities among the side chains, amino acids can be classified into, for example, a group of amino acids having an aliphatic side chain (glycine, alanine, valine, leucine, and isoleucine), a group of amino acids having an aliphatic hydroxyl side chain (serine and threonine), a group of amino acids having an amide-containing side chain (asparagine and glutamine), a group of amino acids having an aromatic side chain (phenylalanine, tyrosine, and tryptophan), a group of amino acids having a basic side chain (arginine, lysine, and histidine), a group of amino acids having an acidic side chain (aspartic acid and glutamic acid), and a group of amino acids having a sulfur-containing side chain (cysteine and methionine). Substitution with another amino acid belonging to the same group is conservative substitution. Typical examples of the amino acid sequence encoded by the base sequence of (iii) include, but are not limited to, an amino acid sequence in which a conservative substitution(s) is/are introduced in the amino acid sequence encoded by the partial sequence of (i).

The mutant gene sequence and the complementation region can be prepared by PCR amplification using appropriate primers, from a known plasmid in which a gene encoding protein A is incorporated, or from cultured cells or a cDNA library of an organism species that expresses protein A. A stop codon is required at the 3'-end of the mutant gene sequence. The complementation region is a sequence that functions as a substrate for the replacement of the part of the mutant gene sequence by homologous recombination, and is a region not translated before the homologous recombination occurs. Therefore, it is not necessary to add a stop codon to the 3'-end of the complementation region. The following Examples describe cases where a stop codon is introduced at the 3'-end of an iNluc fragment or an iDTA fragment, which corresponds to the complementation region. Although a stop codon may be introduced as in these cases, the introduction is not essentially required. When desired, in the PCR amplification, in addition to the stop codon, an appropriate adaptor sequence(s) used for cloning of the nucleic acid construct of the present invention and/or the like may be introduced to the two gene fragments. The mutant gene sequence comprising, at a site(s) other than the site where the mutation is introduced, one or more bases that are different from those in the naturally occurring gene sequence, and the sequences of (ii) and (iii) of the complementation region, can be prepared by a method such as in vitro mutagenesis or artificial gene synthesis. The poly-A addition signal may be placed downstream of the mutant gene sequence (functionally linked downstream of the mutant gene sequence) in the first nucleic acid molecule. The functional linkage as used herein means that a poly-A addition signal is placed downstream of the mutant gene sequence such that an mRNA having a poly-A tail is expressed from the gene sequence encoding protein A formed after the homologous recombination reaction. However, in the nucleic acid construct of the present invention, the poly-A addition signal is not an indispensable element. Since mRNAs that appropriately function without a poly-A, such as a histone mRNA, are known, the poly-A addition signal may be omitted also in the nucleic acid construct of the present invention. Further, the complementation region may be constituted such that a portion which also contains a partial region adjacent to the mutant gene sequence in the first nucleic acid molecule is replaced by the homologous recombination. For example, the first homologous region contained in the complementation region may contain a region homologous to a partial region adjacent to the 5'-end of the mutant gene sequence in the first nucleic acid molecule. Or, the second homologous region contained in the complementation region may contain a region homologous to a partial region adjacent to the 3'-end of the mutant gene sequence in the first nucleic acid molecule.

Each of the first nucleic acid molecule and the second nucleic acid molecule may be either a circular molecule or linear molecule, may be in a form in which the nucleic acid molecule is incorporated in a plasmid vector or virus vector, or may be in a form of a nucleic acid fragment not incorporated in a vector. As described later, the nucleic acid construct of the present invention can be used as a pharmaceutical such as a therapeutic agent or diagnostic agent, or as a reagent such as a measurement reagent. In cases where the first nucleic acid molecule is a circular molecule, it may be used as it is in the circular form as the pharmaceutical or reagent, or may be used in the form of a linear molecule prepared by cleaving the circular first nucleic acid molecule at the cleavage site arranged inside the mutant gene sequence. Further, the circular first nucleic acid molecule may be used as two nucleic acid molecules prepared by cleaving the first nucleic acid molecule at the cleavage site in the mutant gene sequence and at an arbitrary site which is located downstream of the mutant gene sequence (in cases where a poly-A addition signal is contained, downstream of the poly-A addition signal) and upstream of the promoter region. In cases where the second nucleic acid molecule is a circular molecule, it may be used as it is in the circular form as the pharmaceutical or reagent, or may be used in the form of a linear molecule prepared by cleavage at a position outside the complementation region. The homologous recombination reaction can be allowed to occur more easily in cases where the circular second nucleic acid molecule is used as it is in the circular form. In cases where each of the first nucleic acid molecule and the second nucleic acid molecule is in a form in which the nucleic acid molecule is incorporated in a vector, the vector sequence portion may contain a common element(s) such as an origin of replication, a translation initiation site, or a selection marker gene including a drug resistance gene, depending on the type of the vector.

The second nucleic acid molecule may be a nucleic acid molecule composed only of the complementation region. However, the molecule preferably contains an additional sequence of one or more bases linked to the complementation region. By linking the additional sequence to the complementation region, homologous recombination reaction with the first nucleic acid molecule can be promoted.

In cases where the second nucleic acid molecule is a circular molecule, the molecule preferably further contains an additional sequence of 1 to 20000 bases linked to the complementation region. The additional sequence may be a sequence of, for example, not less than 20 bases, not less than 50 bases, not less than 100 bases, not less than 200 bases, not less than 500 bases, not less than 700 bases, or not less than 1000 bases, and not more than 10000 bases or not more than 5000 bases.

In cases where the second nucleic acid molecule is a linear molecule, an additional sequence of about 1 to 10000 bases is preferably linked to at least one of the 5'-end and 3'-end of the complementation region. When additional sequences are linked to both ends of the complementation region, "an additional sequence of about 1 to 10000 bases is linked to at least one" means that each additional sequence has a size of 1 to 10000 bases. In such cases, the additional sequence may be a sequence of, for example, not less than 20 bases, not less than 50 bases, not less than 100 bases, not less than 200 bases, not less than 500 bases, not less than 700 bases, or not less than 1000 bases, and may be not more than 8000 bases or not more than 5000 bases.

As the additional sequence, a base sequence not homologous to the mutant gene sequence needs to be employed in order to prevent occurrence of homologous recombination between the additional sequence and the mutant gene sequence on the first nucleic acid molecule. "Not homologous" means that the additional sequence does not contain a partial region which has a size of not less than 20 bases and which is 90% to 100% homologous to part or all of the mutant gene sequence. Further, the additional sequence should not be homologous to the regions outside the mutant gene sequence on the first nucleic acid molecule. Furthermore, in cases where the nucleic acid construct of the present invention is used by introduction into cells, it is desirable to employ a base sequence which does not have homology to the genomic sequence of the cell into which the construct is to be introduced. For example, an artificial nucleic acid molecule such as a known plasmid expression vector commercially available for transfection of prokaryotic cells such as E. *coli,* or eukaryotic cells such as yeast or mammalian cell lines, which artificial nucleic acid molecule is not used for the preparation of the first nucleic acid molecule, may be preferably used as the additional sequence. In cases where the second nucleic acid molecule is a circular molecule containing an additional sequence(s), an appropriate cleavage site (such as a restriction enzyme recognition site) which cleaves only the additional sequence and does not cleave the complementation region may be contained in the additional sequence. In cases where the cleavage site is contained in the additional sequence, the cleavage site may be set at a site almost equally distant from both ends of the complementation region, so as to design the second nucleic acid molecule such that the second nucleic acid molecule after the cleavage has additional sequence fragments having almost the same size at both ends of the complementation region.

The first and second nucleic acid molecules constituting the nucleic acid construct of the present invention may be either DNA or RNA. One of the first and second nucleic acid molecules may be DNA, and the other may be RNA. Or, both molecules may be DNA, or both molecules may be RNA. In general, DNA is preferred in cases of a plasmid-vector-type nucleic acid molecule. Typical examples of a virus-vector-type nucleic acid molecule include a nucleic acid molecule that is a double-stranded DNA in the form of a virus expression plasmid. Examples of the virus-vector-type nucleic acid molecule also include a virus expressed from a virus expression plasmid. The nucleic acid molecule that is a virus expressed from the virus expression plasmid is RNA in cases where the vector is an RNA virus vector, and DNA in cases where the vector is a DNA virus vector. In cases of a nucleic acid molecule in a form in which it is not incorporated in a vector, the nucleic acid molecule is usually DNA. The first and second nucleic acid molecules constituting the nucleic acid construct of the present invention may comprise a nucleotide analog in part thereof. Examples of the nucleotide analog include, but are not limited to, bridged nucleic acids such as LNA, ENA, and PNA; and modified bases such as Super T (registered trademark) and Super G (registered trademark).

The nucleic acid construct of the present invention may be used in a variety of uses as described later. In cases where the nucleic acid construct of the present invention is used as a reagent such as a measurement reagent, or as a diagnostic agent, a companion diagnostic agent, or a detection agent to be used in vitro, the nucleic acid construct may be provided in a form in which the first nucleic acid molecule and the second nucleic acid molecule are contained in separate containers, or may be provided in a form in which the first nucleic acid molecule and the second nucleic acid molecule are preliminarily mixed together at an appropriate ratio (which is, for example, about 1:0.001 to 1000, about 1:0.01 to 100, or about 1:0.1 to 10, or may be about 1:0.2 to 5, or 1:0.5 to 2, in terms of the molar ratio), and contained in the same container. The nucleic acid construct of the present invention may be a reagent or an agent in the form of a liquid containing an appropriate additive(s) for the stability or the like of the first and second nucleic acid molecules, or may be a reagent or an agent in the form of a powder prepared by freeze-drying the first and second nucleic acid molecules. In cases where the nucleic acid construct of the present invention is used as a diagnostic agent or a therapeutic agent to be used by administration to a patient, the diagnostic agent or therapeutic agent may be in a form in which the first nucleic acid molecule and the second nucleic acid molecule are formulated as separate agents, or may be in a form in which the first nucleic acid molecule and the second nucleic acid molecule are formulated to be contained in a single agent. "Diagnostic agent or therapeutic agent containing the nucleic acid construct of the present invention" encompasses a mode comprising the set of an agent containing the first nucleic acid molecule and an agent containing the second nucleic acid molecule, and a mode that is an agent comprising a mixture of the first nucleic acid molecule and the second nucleic acid molecule.

In the present invention, "introduction of a nucleic acid construct (into cells)" means co-introduction of the first nucleic acid molecule and the second nucleic acid molecule. In cases where the first nucleic acid molecule is a circular molecule, although the molecule can be introduced as it is in the circular form, it is preferred to use the molecule after linearizing it by cleavage at the cleavage site located in the mutant gene sequence. In cases where the second nucleic acid molecule is a circular molecule, the molecule may be introduced into cells after linearizing it by cleavage at an arbitrary site (for example, the cleavage site provided in the additional sequence) outside the complementation region, or may be introduced into cells as it is in the circular form.

"Administration of the nucleic acid construct to a patient" means administration of the first nucleic acid molecule and the second nucleic acid molecule to a patient. The first nucleic acid molecule and the second nucleic acid molecule are preferably administered simultaneously or sequentially. In cases of the sequential administration, either nucleic acid molecule may be administered prior to the other.

The nucleic acid construct of the present invention can be used, for example, as a measurement reagent for homologous recombination activity. In this mode of use, as the gene sequence encoding protein A, a gene sequence encoding a protein whose intracellular expression is detectable may be preferably used. The nucleic acid construct of the present invention may be introduced (that is, the first nucleic acid molecule and the second nucleic acid molecule are co-introduced as meant by the above definition) into test cells whose homologous recombination activity is to be measured, and cells having normal homologous recombination activity or cells not deficient in homologous recombination activity (HR-proficient control cells), and then expression of protein A may be measured, followed by comparing the expression level of protein A (a signal of protein A) between the test cells and the HR-proficient control cells. The introduction of the nucleic acid molecules may be transient. In the present invention, "introduction of a nucleic acid construct" means co-introduction of the first nucleic acid molecule and the second nucleic acid molecule. Further, cells deficient in homologous recombination activity may be used as HR-deficient control cells.

The HR-proficient control cells are not limited as long as they are cells having normal homologous recombination activity. Any of a number of known wild-type mammalian cell lines having no mutation in HR-related genes (such as *ATM, CDK1*, *CDK12*, *MRE11*, *NBS1*, *RAD50*, *PLK1*, *RNF1*, *RNF8*, *RNF168*, *ARID1A*, *SMARCAD1*, *CHD1*, *CHD4, LEDGF, RBBP8 (CtIP), BLM, Exo1*, *DNA2, BRCA1*, *BRCA2*, *PALB2*, *BARD1*, *BRIP1*, *BAP1*, *RAD51B*, *RAD51C*, *RAD51D*, *RAD51AP1, RAD52, XRCC2, XRCC3, RAD51, RAD54L (RAD54), RAD54B, FBH1*, *WRN*, *SMARCAL1*, *ATRX*, *PTEN*, *MUS81*, *EME1*, *EME2*, *SLX1*, *SLX4*, *XPF*, *ERCC1*, *RPA1, RPA2, PRA3, FANCA, FANCB, FANCC, FANCD2, FANCE, FANCF, FANCG, FANCI, FANCL, FANCM, LIG1*, *LIG3, POLD, HROB, MCM8, MCM9, HELQ, RAD21, RAD21L1*, *CHEK1*, *CHEK2, CHAMP1*, and *POGZ*) may be preferably used as the HR-proficient control cells. Specific examples of known mammalian cell lines having normal homologous recombination activity include, but are not limited to, human cell lines such as Nalm-6 (a human pre-B-cell leukemia cell-derived cell line) and HT1080 (a human fibrosarcoma cell line), which are also used in the Examples below, U2OS (a human osteosarcoma-derived cell line), HeLa (a human cervical cancer-derived cell line), HCT116 (a human colon adenocarcinoma-derived cell line), MCF-7 (a human breast adenocarcinoma-derived cell line), HAP1 (a human chronic myelogenous leukemia-derived cell line), HEK293 (a human embryonic kidney-derived cell line), TIG-7 (a human lung-derived cell line), TIG-3 (a human lung-derived cell line), iPS cells (human induced pluripotent stem cells; established from normal human cells), and ES cells (human embryonic stem cells). Cell lines derived from human hereditary breast cancer or hereditary ovarian cancer often have a mutation(s) in the *BRCA1* gene or the *BRCA2* gene, which are HR-related genes, and are hence often deficient in homologous recombination activity. Therefore, they are inappropriate as HR-proficient control cells. Whether or not an arbitrary cell line has normal homologous recombination activity (whether or not the cell line is deficient in homologous recombination activity) can be investigated by using the nucleic acid construct of the present invention, or by using a conventionally known technique. In cases where the investigation is carried out using the nucleic acid construct of the present invention, when the cells have homologous recombination activity almost equal to or higher than the above-described specific example of the HR-proficient control cells, the cells may be judged as having normal homologous recombination activity (being not deficient in homologous recombination activity).

Further, in order to provide an additional positive control, a construct in which a normal gene sequence encoding protein A is linked to a promoter under the control of the promoter (positive control construct) may be introduced into the test cells (and, when desired, into the control cells). The lower the homologous recombination activity of the test cells, the lower the expression (a signal of protein A detected from the test cells) of protein A in the test cells. Thus, a signal of protein A in the test cells that is lower than the signal of protein A in the HR-proficient control cells is an index indicating that the test cells have a lower homologous recombination activity.

Examples of the HR-deficient control cells include cell lines derived from homologous recombination-deficient cancers. A variety of kinds of cell lines derived from homologous recombination-deficient cancers having mutations in the HR-related genes described above (for example, human cell lines such as cell lines derived from human hereditary breast cancer or hereditary ovarian cancer wherein the *BRCA1* gene or the *BRCA2* gene has a mutation(s)), and cell lines prepared by introducing a mutation(s) to one or more HR-related genes and methods of preparing such cell lines are known, and any of such cell lines may be used as the HR-deficient control cells. Since the technique for gene knockout has also been established, HR-deficient cells of various animal species can be prepared by knocking out one or more HR-related genes in mammalian cultured cells having normal homologous recombination activity. Specific examples of known homologous recombination-deficient cell lines include human cell lines including: HR-deficient human cell lines derived from Nalm-6 cells (a *RAD54*-deficient line, a *RAD54B*-deficient line, and a *RAD54*/*RAD54B*-double-deficient line), which are used also in the Examples below; HR-deficient human cell lines available from ATCC (American Type Culture Collection), such as HCC1395 (a human breast cancer-derived cell line, deficient in *BRCA1*), HCC1599 (a human breast cancer-derived cell line, deficient in *BRCA2),* HCC1937 (a human breast cancer-derived cell line, deficient in *BRCAI),* MDA-MB-436 (a human breast cancer-derived cell line, deficient in *BRCA1*), DoTc2-4510 (a human cervical cancer-derived cell line, deficient in *BRCA2),* RL95-2 (a human uterine cancer-derived cell line, deficient in *BRCA2),* AGS (a human gastric cancer-derived cell line, deficient in *CHD1*), SNU-5 (a human gastric cancer-derived cell line, deficient in *CHD1*), UWB1.289 (a human ovarian cancer-derived cell line, deficient in *BRCA1*), CAPAN-1 (a human pancreatic cancer-derived cell line, deficient in *BRCA2),* and LNCaP (a human prostate cancer-derived cell line, deficient in *BRCA2);* and HR-deficient human cell lines available from Horizon Discovery Ltd., such as genetically modified HAP1 cell lines (human chronic myelogenous leukemia-derived cell lines) (an ATRX-deficient line, a *CHDI-*deficient line, a CHD4-deficient line, an *EME1*-deficient line, an EME2-deficient line, a *FANCA*-deficient line, a *FANCC*-deficient line, a *FANCE*-deficient line, a *FANCG*-deficient line, a *FANCF*-deficient line, a LEDGF-deficient line, a *LIG3-*deficient line, an *MUS81*-deficient line, a *RAD51AP1*-deficient line, a *RAD54L-*deficient line, and an *RNF8*-deficient line), genetically modified HCT116 cells (human colon adenocarcinoma-derived cell lines) (an ARIDIA-deficient line, a *LIG3-*deficient line, and a *PTEN*-deficient line), and a genetically modified DLD-1 cell line (a human colon adenocarcinoma-derived cell line) (a *BRCA2*-deficient line). Specific examples of cell lines of non-human animals include: HR-deficient mice (frozen embryos) available from The NCI Mouse Repository (*BRCA1-deficient* mice and BRCA2-deficient mice); and HR-deficient dog cells Parks (*MCM8*-deficient) (Sunetra Das et al., Mol Cancer Ther. Author manuscript; available in PMC 2020 Feb 1. Published in final edited form as: Mol Cancer Ther. 2019 Aug; 18(8): 1460-1471. Published online 2019 Jun 7. doi:10.1158/1535-7163.MCT-18-1346). However, as described above, the HR-deficient cells are not limited to these specific examples since HR-deficient cells can be prepared in various animal species by preparing cells in which one or more HR-related genes are knocked out.

The measurement reagent of the present invention can be provided also as a measurement kit for homologous recombination activity by combining the reagent with at least one item selected from, for example, a plasmid for a positive control (such as a plasmid in which a normal protein A-coding sequence is placed downstream of a promoter, which plasmid is capable of constitutively expressing protein A), a plasmid for a negative control (a plasmid in which a gene sequence encoding protein A is not incorporated), HR-proficient control cells, HR-deficient control cells, manufacturer's instructions, an appropriate substrate substance (in a case where protein A is an enzyme), and the like.

The nucleic acid construct of the present invention can be used also as, for example, a screening system for agents that affect homologous recombination activity. In other words, the nucleic acid construct of the present invention can be used for identification of candidates of agents that affect homologous recombination activity. A method of identifying a candidate of an agent that affects homologous recombination activity using the nucleic acid construct of the present invention comprises: introducing the nucleic acid construct of the present invention into cells having normal homologous recombination activity, and then treating the cells with each compound in a compound group; or treating cells having normal homologous recombination activity with each compound in a compound group, and then introducing the nucleic acid construct of the present invention into the cells; and measuring expression of protein A in the nucleic acid construct-introduced cells treated with the compound and in the nucleic acid construct-introduced cells not treated with the compound. As described above, the measurement of expression of protein A is preferably measurement of the activity of protein A. In this mode of use, as the gene sequence encoding protein A, a gene sequence encoding a protein whose intracellular expression is detectable, especially a gene sequence encoding a protein whose intracellular expression is detectable as a signal, may be preferably used, and a gene sequence encoding a protein that acts to decrease the cell survival rate may also be used. After cleaving, when desired, the first nucleic acid molecule at the cleavage site arranged inside the mutant gene sequence to prepare the first nucleic acid molecule into the linear form, the first nucleic acid molecule, together with the second nucleic acid molecule, may be introduced into cells having normal homologous recombination activity (HR-normal cells, usually mammalian cells such as cultured human cells, are preferably used; the details are the same as those of the HR-proficient control cells), and then the cells may be treated with each compound in a compound group such as a compound library (the order of the introduction of the nucleic acid construct and the treatment with each compound may be reversed), followed by measuring the expression of protein A to investigate whether or not a decrease or an increase in the expression level of protein A, preferably the activity level of protein A, has occurred (whether or not a decrease or an increase in the homologous recombination activity has occurred) due to the treatment with the compound. In cases where protein A is a luciferase, a change in the luciferase activity (luminous reaction) may be measured. In cases where protein A is a protein that acts to decrease the cell survival rate, a change in the cell survival rate may be investigated. The evaluation on whether the expression of protein A is decreased or increased can be carried out by introducing the nucleic acid construct into cells having normal homologous recombination activity, measuring the expression of protein A without carrying out the treatment with the compound, and then investigating whether the expression level is increased or decreased relative to this measured value. In addition to the HR-normal cells, HR-deficient cells that are deficient in homologous recombination activity (for example, a *RAD54*/*RAD54B-*double-deficient line, which is also used in the Examples below; the details are the same as those of the HR-deficient control cells) may be used.

In cases where protein A is a protein whose intracellular expression is detectable as a signal, the selection of a compound can be carried out as follows.

When the signal of protein A detected is lower in HR-normal cells treated with a compound than in HR-normal cells not treated with the compound, the compound can be selected as a candidate of an HR inhibitor that inhibits HR activity.

When the signal of protein A rises more rapidly or a higher signal is detected in HR-normal cells treated with a compound than in HR-normal cells not treated with the compound, the compound can be selected as a candidate of an HR enhancer that promotes HR activity.

In cases where protein A is a protein that acts to decrease the cell survival rate, the selection of a compound can be carried out as follows.

When the decrease in the cell survival rate is more suppressed in HR-normal cells treated with a compound than in HR-normal cells not treated with the compound (in other words, when the survival rate of HR-normal cells treated with a compound is higher than the survival rate of HR-survival cells not treated with the compound), the compound can be selected as a candidate of an HR inhibitor that inhibits HR activity.

When the decrease in the cell survival rate occurred earlier in HR-normal cells treated with a compound than in HR cells not treated with the compound, or the survival rate of HR-normal cells treated with a compound is lower than the survival rate of HR cells not treated with the compound, the compound can be selected as a candidate of an HR enhancer that promotes HR activity.

The nucleic acid construct of the present invention can also be used, for example, as a diagnostic agent for homologous recombination-deficient cancer. Deficiency in homologous recombination has been reported in various cancers. More specifically, for example, deficiency in homologous recombination has so far been reported in breast cancer and ovarian cancer (including hereditary breast ovarian cancer), and also in colorectal cancer, endometrial cancer, gastric cancer, esophagus cancer, pancreatic cancer, hepatobiliary cancer, prostate cancer, liver cancer, non-small-cell lung cancer, and small-cell lung cancer (Non-Patent Documents 6 to 9). It is expected that homologous recombination-deficient cancers will be found also in various cancers other than these (including cancers of various animal species including non-human animals) in the future. It has also been reported that molecular targeted cancer therapies using an EGFR inhibitor, a BRAF inhibitor or the like cause decrease in the homologous recombination activity (Science 20 Dec 2019: Vol. 366, Issue 6472, pp 1473-1480, DOI: 10.1126/science.aav4474). Examples of the homologous recombination-deficient cancer in the present invention include various cancers including the specific examples described above.

In cases where the nucleic acid construct of the present invention is used as a diagnostic agent for homologous recombination-deficient cancer, the construct is introduced into cancer cells of a cancer patient, and expression of protein A is measured. As described above, the measurement of expression of protein A is preferably measurement of the activity of protein A. As the gene sequence encoding protein A, a gene sequence encoding a protein whose intracellular expression is detectable, especially a gene sequence encoding a protein whose intracellular expression is detectable as a signal, may be preferably used, and a gene sequence encoding a protein that acts to decrease the cell survival rate may also be used. In cases where a cell is not deficient in homologous recombination, homologous recombination occurs between part of the mutant gene sequence present in the first nucleic acid molecule and the complementation region present in the second nucleic acid molecule to reconstruct a gene sequence capable of expressing protein A, in the cell. This results in expression of protein A. When protein A is a protein whose intracellular expression is detectable as a signal, the signal of protein A is detected. When protein A is a protein that acts to decrease the cell survival rate, the cell survival rate decreases. On the other hand, in cases where a cell is deficient in homologous recombination, the gene sequence capable of expressing protein A is not reconstructed, so that protein A is not expressed. When protein A is a protein whose intracellular expression is detectable as a signal, the signal of protein A is not detected. When protein A is a protein that acts to decrease the cell survival rate, decrease in the cell survival rate does not occur.

In one mode of the diagnostic agent for homologous recombination-deficient cancer, the cancer cells are cells separated from a cancer patient, and the introduction of the nucleic acid construct into the cancer cells is carried out in vitro. Among the modes of use described later, (1) and (3) are specific examples of this mode. In this mode, either a protein whose intracellular expression is detectable or a protein that acts to decrease the cell survival rate can be used as protein A, and the former is more preferred. In this mode, the cancer patient includes solid cancer patients and blood cancer patients. Cells in a cancer tissue separated from a cancer patient by biopsy or surgery, circulating tumor cells (CTCs) present in blood of a cancer patient, or, in cases of leukemia, cancer cells in blood (leukemia cells) collected from a cancer patient, can be used. If desired, the cancer cells may be concentrated before the introduction of the nucleic acid construct. The introduction of the nucleic acid construct may be transient. Whether or not cancer cells are deficient in homologous recombination activity can be investigated by measuring the homologous recombination activity of the cancer cells in the same manner as the above-described method of using the measurement reagent for homologous recombination activity. As the HR-proficient control cells, not only the above-described cells having normal homologous recombination activity, but also, for example, non-cancerous cells (non-cancerous peripheral blood cells or the like) collected from the same cancer patient may be used. As the HR-deficient control cells, the above-described homologous recombination-deficient cells may be used.

Preferably, in this mode, the nucleic acid construct is introduced into cancer cells separated from a cancer patient, and into control cells (HR-proficient control cells or HR-deficient control cells, or both), and then expression of protein A is compared between the cancer cells and the control cells.

In cases where expression/activity of protein A is detected in the cancer cells, for example, in cases where the expression level or activity level of protein A detected in the cancer cells is higher than the expression level or activity level of protein A in the HR-deficient control cells, the cancer in the cancer patient is indicated not to be a homologous recombination-deficient cancer. In cases where protein A is a protein whose intracellular expression is detectable as a signal, when the signal of protein A is detected in the cancer cells, for example, when the signal of protein A detected in the cancer cells is higher than the signal of protein A in the HR-deficient control cells, the cancer in the cancer patient is indicated not to be a homologous recombination-deficient cancer. In cases where protein A is a protein that acts to decrease the cell survival rate, when the survival rate of the cancer cells decreases, for example, when the survival rate of the cancer cells is lower than the survival rate of the HR-deficient control cells, the cancer in the cancer patient is indicated not to be a homologous recombination-deficient cancer.

In cases where expression/activity of protein A is not detected in the cancer cells, or in cases where the detected expression or activity of protein A is lower than that in the HR-proficient control cells, the cancer in the cancer patient is indicated to be a homologous recombination-deficient cancer. In cases where protein A is a protein whose intracellular expression is detectable as a signal, when the signal of protein A is not detected in the cancer cells, or when the detected signal is lower than the signal of protein A in the HR-proficient control, the cancer in the cancer patient is indicated to be a homologous recombination-deficient cancer. In cases where protein A is a protein that acts to decrease the cell survival rate, when the survival rate of the cancer cells does not decrease, or when the survival rate of the cancer cells is higher than the survival rate of the HR-proficient control cells, the cancer in the cancer patient is indicated to be a homologous recombination-deficient cancer.

In another mode, protein A is a protein whose intracellular expression is detectable as a signal; the introduction of the nucleic acid construct into the cancer cells is carried out by administering the nucleic acid construct to the cancer patient; and whether or not the signal of protein A is detected from a cancer lesion is investigated. Among the modes of use described later, (2) is a specific example of this mode. In this mode, the cancer patient is typically a solid cancer patient. The nucleic acid construct is preferably topically administered into the tumor or into the vicinity of the tumor of the cancer patient. From the viewpoint of detecting the signal of protein A from the cancer lesion in the body of the cancer patient, a protein that generates the signal without a reaction with a substrate, such as a fluorescent protein, is preferably employed. When the signal of protein A is detected from the cancer lesion (for example, when the level of the detected signal is almost the same as that of a non-cancerous site in the vicinity of the tumor), the cancer of the cancer patient is indicated not to be a homologous recombination-deficient cancer. When the signal of protein A is not detected from the cancer lesion (for example, when the signal from the cancer lesion is clearly lower than the signal from a non-cancerous site in the vicinity of the tumor), a possibility that the cancer of the cancer patient is a homologous recombination-deficient cancer is indicated. In this mode, when a patient is diagnosed with a possible homologous recombination-deficient cancer, cancer cells may be collected from the patient, and the nucleic acid construct may be introduced into the cells to confirm that the cancer is a homologous recombination-deficient cancer.

In still another mode, protein A is a secretory protein whose intracellular expression is detectable (preferably as a signal); the introduction of the nucleic acid construct into the cancer cells is carried out by administering the nucleic acid construct to the cancer patient; and the activity of protein A in blood separated from the patient after the administration of the nucleic acid construct is measured. Among the modes of use described later, (4) is a specific example of this mode. In this mode, the cancer patient is typically a solid cancer patient. The nucleic acid construct is preferably topically administered into the tumor or into the vicinity of the tumor of the cancer patient. In cases where the cancer of the patient is not deficient in homologous recombination, secretory protein A is expressed from the protein A gene reconstructed in the cancer cells, and secreted to the outside of the cancer cells. Therefore, the activity of protein A can be detected using a blood sample of the cancer patient. Since the activity of protein A secreted into the blood can be detected in vitro, a protein that generates a signal by reaction with a substrate may also be preferably used. Typical examples of such a protein include secretory luciferases. As the gene sequence encoding the secretory protein such as a secretory luciferase, a known secretory protein-coding sequence may be used as it is, or a gene sequence prepared by adding a sequence encoding a secretory signal to a protein-coding sequence may be used. As a negative control sample, for example, a blood sample collected from the patient before the administration of the nucleic acid construct may be used. Further, as a positive control, for example, a culture supernatant obtained by introducing the nucleic acid construct into a cell line having normal homologous recombination activity (the details are the same as those of the HR-proficient control cells described above) and culturing the resulting cells may be used. When the activity of protein A is detected in blood, the cancer of the cancer patient is indicated not to be a homologous recombination-deficient cancer. When the activity of protein A is not detected in blood, a possibility that the cancer of the patient is a homologous recombination-deficient cancer is indicated. In this mode, when a patient is diagnosed with a possible homologous recombination-deficient cancer, cancer cells may be collected from the patient, and the nucleic acid construct may be introduced into the cells to confirm that the cancer is a homologous recombination-deficient cancer.

To a cancer patient diagnosed with homologous recombination-deficient cancer by the diagnostic technique for homologous recombination-deficient cancer by the present invention, a DNA-damaging anticancer drug such as a PARP inhibitor or a platinum formulation may be preferably administered. To a cancer patient diagnosed as not having homologous recombination-deficient cancer, an anticancer drug other than a DNA-damaging anticancer drug may be preferably administered.

Examples of a mode of use of the diagnostic agent for homologous recombination-deficient cancer include, but are not limited to, the following modes.

(1) Cancer cells collected from a cancer patient are cultured, and the nucleic acid construct of the present invention is introduced into the cultured cancer cells, followed by investigating whether or not a signal of protein A is detected (or investigating whether or not a cytocidal effect by protein A is found). If desired, non-cancerous cells (such as peripheral blood cells) collected from the same cancer patient may be used as a control. In cases where protein A is a luciferase, a substrate of the luciferase is added for the detection reaction. In cases where protein A is a fluorescent protein, a fluorescence signal may be detected using a luminometer or the like. When the signal of protein A is detected (especially when the level of the detected signal is almost the same as that of control non-cancerous cells), the cancer of the patient can be judged not to be deficient in homologous recombination. When the signal of protein A is not detected, or when a signal lower than that of control non-cancerous cells is detected, the cancer of the patient can be judged to be deficient in homologous recombination.
   The method of measuring the cell survival rate is well known in the art, and the measurement can be easily carried out using a commercially available kit or the like. When cancer cells into which the nucleic acid construct has been introduced show a decreased survival rate (in particular, when the survival rate is as low as that of control non-cancerous cells), the cancer of the patient can be judged not to be deficient in homologous recombination. When no decrease in the survival rate of cancer cells is found, or when a decrease in the cell survival rate of cancer cells is slower than that of control non-cancerous cells, the cancer of the patient can be judged to be deficient in homologous recombination.
(2) The nucleic acid construct of the present invention is administered to a patient, and whether or not a signal of protein A is detected from a cancer lesion is investigated. When the signal is detected, the cancer of the patient can be judged not to be deficient in homologous recombination. When the signal is not detected, a possibility that the cancer of the patient is deficient in homologous recombination is indicated. In this mode, a protein whose intracellular expression is detectable as a signal is used as protein A, and biotoxicity of the protein employed needs to be sufficiently low. The administration of the nucleic acid construct to the patient is preferably carried out by topical administration into the tumor or into the vicinity of the tumor.
(3) Cancer cells present in blood are isolated from a patient, and then concentrated when necessary, followed by introducing the nucleic acid construct of the present invention into the cells and investigating whether or not a signal of protein A is detected (or whether or not a cytocidal effect due to protein A is found) (in cases of leukemia or using CTCs).
(4) A nucleic acid construct that employs a gene sequence encoding a secretory luciferase as the gene sequence encoding protein A is topically administered into the tumor or into the vicinity of the tumor of the patient. Thereafter, a blood sample is collected, and the presence or absence of luciferase reaction is investigated using the blood sample. In cases where the cancer of the patient is not deficient in homologous recombination, the secretory luciferase is produced from the luciferase gene reconstructed in the cancer cells, and secreted to the outside of the cells. Thus, the luciferase reaction can be detected using the blood sample. In cases where the cancer of the patient is deficient in homologous recombination, the secretory luciferase is not produced, so that the luciferase reaction is not detected in the blood sample.

The nucleic acid construct of the present invention can also be used, for example, as a detection agent for homologous recombination-restored cancer cells. A homologous recombination-restored cancer is a cancer which was once deficient in homologous recombination but has restored its homologous recombination activity as a result of therapeutic treatment or the like. Typical examples of the homologous recombination-restored cancer include cancers that acquired resistance to treatment by a PARP inhibitor (PARP-inhibitor-resistant cancers). The term "homologous recombination-restored cancer" also includes cancers whose *BRCA1* deficiency has been restored (by reverse mutation) to have normalized *BRCA1*. In this mode of use, as the gene sequence encoding protein A, a gene sequence encoding a protein whose intracellular expression is detectable may be preferably used. The specific mode of use is the same as the mode of use of the diagnostic agent for homologous recombination-deficient cancer, but the target cancer patient may be mainly a patient under treatment with a PARP inhibitor or a patient who was once diagnosed with homologous recombination-deficient cancer. The nucleic acid construct of the present invention may be introduced into cancer cells of the patient by the same method as the above-described diagnostic technique for homologous recombination-deficient cancer, and the expression of protein A, preferably the activity of protein A, may be measured. Similarly to the diagnostic method for homologous recombination-deficient cancer, control cells (HR-proficient control cells or HR-deficient control cells, or both) may be used in the detection method for homologous recombination-restored cancer cells. When expression or activity of protein A is detected, it can be judged that homologous recombination-restored cancer cells are detected. In a method of using the detection agent, for example, homologous recombination activity in homologous recombination-deficient cancer cells before restoration of homologous recombination may be measured in advance using a measurement reagent or kit for homologous recombination activity according to the present invention, and then whether or not the homologous recombination is restored may be judged by comparison with this measured value. When the homologous recombination activity is increased compared to the past measured value, in other words, when the protein A expression level or activity level is increased compared to the protein A expression level or activity level in the cancer cells measured in the cancer patient using the nucleic acid construct of the present invention in the past, it is indicated that the homologous recombination activity has been restored. For a patient diagnosed with homologous recombination-restored cancer, for example, administration of a PARP inhibitor may be stopped, and treatment with another anticancer drug may be started instead (in cases where the patient is under treatment with the PARP inhibitor). One option of the other anticancer drug is the later-described therapeutic agent of the present invention for homologous recombination-restored cancer. For a patient diagnosed as not having homologous recombination-restored cancer, for example, treatment with the same anticancer drug may be continued, or treatment with another anticancer drug may be started instead.

The nucleic acid construct of the present invention can also be used, for example, as a companion diagnostic agent for predicting the effect of an anticancer drug on homologous recombination-deficient cancer. Examples of the anticancer drug include DNA-damaging anticancer drugs including PARP inhibitors. Specific examples of modes of the use as a companion diagnostic agent include those described above for the diagnostic agent for homologous recombination-deficient cancer. As the gene sequence encoding protein A, a gene sequence encoding a protein whose intracellular expression is detectable may be preferably used, and a gene sequence encoding a protein that acts to decrease the cell survival rate may also be used. In cases where a patient is diagnosed with homologous recombination-deficient cancer, the patient can be judged to be a patient for whom a desired anticancer effect is likely to be obtained with a DNA-damaging anticancer drug such as a PARP inhibitor. Thus, to such a cancer patient, a DNA-damaging anticancer drug may be preferably administered.

DNA-damaging anticancer drugs include inhibitors of proteins having a synthetic lethal (or synthetic growth delay) relationship with homologous recombination, and agents that induce DNA damage repairable by homologous recombination. Examples of the former include inhibitors of proteins such as PARP, PolQ, and RAD52, and examples of the latter include camptothecin, cisplatin, and PARP inhibitors, although they are not limited to these.

Specific examples of the PARP inhibitors include, but are not limited to, Olaparib, Rucaparib, Niraparib, Veliparib, Talazoparib, PJ34 (Visochek et al., Oncotarget, 2019, Vol. 10, (No. 58), pp: 6269-6282), and NU1025 (CAS 90417-38-2).

Regarding the PolQ inhibitors, Novobiocin (Zhou et al., Nature Cancer volume 2, pages 598-610 (2021)) and ART558 (Zatreanu et al., Nature Communications volume 12, Article number: 3636 (2021)) have been reported to have PolQ-specific inhibitory activities. Further, a PolQ inhibitor can be obtained by, for example, the method described in JP 2017-201978 A. The PolQ inhibitors also include a variety of PolQ inhibitors that may be discovered in the future by this method, or by methods different therefrom.

Examples of the RAD52 inhibitors include, but are not limited to, AICAR (CAS 2627-69-2), AICAR 50 phosphate (ZMP), 6-Hydroxy-DL-DOPA (CAS 21373-30-8), D-103, D-G23, (-)-Epigallocatechin, EGC (CAS 490-46-0), and NP-004255 (CAS 23094-69-1) (Hengel SR et al. Cell Chem Biol. 2017 Sep 21;24(9): 1101-1119.).

Further examples of the DNA-damaging anticancer drugs include inhibitors related to DDR, which is a target of synthetic lethality (see, for example, Gourley C et al. J Clin Oncol. 2019 Sep 1;37(25):2257-2269., Gourley C et al. J Clin Oncol. 2019 Sep 1;37(25):2257-2269, and Ashworth A and Lord CJ. Nat Rev Clin Oncol. 2018 Sep;15(9):564-576.). Specific examples thereof include, but are not limited to, CBP-501, Prexasertib, GDC-0575, and SRA-737, which target CHK1/2; AZD-1775, which targets WEE1; AZD-6738, M-4344, and M6620 (VX-970), which target ATR; CC-115, LY-3023414, AsiDNA, and M-3814, which target DNA-PK; and AZD-0156, which targets ATM (Gourley C et al., 2019 (described above)).

Further, specific examples of the DNA-damaging anticancer drugs include platinum formulations such as cisplatin, carboplatin, and oxaliplatin (DNA synthesis inhibition); pyrimidine-based drugs such as fluorouracil and gemcitabine (DNA synthesis inhibition); camptothecin-based drugs such as irinotecan and topotecan (DNA synthesis inhibition); epipodophyllotoxin-based drugs such as etoposide (DNA synthesis inhibition); anthracycline-based drugs such as doxorubicin, epirubicin, pirarubicin (DNA synthesis inhibition); and alkylating agents such as cyclophosphamide and ifosfamide (DNA synthesis inhibition). The term "DNA-damaging anticancer drugs" may also include vinca alkaloid-based drugs such as vinblastine, vincristine, vindesine, and vinorelbine (cell division inhibition); and taxane-based drugs such as paclitaxel and docetaxel (apoptosis-inducing agents). However, the DNA-damaging anticancer drugs are not limited to the examples described above.

The nucleic acid construct of the present invention can also be used, for example, as a therapeutic agent for homologous recombination-restored cancer. The definition of the homologous recombination-restored cancer is as described above, and it includes cancers that acquired resistance to treatment with a PARP inhibitor (PARP inhibitor-resistant cancers) and cancers whose BRCA1 deficiency has been restored (by reverse mutation) to have normalized BRCA1. In this mode of use, the gene sequence encoding protein A is a gene sequence encoding a protein that acts to decrease the cell survival rate. By the nucleic acid construct of the present invention, cancer cells having homologous recombination activity can be killed by the action of protein A. Therefore, the construct enables treatment of homologous recombination-restored cancer such as a PARP inhibitor-resistant cancer, for which no treatment method is available at present.

The nucleic acid construct of the present invention may also be used for, for example, determining or predicting whether or not a gene mutation (especially a mutation in an HR-related gene) identified in a cancer patient is a pathogenic mutation that deteriorates homologous recombination activity.

When a mutation is identified in a certain gene X (the gene X is preferably an HR-related gene) in cancer cells of a cancer patient, first, an expression vector that expresses a mutant gene X having the same mutation is constructed. The mutant gene X can be obtained by extracting mRNA from cancer cells collected from the cancer patient, and performing reverse transcription PCR using a primer set targeting the gene X. In addition, an expression vector that expresses a wild-type gene X having no mutation is also provided. The wild-type gene X can be prepared from, for example, a known cell line having normal homologous recombination activity (for details, see the above description on the HR-proficient control cells), or non-cancerous cells of the patient. In cases where an expression vector that expresses the wild-type gene X is already known, the known expression vector may be used without newly constructing an expression vector.

Subsequently, each of the mutant gene X expression vector and the wild-type gene X expression vector is introduced into cells deficient in gene X, and the nucleic acid construct of the present invention is further introduced thereto. The introduction of each expression vector and the introduction of the nucleic acid construct may be carried out in an arbitrary order. Cells deficient in gene X can be prepared by knocking out gene X in cells having normal homologous recombination activity. Since the technique for knockout of a specific gene has been established, those skilled in the art can easily prepare gene X knockout cells using a well-known technique. Or, when there is a known cell line whose function of gene X is known to be completely deficient, such a known cell line may be used.

Subsequently, in cells in which each expression vector and the nucleic acid construct are introduced, expression of protein A is measured. In addition, measurement of expression of protein A may be carried out also in cells prepared by introducing the nucleic acid construct of the present invention into gene X-deficient cells in which an empty vector comprising no insert is introduced, or into gene X-deficient cells in which no vector is introduced. As described above, the measurement of expression of protein A is preferably measurement of the activity of protein A. Based on the expression level of protein A, whether or not the mutation in gene X is a pathogenic mutation (whether or not the mutation causes deficiency of homologous recombination activity) can be judged. This judgment can be carried out according to the same criteria as in the above-described measurement technique for homologous recombination activity. When the expression level or activity level of protein A in the cells in which the mutant gene X is introduced is lower than the expression level or activity level of protein A in the cells in which the wild-type gene X is introduced, the mutation in gene X is indicated to be a pathogenic mutation that deteriorates homologous recombination activity.

In this pathogenic-mutation prediction technique, as protein A, a protein whose intracellular expression is detectable preferably as a signal may be preferably used, but a protein that acts to decrease the cell survival rate may also be used.

In cases where the agent of the present invention is administered to a patient, the administration route may be oral administration or parenteral administration. In general, parenteral administration such as intravenous administration, intraarterial administration, subcutaneous administration, or intramuscular administration is preferred. The administration may be systemic administration, administration into the tumor or into the vicinity of the tumor, or administration to a regional lymph node of the tumor. However, in cases of a therapeutic agent for homologous recombination-restored cancer, the agent is preferably used by topical administration into the tumor or into the vicinity of the tumor since its systemic administration causes production of the cytocidal effect also on normal non-cancerous cells having homologous recombination activity. The administration of the agent comprising the nucleic acid construct of the present invention as an effective component means administration of the first nucleic acid molecule and the second nucleic acid molecule, and these nucleic acid molecules are preferably administered simultaneously or sequentially. As described above, in cases of the sequential administration, either nucleic acid molecule may be administered prior to the other. The dose is not limited, and may be about 1 pg to 10 g, for example, about 0.01 mg to 100 mg, in terms of the total amount of the nucleic acid molecules (first nucleic acid molecules + second nucleic acid molecules) per day for the patient. The doses of the first nucleic acid molecule and the second nucleic acid molecule in terms of the molar ratio, i.e., first nucleic acid molecules : second nucleic acid molecules, may be about 1:0.001 to 1000, about 1:0.01 to 100, or about 1:0.1 to 10, or may be about 1:0.2 to 5, or about 1:0.5 to 2. The agent of the present invention may be administered in a single dose or several divided doses per day. The agent may be administered every day, or may be administered every several days or every several weeks.

The dosage form of the agent of the present invention to be administered to the patient is not limited. Depending on the administration route, the agent may be prepared by appropriately mixing the nucleic acid construct of the present invention with an additive(s) such as a pharmaceutically acceptable carrier, diluent, or excipient. Examples of the formulation include parenteral formulations such as drops, injection solutions, suppositories, and inhalants; and oral formulations such as tablets, capsules, granules, powders, and syrups. Preparation methods and additives that can be used are well known in the field of pharmaceutical formulations, and any of the methods and additives may be used. The agent of the present invention may be in the form of the set of a formulation comprising the first nucleic acid molecule and a formulation comprising the second nucleic acid molecule, or may be in the form of a formulation comprising both the first nucleic acid molecule and the second nucleic acid molecule.

In cases of a diagnostic agent, a companion diagnostic agent, or a detection agent to be used in vitro, the amount of the agent used for the treatment of the cells may be, for example, about 10 pg to 1 mg, or for example, about 0.1 ng to 100 µg, in terms of the total amount of the nucleic acid molecules (first nucleic acid molecules + second nucleic acid molecules) per 1,000,000 cells. The ratio between the amounts of the first nucleic acid molecule and the second nucleic acid molecule used in terms of the molar ratio, i.e., first nucleic acid molecules : second nucleic acid molecules, may be about 1:0.001 to 1000, about 1:0.01 to 100, or about 1:0.1 to 10, or may be about 1:0.2 to 5, or about 1:0.5 to 2. The dosage form may be a liquid agent supplemented, when desired, with an additive(s) or the like useful for stability or the like of the nucleic acid construct, or may be a powder agent prepared by freeze-drying of the nucleic acid construct. Also in cases of an agent to be used in vitro, the agent may be in the form of the set of a formulation comprising the first nucleic acid molecule and a formulation comprising the second nucleic acid molecule, or may be in the form of a formulation comprising both the first nucleic acid molecule and the second nucleic acid molecule.

In cases where the nucleic acid construct of the present invention is used as a therapeutic agent or a diagnostic agent, examples of the target cancer patient include cancer patients of various mammals such as humans, dogs, cats, ferrets, hamsters, mice, rats, horses, and pigs. The nucleic acid construct of the present invention is applicable to various animal species without changing the components except that the origin of the promoter is changed when necessary.

### EXAMPLES

The present invention is described below more concretely by way of Examples. However, the present invention is not limited to the following Examples. In the following Examples, the size of each DNA fragment may be expressed as the size excluding the stop codon added to the 3'-end.

### Example A: Divided-Type DR-Nluc Construct (Introduction of Both SceNluc + iNluc)

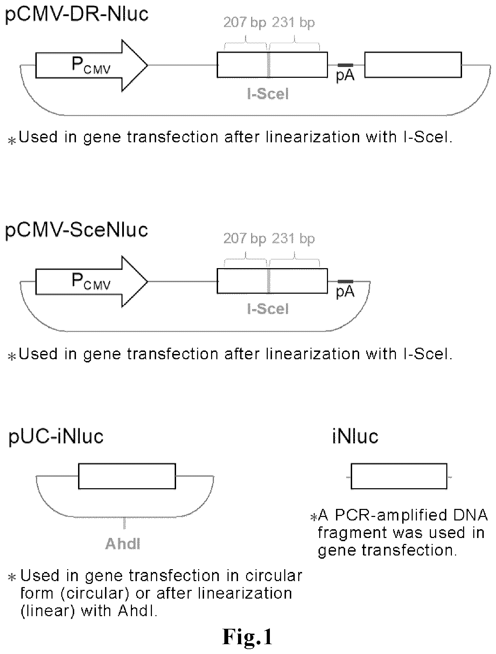

### A-1. Preparation of Vectors (Fig. 1)

### A-1-1. Divided-Type DR-Nluc (Fig. 1, Middle and Bottom Rows)

A 513-bp SceNluc fragment (SEQ ID NO:25; a DNA having a structure in which the region of positions 223 to 243 in the *Nluc* gene sequence (SEQ ID NO:23) was replaced with "stop codon (TGA) + I-SceI recognition sequence", having a stop codon added to the 3'-end) to be used for pCMV-SceNluc and a 459-bp iNluc fragment (SEQ ID NO:28; the region of positions 16 to 474 in the *Nluc* gene (SEQ ID NO:23), having a stop codon added to the 3'-end) to be used for pUC-iNluc were obtained by PCR amplification using pNL1.1 [Nluc] Vector (Promega, Madison, WI, USA) as a template. As a polymerase for the PCR, PrimeSTAR HS (trade name) DNA Polymerase (Takara Bio Inc.) or Tks Gflex (trade name) DNA Polymerase (Takara Bio Inc.) was used. The primers used are shown below in Table 1. In the amplification of the SceNluc fragment, two fragments were separately amplified to obtain a 5' SceNluc fragment and a 3' SceNluc fragment. In the amplification of the 5'SceNluc fragment, Nluc-Fw (SEQ ID NO:1) and Sce-5'Nluc-Rv (SEQ ID NO:2) were used to amplify a DNA fragment having a structure in which a sequence for In-Fusion reaction was added to the 5'-end of the 5'SceNluc fragment, and in which "stop codon (TGA) + I-SceI recognition sequence" were added to the 3'-end of the 5' SceNluc fragment. In the amplification of the 3' SceNluc fragment, Sce-3'Nluc-Fw (SEQ ID NO:3) and Nluc-Rv (SEQ ID NO:4) were used to amplify a DNA fragment having a structure in which "stop codon (TGA) + I-SceI recognition sequence" were added to the 5'-end of the 3' SceNluc fragment, and in which a stop codon (TAA) and a sequence for In-Fusion reaction were added to the 3'-end of the 3' SceNluc fragment. In the reading frame of the SceNluc fragment (SEQ ID NO:25), there are the stop codon TGA introduced upstream of the I-SceI recognition sequence, and the stop codon TAA present inside the I-SceI recognition sequence. The amino acid sequences encoded by positions 1 to 225 (the region up to the first stop codon) and positions 235 to 516 (the region downstream of the second stop codon) of the SceNluc fragment are shown in SEQ ID NOs: 26 and 27, respectively.

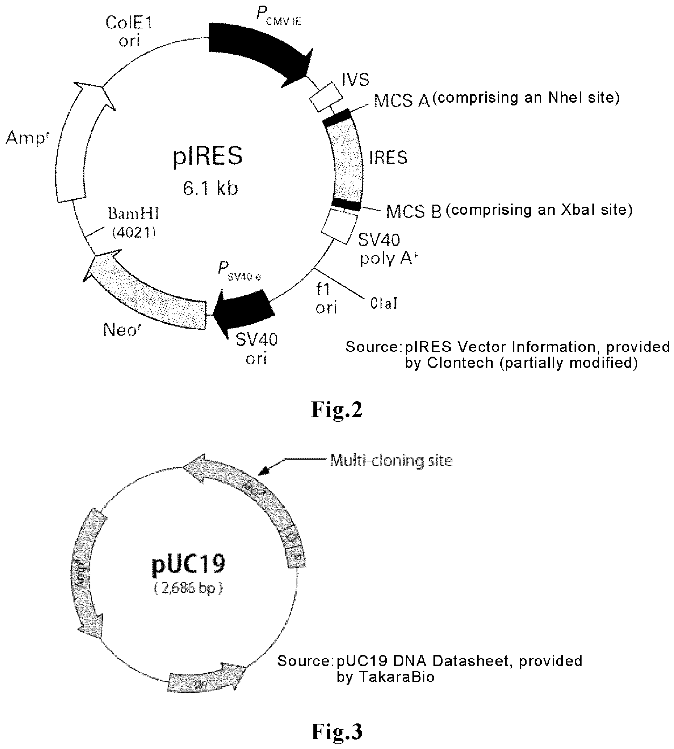

In the amplification of the iNluc fragment, iNluc-Fw (SEQ ID NO:5) and iNluc-Rv (SEQ ID NO:6) were used to amplify a DNA fragment having a structure in which a stop codon (TGA) was linked to the 3'-end of the iNluc fragment, and in which sequences for In-Fusion reaction were added to both ends. The iNluc fragment amplified by this PCR is a DNA fragment (SEQ ID NO:30) having a structure containing a 23-bp additional sequence in the 5'-end side, and a 22-bp additional sequence in the 3'-end side, of the 459-bp Nluc coding region. Subsequently, pIRES (Takara Bio Inc., Fig. 2) was digested with NheI and XbaI to remove the IRES region, and a fragment containing the CMV promoter and the poly-A addition signal was recovered. Using an In-Fusion HD Cloning Kit (Takara Bio Inc.), the 5'SceNluc fragment and the 3'SceNluc fragment were inserted downstream of the CMV promoter of the recovered fragment, to obtain pCMV-Sce-Nluc, in which [CMV promoter] - [SceNluc fragment] - [poly-A addition signal] were linked together. pUC-iNluc was obtained by digesting pUC19 (Takara Bio Inc., Fig. 3) at SmaI in the multicloning site, and inserting the iNluc fragment thereto using a DNA Ligation Kit <Mighty Mix> (Takara Bio Inc.).

The pCMV-SceNluc was purified using a Qiagen Plasmid Plus Midi Kit (Qiagen K.K.), linearized by cleavage with I-SceI (New England Biolabs, Ipswich, MA, USA) and then used in transfection. The pUC-iNluc was purified using a Qiagen Plasmid Plus Midi Kit (Qiagen K.K.), and used in transfection as it was in the circular form, or after linearization by cleavage with AhdI (New England Biolabs). Cleavage of pUC-iNluc with AhdI results in production of a linear DNA containing an additional sequence with a size of 1.3 kbp in the 5'-end side, and an additional sequence with a size of 1.4 kbp in the 3'-end side, of the iNluc fragment. When the iNluc fragment amplified by PCR was used for transfection, it was purified using the Wizard SV Gel and PCR Clean-Up System (Promega) in advance.

### A-1-2. Integrated-Type DR-Nluc (pCMV-DR-Nluc; Fig. 1, Top Row)

pCMV-SceNluc, prepared in A-1-1, was digested with BamHI, and the iNluc fragment amplified by PCR was inserted downstream of poly-A using an In-Fusion HD Cloning Kit (Takara Bio Inc.), to obtain the integrated-type DR-Nluc construct pCMV-DR-Nluc, in which [CMV promoter] - [SceNluc fragment] - [poly-A addition signal] - [iNluc fragment] were linked together. The prepared plasmid was purified using a Qiagen Plasmid Plus Midi Kit (Qiagen K.K.), linearized by cleavage with I-SceI (New England Biolabs, Ipswich, MA, USA) and then used in transfection.

### A-1-3. shRNA Expression Vector

A target sequence to be used for an shRNA expression vector (pshRad51) for knockdown of RAD51 was designed using Enhanced siDirect (registered trademark) (RNAi Inc.). For an shRNA expression vector (pshControl) to be used as a control, a sequence reported in a past report (Bryant et al. (2010) A molecular network for de novo generation of the apical surface and lumen. Nat. Cell Biol. 11: 1035-1045) was used. Subsequently, single-stranded oligo DNAs (a top strand and a bottom strand) containing the array of [linker sequence for ligation] - [target sequence (sense)] - [loop sequence] - [target sequence (antisense)] - [terminator sequence] - [linker sequence for ligation] were annealed to obtain a double-stranded oligo DNA. The sequences of the single-stranded oligo DNAs used are shown below in Table 1. As shRNAs for targeting RAD51, shRad51-top (SEQ ID NO:7) and shRad51-btm (SEQ ID NO:8) were used. As shRNAs for control, shControl-top (SEQ ID NO:9) and shControl-btm (SEQ ID NO: 10) were used. Finally, pBAsi-hU6 Neo DNA (Takara Bio Inc.) was digested with BamHI and HindIII to excise a fragment containing the U6 promoter, and a double-stranded oligo DNA was inserted using a DNA Ligation Kit <Mighty Mix> (Takara Bio Inc.), to obtain pshRad51 and pshControl. The pshRad51 and the pshControl were purified using a Qiagen Plasmid Plus Midi Kit (Qiagen K.K.), and then used in transfection as they were in the circular form.

**[Table 1]**

| Primer name | Sequence (5'→3') | SEQ ID NO |
|---|---|---|
| Nluc-Fw | TAATACGACTCACTATAGGCTAG | 1 |
| Sce-5'Nluc-Rv | ATTACCCTGTTATCCCTA**TCA**CTGGCCCATTTGGTCGCCGCTC | 2 |
| Sce-3'Nluc-Fw | **TGA**TAGGGATAACAGGGTAATGTGTACCCTGTGGATGATCATCAC | 3 |
| Nluc-Rv | CCGCCCCGACTCTAGAGTCGCGG | 4 |
| iNluc-Fw | AAATCGATAAGGATCGCTAGCATGAAGATTTCGTTGGGGACTGG | 5 |
| iNluc-Rv | CACCATACGCGGATCCTTA**TCA**GTTGATGGTTACTCGGAACAGC | 6 |
| shRad51-top | GATCCGCGGTCAGAGATCATACAGATTCTGTGAAGCCACAGATGG GAATCTGTATGATCTCTGACCGCTTTTTTA | 7 |
| shRad51-btm | AGCTTAAAAAAGCGGTCAGAGATCATACAGATTCCCATCTGTGGC TTCACAGAATCTGTATGATCTCTGACCGCG | 8 |
| shControl-top | GATCCGCCTAAGGTTAAGTCGCCCTCTGTGAAGCCACAGATGGGA GGGCGACTTAACCTTAGGCTTTTTTA | 9 |
| shControl-btm | AGCTTAAAAAAGCCTAAGGTTAAGTCGCCCTCCCATCTGTGGCTT CACAGAGGGCGACTTAACCTTAGGCG | 10 |

| | | |
|---|---|---|
| Single-underline: I-SceI recognition sequence Double-underline: A site that anneals to the target gene sequence (Nluc-Fw and Nluc-Rv target vector sequences located upstream and downstream of the Nluc gene in pNL1.1 [Nluc] Vector, respectively.) Bold: Sequence for introduction of a stop codon (TGA) | | |

### A-2. Cells and Gene Transfection, and Luciferase Assay

### A-2-1. Cells and Gene Transfection

The human pre-B cell line Nalm-6 and cells derived therefrom were cultured at 37°C in a 5% CO₂ incubator (So et al. (2004) Genetic interactions between BLM and DNA ligase IV in human cells. J. Biol. Chem. 279: 55433-55442). Acquisition of the *RAD54*/*RAD54B*-double-deficient cells was carried out as described previously (Saito S, Kurosawa A, Adachi N. Mechanistic basis for increased human gene targeting by promoterless vectors-roles of homology arms and Rad54 paralogs. FEBS J. 2017 Sep;284(17):2748-2763. doi: 10. 1111/febs. 14137.). Transfection of the Nalm-6 cells and the cells derived therefrom was carried out by the electroporation method according to a past report (Saito et al. (2017) Dual loss of human POLQ and LIG4 abolishes random integration. Nat. Commun. 8: 16112).

The human breast cancer cell line MDA-MB-436 (ATCC, Manassas, VA, USA) was cultured at 37°C in a 5% CO₂ incubator. As the medium for the culture of MDA-MB-436, Eagle MEM (Nissui Pharmaceutical Co., Ltd., Tokyo, Japan) supplemented with calf serum (Global Life Science Technologies Japan, Tokyo, Japan; 50 ml of calf serum was added per 500 ml of Eagle MEM), L(+)-glutamine (Fujifilm Wako Pure Chemical Corporation, Osaka, Japan; which was added so that the final concentration should be 2 mM), an MEM non-essential amino acid solution (Fujifilm Wako Pure Chemical Corporation; 10 ml of the solution was added per 500 ml of Eagle MEM), 100 mmol/L sodium pyruvate solution (Fujifilm Wako Pure Chemical Corporation; which was added so that the final concentration should be 1 mM), vitamin B 12 (Sigma-Aldrich, St. Louis, MO, USA; which was added so that the final concentration should be 0.15 µM), and 2-mercaptoethanol (Fujifilm Wako Pure Chemical Corporation; which was added so that the final concentration should be 50 µM) was used.

The human fibrosarcoma-derived cell line HT1080 (Institute for Fermentation, Osaka, Japan) was cultured at 37°C in a 5% CO₂ incubator (Saito et al. (2015) Construction and applications of exon-trapping gene-targeting vectors with a novel strategy for negative selection. BMC Res. Notes 8: 278).

Transfection was carried out using jetPEI (Polyplus-transfection, Illkirch, France) according to a protocol provided by the manufacturer.

### A-2-2. Luciferase Assay

A luciferase assay was carried out as follows.

With each construct (1 µg), 2×10⁶ Nalm-6 cells (the wild line or the *RAD54*/*RAD54B*-double-deficient homologous recombination-deficient line) were transfected. The cells after the gene transfection were plated on a 12-well dish at 5×10⁵ cells/ml, and the luciferase activity (RLU value) was measured immediately after the gene transfection and at 4, 8 and 24 hours post-transfection using the Nano-Glo Luciferase Assay System (Promega).

A luciferase assay of MDA-MB-436 cells (a human breast cancer cell line, deficient in *BRCA1*) and HT1080 cells (a human fibrosarcoma cell line, which has normal homologous recombination activity) was carried out as follows. On a 24-well dish, 5×10⁴ cells were plated and cultured overnight, followed by transfecting the cells with each construct (1 µg). Four hours after the gene transfection, the number of cells was counted, and the luciferase activity (RLU value) was measured using the Nano-Glo Luciferase Assay System (Promega).

### A-3. Results

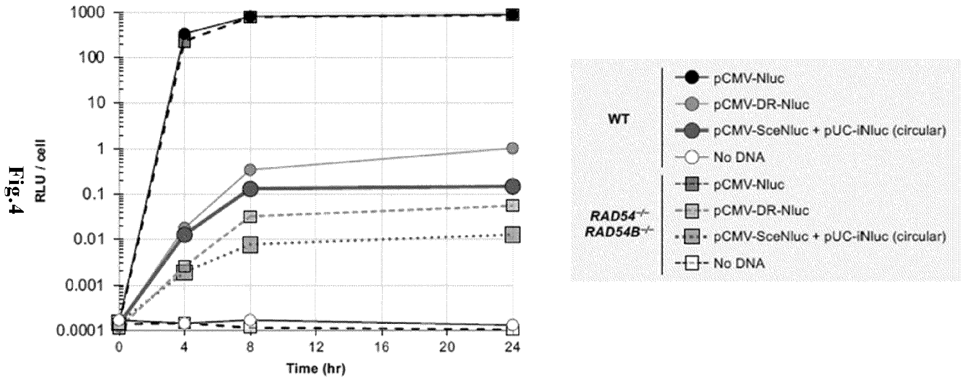

The luciferase activity after the introduction of each construct was measured over time for the wild line and the *RAD54*/*RAD54B*-double-deficient line of Nalm-6 cells. The results are shown in Fig. 4. pCMV-Nluc represents cells into which a construct that expressed the normal *Nluc* gene under the control by the CMV promoter was introduced (positive control). In the Nalm-6 cells of the wild line into which the integrated-type or divided-type construct was introduced, the luciferase activity increased in a short time after the gene transfection, and reached the plateau in about 8 hours. In the *RAD54*/*RAD54B*-double-deficient line (cells whose homologous recombination ability is lost), the luciferase activity detected after the transfection with the construct was lower than that in the wild line, and the value of RLU/cell at 24-hr post transfection, either in the integrated-type or divided-type, was not more than 1/10 compared to that in the wild line. These data suggest that the introduction of the integrated-type or divided-type DR-Nluc construct into the cells having homologous recombination activity allowed production of normal *Nluc* gene by homologous recombination that occurred between the SceNluc fragment and the iNluc fragment, resulting in transient expression of Nluc protein.

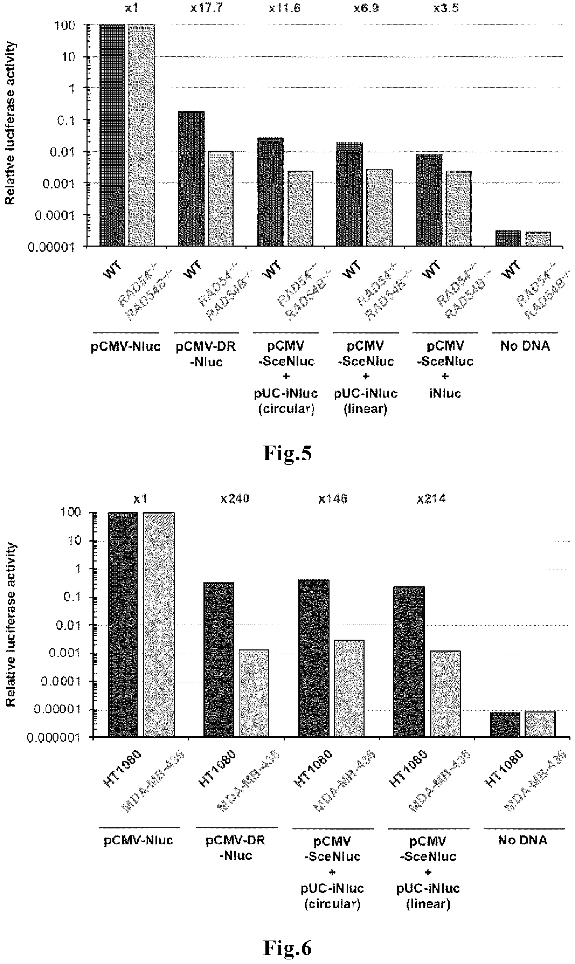

The result of the luciferase assay in Nalm-6 cells (comparison between the wild line and the homologous recombination-deficient line) is shown in Fig. 5. Each value is expressed as a relative value calculated by taking, as 100, the RLU value in the cells transfected with pCMV-Nluc. In the cells having homologous recombination activity, the normal *Nluc* gene sequence is reconstructed by homologous recombination between the SceNluc fragment and the iNluc fragment in the integrated-type construct, or between the divided-type constructs (between pCMV-SceNluc and pUC-iNluc or iNluc), resulting in detection of the luciferase activity, whereas, in the homologous recombination-deficient cells, reconstruction of the normal *Nluc* gene sequence by homologous recombination does not occur, and hence the luciferase activity is not detected (the detected level of the luciferase activity is lowered). As a result, a difference in the luciferase activity is expected to occur between those cells. Actually, as shown in Fig. 5, at the time of 4 hours after transfection with the constructs, the luciferase activity detected in the homologous recombination-deficient cells transfected with the integrated-type or the divided-type construct was lower than the luciferase activity detected in the wild-type cells transfected with the integrated-type or the divided-type construct, showing several-to ten-and-several-fold differences, although the activities varied depending on the structure of each construct. It was thus confirmed that homologous recombination activity of cells can be detected with divided-type constructs as well as with integrated-type constructs (claimed in a pending international application, PCT/JP2021/005381). Among the DNAs carrying the iNluc fragment, a DNA having longer terminal additional sequences (pCMV-SceNluc + iNluc vs. pCMV-SceNluc + pUC-iNluc (linear)), or a DNA having a circular shape (pCMV-SceNluc + pUC-iNluc (linear) vs. pCMV-SceNluc + pUC-iNluc (circular)) rather than a linear shape, showed a larger difference in the luciferase activity due to the presence or absence of the homologous recombination activity, indicating a higher detection performance for homologous recombination activity.

Fig. 6 shows the result of comparison of the luciferase activity between HT1080 cells (cells having normal homologous recombination activity) and MDA-MB-436 cells (cells deficient in homologous recombination activity due to *BRCA1* deficiency). Each value is expressed as a relative value calculated by taking, as 100, the RLU value in the cells transfected with pCMV-Nluc. In any cases using any of the constructs, the luciferase activity detected in the MDA-MB-436 cells, which are homologous recombination-deficient cells, was lower than the luciferase activity detected in the HT1080 cells, which have normal homologous recombination activity, showing 100-fold or more differences.

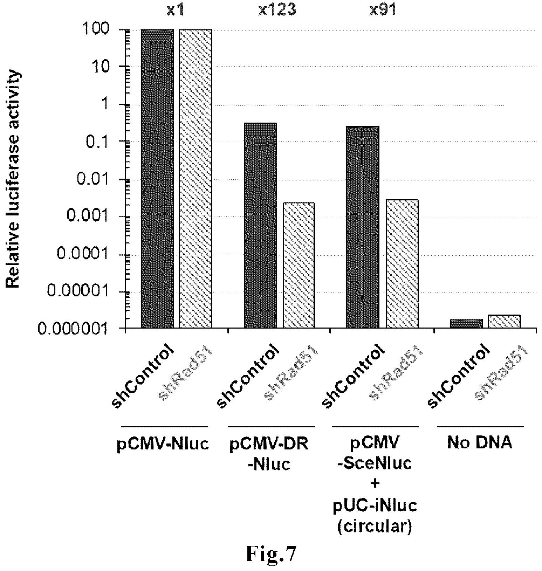

Fig. 7 shows the experimental result of *RAD51* gene knockdown in Nalm-6 cells. Each value is expressed as a relative value calculated by taking, as 100, the RLU value in the cells transfected with pCMV-Nluc. This result confirmed that both integrated-type and divided-type cause remarkable decreases in the detected luciferase activity when the gene expression of RAD51, which is an essential factor for homologous recombination, is suppressed. It was thus demonstrated that the reaction of the constructs of the present invention occurred through homologous recombination.

### Example B: Divided-Type DR-DTA Construct (Co-transfection with SceDTA + iDTA)

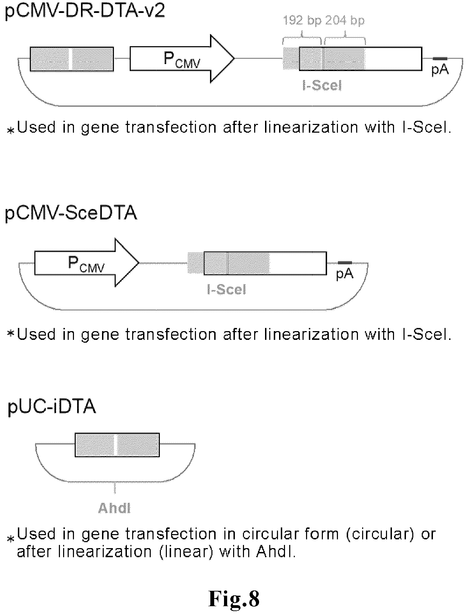

### B-1. Preparation of Vectors (Fig. 8)

### B-1-1. Divided-Type DR-DTA (Fig. 8, Middle and Bottom Rows)

A 588-bp SceDTA fragment (SEQ ID NO:33; a DNA having a structure in which the region of positions 133 to 153 in the *DT-A* gene ORF sequence (SEQ ID NO:31) was replaced with "stop codon (TGA) + I-SceI recognition sequence", having a stop codon added to the 3'-end) to be used for pCMV-SceDTA was obtained by PCR amplification using pMC1DT-ApA (KURABO, Osaka, Japan) as a template. As a polymerase for the PCR, PrimeSTAR HS (trade name) DNA Polymerase (Takara Bio Inc.) was used. The primers used are shown below in Table 2. In the amplification of the SceDTA fragment, two fragments were separately amplified to obtain a 5' SceDTA fragment and a 3' SceDTA fragment. In the amplification of the 5' SceDTA fragment, DTA-Fw (SEQ ID NO: 11) and Sce-5'DTA-Rv (SEQ ID NO: 12) were used to amplify a DNA fragment having a structure in which a sequence for In-Fusion reaction was added to the 5'-end of the 5' SceDTA fragment, and in which "stop codon (TGA) + I-SceI recognition sequence" were added to the 3'-end of the 5' SceDTA fragment. In the amplification of the 3' SceDTA fragment, Sce-3'DTA-Fw (SEQ ID NO: 13) and DTA-Rv (SEQ ID NO: 15) were used to amplify a DNA fragment having a structure in which "stop codon (TGA) + I-SceI recognition sequence" were added to the 5'-end of the 3' SceDTA fragment, and in which a stop codon (TGA) and a sequence for In-Fusion reaction were added to the 3'-end of the 3' SceDTA fragment. Subsequently, pIRES (Takara Bio Inc., Fig. 2) was digested with NheI and XbaI to remove the IRES region, and a fragment containing the CMV promoter and the poly-A addition signal was recovered. Using an In-Fusion HD Cloning Kit (Takara Bio Inc.), the 5' SceDTA fragment and the 3' SceDTA fragment were inserted downstream of the CMV promoter of the recovered fragment, to obtain pCMV-SceDTA, in which [CMV promoter] - [SceDTA fragment] - [poly-A addition signal] were linked together. In the reading frame of the SceDTA fragment (SEQ ID NO:33), there are the stop codon TGA introduced upstream of the I-SceI recognition sequence, and the stop codon TAA present inside the I-SceI recognition sequence. The amino acid sequences encoded by positions 1 to 135 (the region up to the first stop codon) and positions 145 to 591 (the region downstream of the second stop codon) of the SceDTA fragment are shown in SEQ ID NOs: 34 and 35, respectively.

A *DT-A* gene fragment of 588 bp + stop codon to be used for pCMV-DTA (SEQ ID NO:31) was obtained by PCR amplification using pMC1DT-ApA (KURABO, Osaka, Japan) as a template. As a polymerase for the PCR, PrimeSTAR HS (trade name) DNA Polymerase (Takara Bio Inc.) was used. By using DTA-Fw (SEQ ID NO: 11) and DTA-Rv (SEQ ID NO: 14) as primers, a DNA fragment containing sequences for In-Fusion reaction added to the 5'-end and the 3'-end was amplified. Subsequently, pIRES (Takara Bio Inc.) was digested with NheI and XbaI to remove the IRES region, and a fragment containing the CMV promoter and the poly-A addition signal was recovered. Using an In-Fusion HD Cloning Kit (Takara Bio Inc.), the *DT-A* gene fragment was inserted downstream of the CMV promoter of the recovered fragment, to obtain pCMV-DTA, in which [CMV promoter] - [*DT-A* gene] - [poly-A addition signal] were linked together.

An iDTA fragment of 417 bp + stop codon having a region homologous to pCMV-SceDTA (SEQ ID NO:45; the 60-bp vector sequence upstream of the SceDTA fragment in pCMV-SceDTA + the region of positions 1 to 357 in the *DT-A* gene (SEQ ID NO:31) + stop codon) was obtained by PCR amplification using pCMV-DTA as a template. As a polymerase for the PCR, PrimeSTAR HS (trade name) DNA Polymerase (Takara Bio Inc.) or Tks Gflex (trade name) DNA Polymerase (Takara Bio Inc.) was used. iDTA-Fw (SEQ ID NO: 15) and iDTA-Rv (SEQ ID NO: 16) were used as PCR primers to obtain an iDTA fragment (SEQ ID NO:45) having a structure in which the 60-bp vector sequence was added to the 5'-end of the base sequence of the region of positions 1 to 357 in the *DT-A* gene + stop codon (SEQ ID NO:36). Subsequently, pUC19 (Takara Bio Inc., Fig. 3) was digested at SmaI in the multicloning site, and the iDTA fragment was inserted thereto using a DNA Ligation Kit <Mighty Mix> (Takara Bio Inc.), to obtain pUC-iDTA.

The pCMV-SceDTA was purified using a Qiagen Plasmid Plus Midi Kit (Qiagen K.K.), linearized by cleavage with I-SceI (New England Biolabs, Ipswich, MA, USA) and then used in transfection. The pUC-iDTA was purified using a Qiagen Plasmid Plus Midi Kit (Qiagen K.K.), and then used in transfection as it was in the circular form.

### B-1-2. Integrated-Type DR-DTA (pCMV-DR-DTA-v2; Fig. 8, Top Row)

The pCMV-SceDTA prepared in B-1-1 was digested with BglII, and the iDTA fragment was inserted upstream of the CMV promoter using an In-Fusion HD Cloning Kit (Takara Bio Inc.), to obtain pCMV-DR-DTA-v2, in which [iDTA fragment] - [CMV promoter] - [SceDTA fragment] - [poly-A addition signal] were linked together. The prepared plasmid was purified using a Qiagen Plasmid Plus Midi Kit (Qiagen K.K.), linearized by cleavage with I-SceI (New England Biolabs) and then used in transfection.

### B-2. Evaluation of Cytocidal Effect

Evaluation of the cytocidal effect in Nalm-6 cells was carried out as follows.

With each construct (1 µg), 2×10⁶ Nalm-6 cells (the wild line or the *RAD54*/*RAD54B*-double-deficient line) were transfected, and the cells were plated on a 24-well dish at 1×10⁵ cells/ml, followed by measurement of the cell survival rate 96 hours later using CellTiter-Glo (CellTiter-Glo (registered trademark) Luminescent Cell Viability Assay, Promega).

Evaluation of the cytocidal effects in MDA-MB-436 and HT1080 cells was carried out as follows.

On a 24-well dish, 5×10⁴ cells were plated and cultured overnight, followed by transfecting the cells with each construct (1 µg). After 96 hours of culture, the cell survival rate was measured using CellTiter-Glo (CellTiter-Glo (registered trademark) Luminescent Cell Viability Assay, Promega).

**[Table 2]**

| Primer name | Sequence (5'→3') | SEQ ID NO |
|---|---|---|
| DTA-Fw | ACTCACTATAGGCTAGCGTCCTCGCCATGGATCCTGATGATGTTG | 11 |
| Sce-5'DTA-Rv | ATTACCCTGTTATCCCTA**TCA**TTGTGTACCAGATTTTGGC | 12 |
| Sce-3'DTA-Fw | TAGGGATAACAGGGTAATAAAGGGTTTTATAGTACCGACAA | 13 |
| DTA-Rv | CCGCCCCGACTCTAGAA**TCA**CAAAGATCGCCTGACACGATTTCCTG | 14 |
| iDTA-Fw | AAATGTGGTAAAATCGATGAATTACAGCTCTTAAGGCTAGAG | 15 |
| iDTA-Rv | ACGCGGATCCTTATCG**TCA**GACTTGCTCCATCAACGGTTCAG | 16 |

| | | |
|---|---|---|
| Single-underline: I-SceI recognition sequence Double-underline: A site that anneals to the target gene sequence (iDTA-Fw and iDTA-Fw2 target vector sequences located upstream of the DT-A gene in pMC1DT-ApA.) Bold: Sequence for introduction of a stop codon (TGA) | | |

### B-3. Results

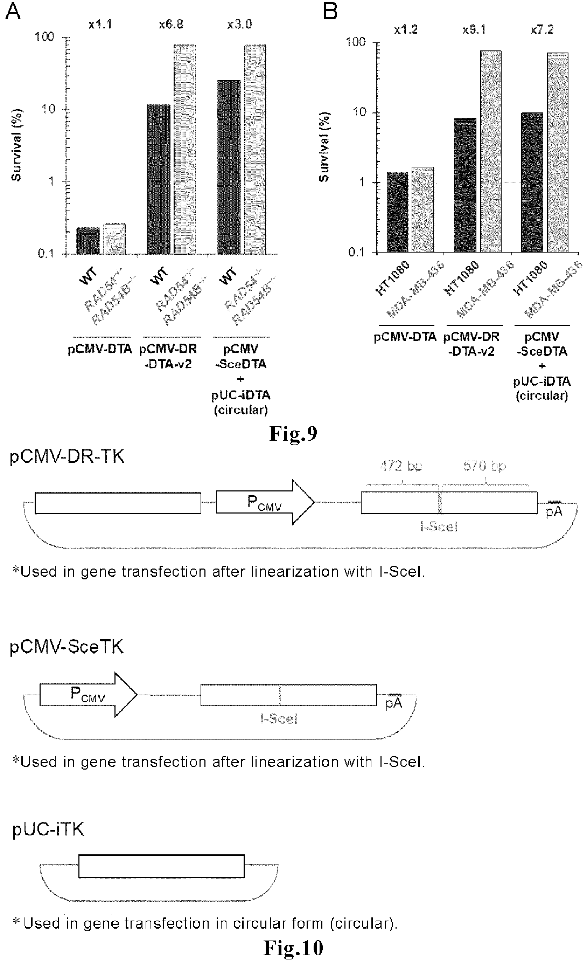

Fig. 9A shows a result of comparison of the survival rate 96 hours after the transfection of the construct, between the wild line and the *RAD54*/*RAD54B*-double-deficient line of Nalm-6 cells. Fig. 9B shows a result of comparison of the survival rate 96 hours after the transfection of each construct, between the human tumor-derived cell line HT1080, which is proficient in homologous recombination, and the human tumor-derived cell line MDA-MB-436, which is deficient in homologous recombination activity due to *BRCA1* deficiency. Similarly to the integrated-type construct, the divided-type construct carrying a suicide gene was confirmed to be capable of selectively killing cells having normal homologous recombination activity.

### Example C: Divided-Type DR-TK Construct (Co-transfection with SceTK + iTK)

### C-1. Preparation of Vectors

### C-1-1. Divided-Type DR-TK (Fig. 10, Middle and Bottom Rows)

A 1128-bp SceTK fragment (SEQ ID NO:40; a DNA having a structure in which the region of positions 499 to 519 in the ORF sequence (SEQ ID NO:38) of the *HSV-TK* gene was replaced with "stop codon (TGA) + I-SceI recognition sequence", having a stop codon added to the 3'-end) to be used for pCMV-SceTK was obtained by PCR amplification using an *HSV-TK* gene fragment (GenBank: V00470.1, Kobayashi et al. (2001) Decreased topoisomerase IIalpha expression confers increased resistance to ICRF-193 as well as VP-16 in mouse embryonic stem cells. Cancer Lett. 166(1): 71-77) as a template. As a polymerase for the PCR, PrimeSTAR HS (trade name) DNA Polymerase (Takara Bio Inc.) was used. The primers used are shown below in Table 3. In the amplification of the SceTK fragment, two fragments were separately amplified to obtain a 5'SceTK fragment, and a 3' SceTK fragment containing the poly-A addition signal. In the amplification of the 5'SceTK fragment, Sce-5'TK-Fw (SEQ ID NO:17) and Sce-5'TK-Rv (SEQ ID NO:18) were used to amplify a DNA fragment having a structure in which a sequence for In-Fusion reaction was added to the 5'-end of the 5'SceTK fragment, and in which a stop codon (TGA) was added to the 3'-end of the 5'SceTK fragment. In the amplification of the 3'SceTK fragment, Sce-3'TK-Fw (SEQ ID NO: 19) and Sce-3'TK-Rv (SEQ ID NO:20) were used to amplify a DNA fragment having a structure in which an I-SceI recognition sequence was added to the 5'-end of the 3'SceTK fragment, and in which a sequence for In-Fusion reaction was added to the 3'-end of the 3'SceTK fragment. Subsequently, pIRES (Takara Bio Inc., Fig. 2) was digested with NheI and BamHI to excise a fragment containing the CMV promoter. Using an In-Fusion HD Cloning Kit (Takara Bio Inc.), the 5'SceTK fragment and the 3'SceTK fragment were inserted downstream of the CMV promoter in the excised fragment, to obtain pCMV-SceTK, in which [CMV promoter] - [SceTK fragment] - [poly-A addition signal] were linked together. In the reading frame of the SceTK fragment (SEQ ID NO:40), there are the stop codon TGA introduced upstream of the I-SceI recognition sequence, and the stop codon TAA present inside the I-SceI recognition sequence. The amino acid sequences encoded by positions 1 to 501 (the region up to the first stop codon) and positions 511 to 1131 (the region downstream of the second stop codon) of the SceTK fragment are shown in SEQ ID NOs: 41 and 42, respectively.

An iTK fragment (1063 bp, SEQ ID NO:43; the region of positions 27 to 1089 in the ORF sequence of the *HSV-TK* gene), which is homologous to pCMV-SceTK, was obtained by PCR amplification using an *HSV-TK* gene fragment as a template. As a polymerase for the PCR, PrimeSTAR HS (trade name) DNA Polymerase (Takara Bio Inc.) was used. By using iTK-Fw (SEQ ID NO:21) and iTK-Rv (SEQ ID NO:22) as PCR primers, a DNA fragment having a structure in which sequences for In-Fusion reaction were added to both ends was amplified. Subsequently, pUC19 (Takara Bio Inc.) was digested at SmaI in the multicloning site, and the iTK fragment was inserted thereto using a DNA Ligation Kit <Mighty Mix> (Takara Bio Inc.), to obtain pUC-iTK.

The pCMV-SceTK was purified using a Qiagen Plasmid Plus Midi Kit (Qiagen K.K.), linearized by cleavage with I-SceI (New England Biolabs, Ipswich, MA, USA) and then used in transfection. The pUC-iTK was purified using a Qiagen Plasmid Plus Midi Kit (Qiagen K.K.), and then used in transfection as it was in the circular form.

### C-1-2. Integrated-Type DR-TK (pCMV-DR-TK; Fig. 10, Top Row)

The pCMV-SceTK prepared in B-1-1 was digested with BglII, and the iTK fragment was inserted upstream of the CMV promoter using an In-Fusion HD Cloning Kit (Takara Bio Inc.), to obtain pCMV-DR-TK, in which [iTK fragment] - [CMV promoter] - [SceTK fragment] - [poly-A addition signal] were linked together. The prepared plasmid was purified using a Qiagen Plasmid Plus Midi Kit (Qiagen K.K.), linearized by cleavage with I-SceI (New England Biolabs) and then used in transfection.

**[Table 3]**

| Primer name | Sequence (5'→3') | SEQ ID NO |
|---|---|---|
| Sce-5'TK-Fw | CTTAATACGACTCACTATAGGCTAGC | 17 |
| Sce-5'TK-Rv | TTACCCTGTTATCCCTA**TCA**GATGGGATGGCGGTCGAAGATGAG | 18 |
| Sce-3'TK-Fw | GATAGGGATAACAGGGTAATGCCGCGCGGTACCTTATGG | 19 |
| Sce-3'TK-Rv | GAGTGCACCATACGCGGATC | 20 |
| iTK-Fw | ACATGGCTCGACAGATCTATGCGCGTCTGCGTTCGACCAGG | 21 |
| iTK-Rv | GGCCAATATTGAAGATCTGTCGCATATCGTCGGTATGG | 22 |

| | | |
|---|---|---|
| Single-underline: I-SceI recognition sequence Double-underline: A site that anneals to the target gene sequence (Sce-5'TK-Fw and Sce-3'TK-Rv target vector sequences located upstream and downstream of the HSV-TK gene fragment, respectively.) Bold: Sequence for introduction of a stop codon (TGA) | | |

### C-2. Evaluation of Cytocidal Effect

Evaluation of the cytocidal effects in MDA-MB-436 and HT1080 cells was carried out as follows.

On a 24-well dish, 5×10⁴ cells were plated and cultured overnight, followed by transfecting the cells with each construct (1 µg). After the transfection, ganciclovir (GANC; Fujifilm Wako Pure Chemical Corporation) was added to a final concentration of 500 nM, and culture was performed for 96 hours, followed by measurement of the cell survival rate using CellTiter-Glo (CellTiter-Glo (registered trademark) Luminescent Cell Viability Assay, Promega).

### C-3. Results

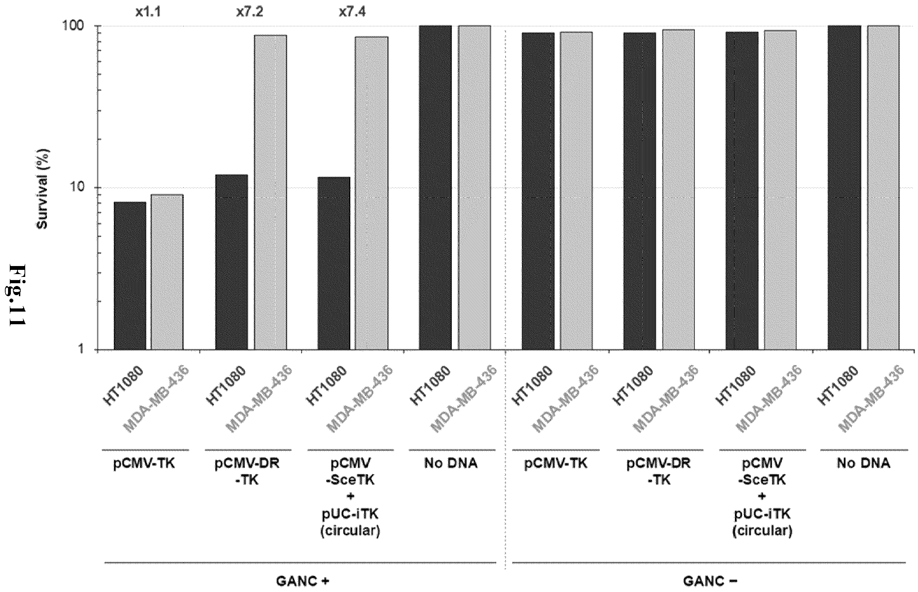

Fig. 11 shows a result of comparison of the cytocidal effects of each construct in MDA-MB-436 and HT1080 cells. The survival rate is expressed as a relative value calculated by taking, as 100%, the survival rate of cells not transfected with DNA (No DNA) cultured in the presence of GANC (500 nM). HSV-TK acts on GANC and FIAU to convert them to toxic substances having DNA synthesis inhibitory activity, to exert the cytocidal action. This result confirmed that, also in cases where HSV-TK is used as a suicide gene, both the integrated-type and the divided-type selectively kill cells having normal homologous recombination activity, in the presence of GANC.

### Example D: Divided-Type DR-TK30 Construct (Co-transfection with SceTK + iTK30)

The *TK30* gene is a mutant of the *HSV-TK* gene, prepared by introducing mutations in amino acids near the active center of the *HSV-TK* gene so as to enhance the action on ganciclovir, and exhibits a stronger cytotoxicity than that of the wild-type HSV-TK (Kokoris MS et al. Gene Ther. 1999, Aug; 6(8): 1415-1426.). In the present Example, constructs of the present invention were prepared using the *TK30* gene as a suicide gene, and their cytocidal effects were evaluated.

### D-1. Preparation of Vectors

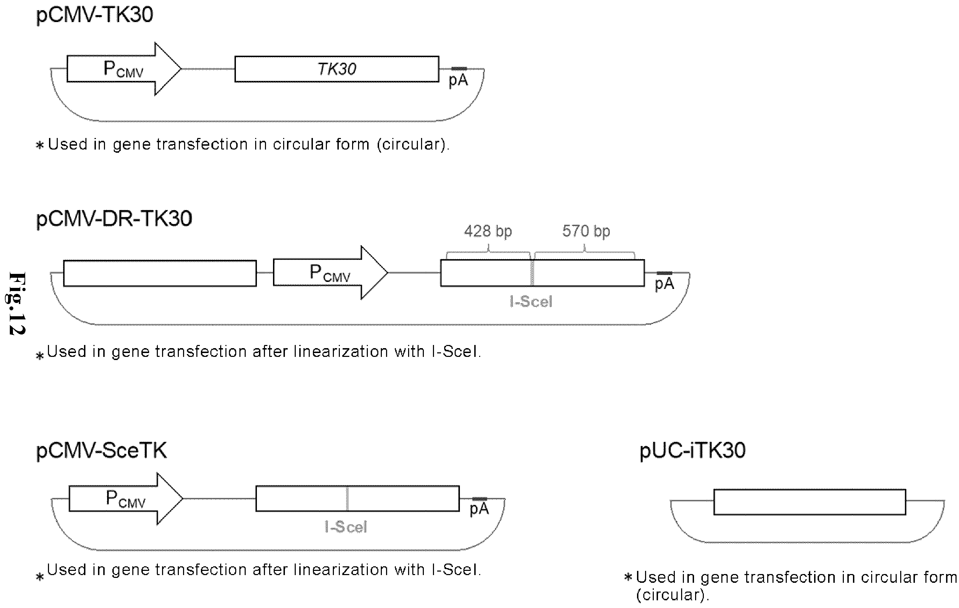

### D-1-1. Divided-Type DR-TK30 (Fig. 12, Bottom Row)

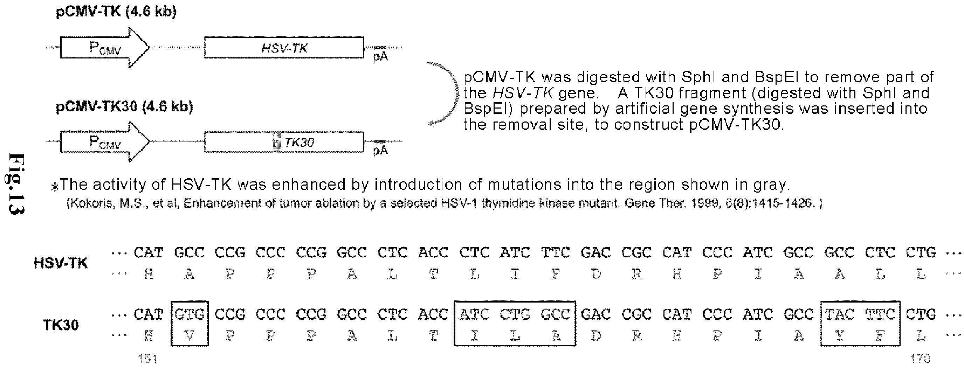

In order to prepare a construct that expresses the *TK30* gene, mutations were introduced into codons in the *HSV-TK* gene such that the alanine at position 152 was substituted with valine; the leucine at position 159 was substituted with isoleucine; the isoleucine at position 160 was substituted with leucine; the phenylalanine at position 161 was substituted with alanine; the alanine at position 168 was substituted with tyrosine; and the leucine at position 169 was substituted with phenylalanine (Fig. 13). As an *HSV-TK* gene fragment containing these mutations (*TK30* gene, SEQ ID NO:46), the portion from the SphI recognition sequence site (position 386 in the ORF sequence of the *HSV-TK* gene) located 5'-upstream of the mutation sites to the BspEI recognition sequence site (position 629 in the ORF sequence of the *HSV-TK* gene) located 3'-downstream the mutation sites was prepared by artificial gene synthesis (pTK30). The prepared pTK30 was digested with SphI and BspEI, and a fragment containing the *TK30* gene was recovered. Subsequently, pCMV-TK was digested with SphI and BspEI to remove positions 391 to 624 in the ORF sequence of the *HSV-TK* gene, and a fragment containing the CMV promoter, the poly-A addition signal, and part of the *HSV-TK* gene was recovered, followed by inserting the TK30 fragment thereto using a DNA Ligation Kit <Mighty Mix> (Takara Bio Inc.), to obtain a vector that expressed TK30, in which [CMV promoter] - [TK30 fragment] - [poly-A addition signal] were linked together (pCMV-TK30; Fig. 12, top row). The amino acid sequence of the TK30 protein encoded by the TK30 fragment (SEQ ID NO:46) is shown in SEQ ID NO:47.

pCMV-SceTK, in which [CMV promoter] - [SceTK fragment] - [poly-A addition signal] were linked together (Fig. 12, bottom row, left), was prepared as described in C-1-1.

An iTK30 fragment (1063 bp, the region of positions 27 to 1089 in the ORF sequence of the *TK30* gene), homologous to pCMV-SceTK, was obtained by PCR amplification using pCMV-TK30 as a template. As a polymerase for the PCR, PrimeSTAR HS (trade name) DNA Polymerase (Takara Bio Inc.) was used. iTK-Fw (SEQ ID NO:21) and iTK-Rv (SEQ ID NO:22) were used as PCR primers. The 428-bp base sequence in the 5'-end side and the 570-bp base sequence in the 3'-end side of the iTK30 fragment amplified by the PCR have homologies of 100% with the 428 bp in the 3'-end side (positions 27 to 454 of SEQ ID NO:40) and the 570 bp in the 5'-end side (positions 520 to 1089 of SEQ ID NO:40), respectively, of pCMV-SceTK cleaved with I-SceI. Subsequently, pUC19 (Takara Bio Inc.) was digested at SmaI in the multicloning site, and the iTK30 fragment was inserted thereto using a DNA Ligation Kit <Mighty Mix> (Takara Bio Inc.), to obtain pUC-iTK30 (Fig. 12, bottom row, right). The base sequence of the iTK30 fragment, and the amino acid sequence of the iTK30 protein encoded by positions 2 to 1063 of the base sequence, are shown in SEQ ID NOs:48 and 49, respectively.

### D-1-2. Integrated-Type DR-TK30 (pCMV-DR-TK30; Fig. 12, Middle Row)

In order to construct pCMV-DR-TK30, which carried the *SceTK* gene and the *iTK30* gene in one vector, a DNA fragment having a structure in which sequences for In-Fusion reaction were added to both ends of a 1063-bp iTK30 fragment was obtained by PCR amplification using pCMV-TK30 as a template. As a polymerase for the PCR, PrimeSTAR HS (trade name) DNA Polymerase (Takara Bio Inc.) was used. As primers, iTK-Fw (SEQ ID NO:21) and iTK-Rv (SEQ ID NO:22) were used. Subsequently, pCMV-SceTK30 was digested with BglII, and the iTK30 fragment was inserted upstream of the CMV promoter using an In-Fusion Snap Assembly Master Mix (Takara Bio Inc.), to obtain pCMV-DR-TK30, in which [iTK30 fragment] - [CMV promoter] - [SceTK fragment] - [poly-A addition signal] were linked together.

The pCMV-TK30, the pCMV-SceTK, the pUC-iTK30, and the pCMV-DR-TK30 were purified using a Qiagen Plasmid Plus Midi Kit (Qiagen K.K.). The purified pCMV-TK30 and pUC-iTK30 were used in transfection as they were in the circular form. The purified pCMV-SceTK and pCMV-DR-TK30 were linearized by cleavage with I-SceI (New England Biolabs) and then used in transfection.

### D-2. Cells and Gene Transfection, and Evaluation of Cytocidal Effect

### D-2-1. Cells and Gene Transfection

Culture and transfection of the human pre-B cell line Nalm-6 and its derivative cells, and culture of the human breast cancer-derived cell line MDA-NM-436, were carried out as described in A-2-1.

An olaparib-resistant clone (Olap^{R} cells) was established by culturing MDA-MB-436 cells for 2 weeks in a medium containing olaparib (Selleck Chemicals LLC, Houston, TX, USA) at a final concentration of 10 nM, and isolating the resulting colonies (according to a method in a past report (Zhou J et al. Nat Cancer. 2021 Jun; 2(6): 598-610.)). Olap^{R} cells have restored homologous recombination activity. Therefore, a construct showing a large difference in the luciferase activity or the cytocidal effect (that is, the recombination efficiency) between MDA-MB-436 cells and Olap^{R} cells can be judged to be a construct that sharply responds to the presence or absence of the homologous recombination activity.

Transfection of MDA-MB-436 cells and Olap^{R} cells was carried out using jetPEI (Polyplus-transfection) according to a protocol provided by the manufacturer.

### D-2-2. Evaluation of Cytocidal Effect

With each construct (1 µg), 2×10⁶ Nalm-6 cells were transfected. The cells after the gene transfection were cultured for 48 hours in a medium containing ganciclovir (Fujifilm Wako Pure Chemical Corporation) at a final concentration of 500 nM, and then transfected again with each construct (1 µg). The cells after the gene transfection were plated on a 24-well dish at 1×10⁵ cells/ml, and ganciclovir was added to the medium to a final concentration of 500 nM. After 96 hours of culture, the cell survival rate was measured using CellTiter-Glo (CellTiter-Glo (registered trademark) Luminescent Cell Viability Assay, Promega).

Evaluation of the cytocidal effects in MDA-MB-436 cells and Olap^{R} cells was carried out as follows. On a 24-well dish, 5×10⁴ cells were plated and cultured overnight, followed by transfecting the cells with each construct (1 µg). After the gene transfection, the cells were cultured for 24 hours, and then transfected again with each construct (1 µg), followed by adding ganciclovir (Fujifilm Wako Pure Chemical Corporation) to the medium to a final concentration of 500 nM. After 72 hours of culture, the cell survival rate was measured using CellTiter-Glo (CellTiter-Glo (registered trademark) Luminescent Cell Viability Assay, Promega).

### D-3. Results

The construction of the constructs in the present Example was carried out such that homologous recombination (HR) between the SceTK fragment and the iTK30 fragment resulted in formation of a normal *TK30* gene (modified *HSV-TK* gene).

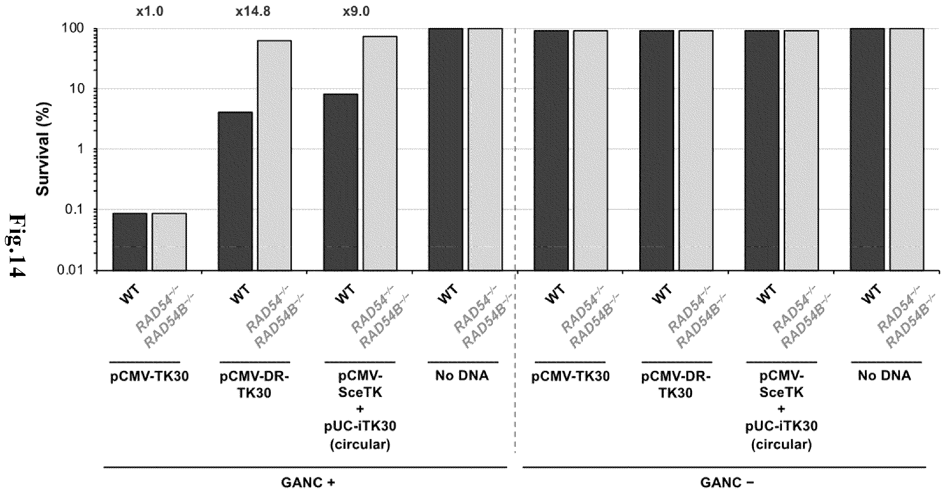

Fig. 14 shows a result of comparison of the survival rate between Nalm-6 cells and their HR-deficient cells (*RAD54*/*RAD54B*-double-deficient line) transfected with each constructed construct (Fig. 12). The survival rate is expressed as a relative value calculated by taking, as 100%, the survival rate of cells not transfected with DNA (No DNA) cultured in the presence of GANC (500 nM). This result confirmed that, also in cases where the *TK30* gene is used as a suicide gene, both the integrated-type and the divided-type selectively kill cells having normal homologous recombination activity, in the presence of GANC.

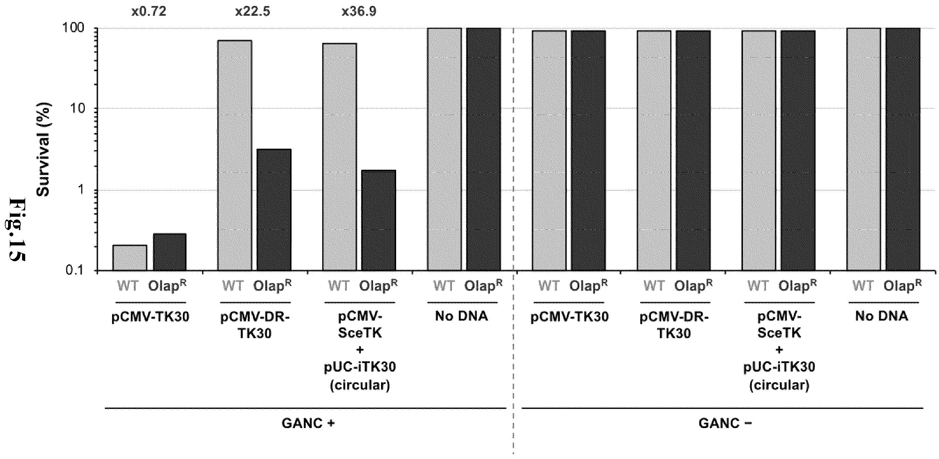

Fig. 15 shows a result of comparison of the survival rate after the transfection of each construct, between MDA-MB-436 cells (*BRCA1*-deficient cells) and Olap^{R} cells (MDA-MB-436 cells with restored homologous recombination activity). The survival rate is expressed as a relative value calculated by taking, as 100%, the survival rate of cells without transfection (No DNA) cultured in the presence of GANC (500 nM). When the integrated-type was transfected, the survival rate of the MDA-MB-436 cells hardly decreased, whereas, the survival rate of the Olap^{R} cells remarkably decreased in the presence of GANC. By the use of the divided type, the difference in the survival rate between the MDA-MB-436 cells and the Olap^{R} cells further increased (37-fold).

These results confirmed that use of the *TK30* gene as a suicide gene enables selective killing of cells having normal homologous recombination activity, in the presence of GANC. It was found that the divided-type construct has an especially high selective killing effect.

### Example E: Divided-Type DR-CDUPRT Construct (Co-transfection with SceCDUPRT + iCDUPRT)

In the present Example, the *CD::UPRT* gene was used as a gene encoding a protein that acts to decrease the survival rate of cells. The *CD::UPRT* gene is a fusion gene of the *Fcy1* gene (which encodes CD (cytosine deaminase)) derived from budding yeast and the *Fur1* gene (which encodes UPRT (uracil phosphoribosyl transferase)) derived from budding yeast. CD converts 5-fluorocytosine (5-FC) to 5-fluorouracil by deamination. 5-Fluorouracil is intracellularly metabolized to become a substance that inhibits DNA synthesis and RNA synthesis, thereby inducing cell death (Austin EA and Huber BE. Mol Pharmacol. 1993 Mar; 43(3): 380-387.). UPRT is known to catalyze a metabolic pathway in which 5-fluorouracil is converted to a toxic substance in the cell. By using UPRT in combination with CD, a cytotoxicity-enhancing effect and a bystander effect due to 5-fluorocytosine is obtained (Tiraby M et al. FEMS Microbiol Lett. 1998 Oct 1; 167(1): 41-49., Bourbeau D et al. J Gene Med. 2004 Dec; 6(12): 1320-1332.).

### E-1. Preparation of Vectors

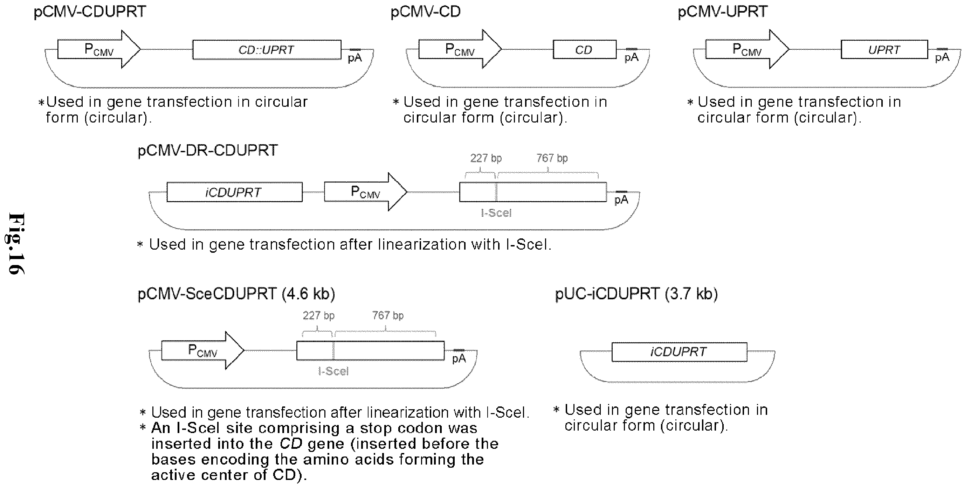

### E-1-1. CD: :UPRT Expression Vector, CD Expression Vector, and UPRT Expression Vector (Fig. 16, Top Row)

In order to prepare a construct that expressed the fusion gene (*CD::UPRT*) of the *Fcy1* gene (*CD*) derived from budding yeast and the *Fur1* gene (*UPRT*) derived from budding yeast, the codons of the *CD::UPRT gene* were optimized for use in human cells, according to a past report (Ho YK et al. Sci Rep. 2020 Aug 31; 10(1): 14257). Further, in order to perform cloning using restriction enzymes, an XhoI recognition sequence was added 5'-upstream, and an XbaI recognition sequence was added 3'-downstream, of the *CD::UPRT* gene. A sequence of the *CD::UPRT* gene (SEQ ID NO:50; which encodes a fusion protein (SEQ ID NO:51) having a structure in which the full-length CD was linked to the 3rd to 216th residues of UPRT through one alanine residue) was prepared by artificial gene synthesis (pCDUPRT). The prepared pCDUPRT was digested with XhoI and XbaI, and a fragment containing the *CD::UPRT* gene was recovered. Subsequently, pIRES (Takara Bio Inc.) was digested with XhoI and XbaI to remove the IRES region, and a fragment containing the CMV promoter and the poly-A addition signal was recovered, followed by inserting the CD: :UPRT fragment thereto using a DNA Ligation Kit <Mighty Mix> (Takara Bio Inc.), to obtain a CD: :UPRT expression vector (pCMV-CDUPRT), in which [CMV promoter] - [CD::UPRT fragment] - [poly-A addition signal] were linked together.

Subsequently, in order to prepare constructs that separately expressed CD or UPRT, a 477-bp *Fcy1* gene (in which a stop codon (TGA) was added to the 3'-end of the region of positions 1 to 474 in the *CD::UPRT* gene sequence; SEQ ID NO:52) and a 651-bp *Fur1* gene (in which an initiation codon (ATG) and a codon encoding serine (TCC) were added to the 5'-end of the region of positions 478 to 1122 in the *CD::UPRT* gene sequence; SEQ ID NO:54) were obtained by PCR amplification using pCMV-CDUPRT as a template (the codons of the base sequences of the *Fcy1* gene fragment and the *Fur1* gene fragment obtained had been optimized for expression in human cells). As a polymerase for the PCR, PrimeSTAR HS (trade name) DNA Polymerase (Takara Bio Inc.) was used. The primers used are shown below in Table 4. In the amplification of the *Fcy1* gene, Sce-5'CDUPRT-Fw (SEQ ID NO:60) and CD-Rv (SEQ ID NO:61) were used to amplify a DNA fragment having a structure in which a sequence for In-Fusion reaction was added to the 5'-end of the *Fcy1* gene fragment, and in which a stop codon (TGA) and a sequence for In-Fusion reaction were added to the 3'-end of the *Fcy1* gene fragment. Subsequently, pIRES (Takara Bio Inc., Fig. 2) was digested with XhoI and XbaI to remove the IRES region, and a fragment containing the CMV promoter and the poly-A addition signal was recovered, followed by inserting the *Fcy1* gene fragment thereto using a DNA Ligation Kit <Mighty Mix> (Takara Bio Inc.), to obtain a codon-optimized CD expression vector (pCMV-CD) in which [CMV promoter] - [*Fcy1* gene fragment] - [poly-A addition signal] were linked together. In the amplification of the *Fur1* gene, UPRT-Fw (SEQ ID NO:62) and Sce-3'CDUPRT-Rv (SEQ ID NO:63) were used to amplify a DNA fragment having a structure in which an initiation codon, a codon encoding serine, and a sequence for In-Fusion reaction were added to the 5'-end of the *Fur1* gene fragment, and in which a sequence for In-Fusion reaction was added to the 3'-end of the *Fur1* gene fragment. Subsequently, pCMV-CDUPRT was digested with AgeI and XbaI to remove the *CD::UPRT* gene*,* and a fragment containing the CMV promoter and the poly-A addition signal was recovered, followed by inserting the *Fur1* gene fragment thereto using a DNA Ligation Kit <Mighty Mix> (Takara Bio Inc.), to obtain a UPRT expression vector (pCMV-UPRT) in which [CMV promoter] - [*Fur1* gene fragment] - [poly-A addition signal] were linked together.

### E-1-2. Divided-Type DR-CDUPRT Construct (Fig. 16, Bottom Row)

In order to prepare a construct that decreased the survival rate of cells depending on the homologous recombination activity of the cells, a 1120-bp SceCDUPRT fragment (SEQ ID NO:56; a DNA having a structure in which the region of positions 271 to 300 in the *CD::UPRT* gene sequence was replaced with "stop codon (TGA) + cgcg + I-SceI recognition sequence", having a stop codon added to the 3'-end) and a 1022-bp iCDUPRT fragment (SEQ ID NO:58; the region of positions 46 to 1067 in the *CD::UPRT* gene sequence) were obtained by PCR amplification using pCMV-CDUPRT as a template. As a polymerase for the PCR, PrimeSTAR HS (trade name) DNA Polymerase (Takara Bio Inc.) was used. The primers used are shown below in Table 4.

In the amplification of the SceCDUPRT fragment, two fragments were separately amplified to obtain a 5'SceCDUPRT fragment and a 3'SceCDUPRT fragment. In the amplification of the 5'SceCDUPRT fragment, Sce-5'CDUPRT-Fw (SEQ ID NO:60) and Sce-5'CDUPRT-Rv (SEQ ID NO:64) were used to amplify a DNA fragment having a structure in which a sequence for In-Fusion reaction was added to the 5'-end of the 5'SceCDUPRT fragment, and in which "stop codon (TGA) + cgcg + I-SceI recognition sequence" were added to the 3'-end of the 5'SceCDUPRT fragment. In the amplification of the 3'SceCDUPRT fragment, Sce-3'CDUPRT-Fw (SEQ ID NO:65) and Sce-3'CDUPRT-Rv (SEQ ID NO:63) were used to amplify a DNA fragment having a structure in which "I-SceI recognition sequence" was added to the 5'-end of the 3'SceCDUPRT fragment, and in which a sequence for In-Fusion reaction was added to the 3'-end of the 3' SceCDUPRT fragment. Subsequently, pIRES (Takara Bio Inc., Fig. 2) was digested with NheI and BamHI to remove the IRES region and the poly-A addition signal, and a fragment containing the CMV promoter was recovered. Using an In-Fusion Snap Assembly Master Mix (Takara Bio Inc.; the former product name, In-Fusion HD Cloning Kit), the 5'SceCDUPRT fragment and the 3'SceCDUPRT fragment were inserted downstream of the CMV promoter of the recovered fragment, to obtain pCMV-SceCDUPRT, in which [CMV promoter] - [SceCDUPRT fragment] - [poly-A addition signal] were linked together. In the reading frame of the SceCDUPRT fragment (SEQ ID NO:56), there is the stop codon TGA introduced upstream of the I-SceI recognition sequence, and in addition, there are a large number of stop codons downstream thereof generated due to frameshift. The amino acid sequence encoded by positions 1 to 270 (the region up to the first stop codon) of the SceCDUPRT fragment is shown in SEQ ID NO: 57.

For amplification of the iCDUPRT fragment (SEQ ID NO:58), which was homologous to pCMV-SceCDUPRT, iCDUPRT-Fw (SEQ ID NO:66) and iCDUPRT-Rv (SEQ ID NO:67) were used. The 227-bp base sequence in the 5'-end side and the 767-bp base sequence in the 3'-end side of the iCDUPRT fragment amplified by the PCR have homologies of 100% with the 227 bp in the 3'-end side (positions 46 to 272 of SEQ ID NO:56) and the 767 bp in the 5'-end side (positions 299 to 1065 of SEQ ID NO:56), respectively, of pCMV-SceCDUPRT cleaved with I-SceI. Subsequently, pUC19 (Takara Bio Inc.) was digested at SmaI in the multicloning site, and the iCDUPRT fragment was inserted thereto using a DNA Ligation Kit <Mighty Mix> (Takara Bio Inc.), to obtain pUC-iCDUPRT. The amino acid sequence of the iCDUPRT protein encoded by the iCDUPRT fragment (SEQ ID NO:58) is shown in SEQ ID NO:59.

### E-1-3. Integrated-Type DR-CDUPRT Construct (Fig. 16, Middle Row)

In order to construct pCMV-DR-CDUPRT, which carried the *SceCDUPRT* gene and the *iCDUPRT* gene in one vector, a DNA fragment having a structure in which sequences for In-Fusion reaction were added to both ends of a 1022-bp iCDUPRT fragment was obtained by PCR amplification using pCMV-CDUPRT as a template. As a polymerase for the PCR, PrimeSTAR HS (trade name) DNA Polymerase (Takara Bio Inc.) was used. As primers, iCDUPRT-Fw (SEQ ID NO:66) and iCDUPRT-Rv (SEQ ID NO:67) were used. Subsequently, pCMV-SceCDUPRT was digested with BglII, and the iCDUPRT fragment was inserted upstream of the CMV promoter using an In-Fusion Snap Assembly Master Mix (Takara Bio Inc.), to obtain pCMV-DR-CDUPRT, in which [iCDUPRT fragment] - [CMV promoter] - [SceCDUPRT fragment] - [poly-A addition signal] were linked together.

All the plasmids prepared were purified using a Qiagen Plasmid Plus Midi Kit (Qiagen K.K.). The purified pCMV-CDUPRT, pCMV-CD, pCMV-UPRT, and pUC-iCDUPRT were used in transfection as they were in the circular form. The purified pCMV-SceCDUPRT and pCMV-DR-CDUPRT were linearized by cleavage with I-SceI (New England Biolabs) and then used in transfection.

**[Table 4]**

| Primer name | Sequence (5'→3') | SEQ ID NO |
|---|---|---|
| Sce-5'CDUPRT-Fw | TAATACGACTCACTATAGGCTAG | 60 |
| CD-Rv | GCCGCCCCGACTCTAG**TCA**CTCCCCAATGTCCTCAAACCAG | 61 |
| UPRT-Fw | CTAGCCTCGAGACCGGTCACCATGTCCTCTGAGCCCTTCAAGAATG | 62 |
| Sce-3'CDUPRT-Rv | GAGTGCACCATACGCGGATC | 63 |
| Sce-5'CDUPRT-Rv | ATTACCCTGTTATCCCTACGCG**T****CA**AGGGCTGAGGGTGGTGTACAG | 64 |
| Sce-3'CDUPRT-Fw | GCGTAGGGATAACAGGGTAATGCATATGGCATTCCCAGGTGTGTGG | 65 |
| iCDUPRT-Fw | CATGGCTCGACAGATCTGACATTGCCTATGAGGAGGCTG | 66 |
| iCDUPRT-Rv | GGCCAATATTGAAGATCTTCTCATCCAGGCCCCTGTCC | 67 |

| | | |
|---|---|---|
| Single-underline: I-SceI recognition sequence Double-underline: A site that anneals to the target gene sequence (Sce-5'CDUPRT-Fw and Sce-3'CDUPRT-Rv target vector sequences.) Bold: Sequence for introduction of a stop codon (TGA) | | |

### E-2. Cells and Gene Transfection, and Evaluation of Cytocidal Effect

### E-2-1. Cells and Gene Transfection

Same as D-2-1.

### E-2-2. Evaluation of Cytocidal Effect

With each construct (1 µg), 2×10⁶ Nalm-6 cells were transfected. The cells after the gene transfection were cultured for 48 hours in a medium containing 5-fluorocytosine (Fujifilm Wako Pure Chemical Corporation) at a final concentration of 100 µM, and then transfected again with each construct (1 µg). The cells after the gene transfection were plated on a 24-well dish at 1×10⁵ cells/ml, and 5-fluorocytosine (5-FC) was added to the medium to a final concentration of 100 µM. After 96 hours of culture, the cell survival rate was measured using CellTiter-Glo (CellTiter-Glo (registered trademark) Luminescent Cell Viability Assay, Promega).

Evaluation of the cytocidal effects in MDA-MB-436 cells and Olap^{R} cells was carried out as follows. On a 24-well dish, 5×10⁴ cells were plated and cultured overnight, followed by transfecting the cells with each construct (1 µg). After the gene transfection, the cells were cultured for 24 hours, and then transfected again with each construct (1 µg), followed by adding 5-fluorocytosine (Fujifilm Wako Pure Chemical Corporation) to the medium to a final concentration of 100 µM. After 72 hours of culture, the cell survival rate was measured using CellTiter-Glo (CellTiter-Glo (registered trademark) Luminescent Cell Viability Assay, Promega).

### E-3. Results

### E-3-1. Confirmation of Effects of Separate Expression of CDUPRT, CD, or UPRT

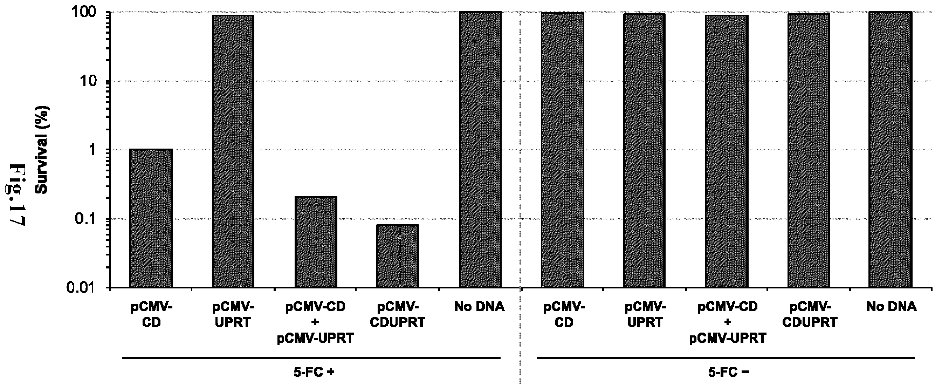

In order to investigate whether the cytotoxicity by 5-FC is enhanced by co-expression of CD and UPRT, each construct was introduced into Nalm-6 cells of the wild line, and the survival rate was compared. The result is shown in Fig. 17. The survival rate is expressed as a relative value calculated by taking, as 100%, the survival rate of cells without transfection(No DNA) cultured in the presence of 5-FC (100 µM). The cells transfected with pCMV-CD only showed a decrease in the survival rate in the presence of 5-FC, but no decrease in the survival rate was found when transfected with pCMV-UPRT only. From this result, it was found that expression of UPRT alone does not produce cytotoxicity.

Subsequently, the effect of co-expression of CD and UPRT was investigated. As a result, compared to when pCMV-CD was transfected alone, co-transfection with pCMV-CD and pCMV-UPRT produced an about 5-fold higher cytocidal effect, and transfection with pCMV-CDUPRT (a construct that expresses a fusion gene of CD and UPRT) produced an about 13-fold higher cytocidal effect.

The above results confirmed that, although a cytocidal effect by 5-FC is obtained even by expression of CD alone, a higher cytocidal effect is obtained by expression of both CD and UPRT.

### E-3-2. Cytocidal Effect

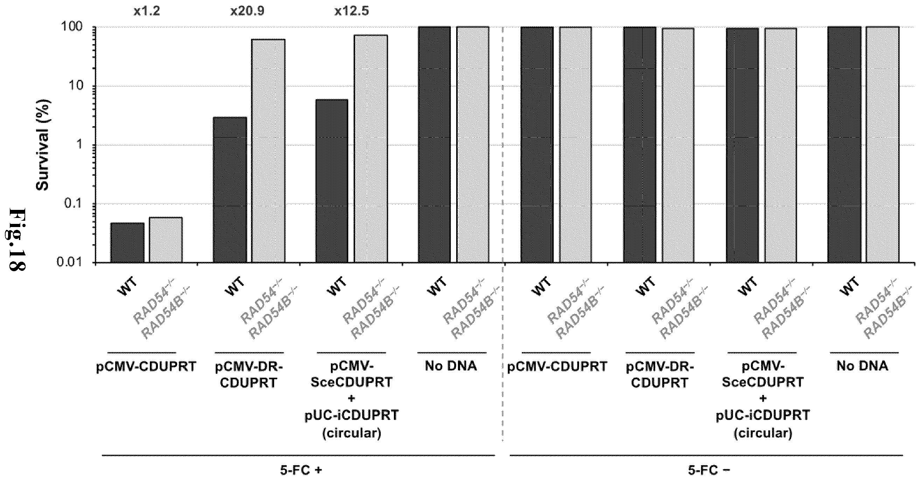

The construction of the constructs in the present Example was carried out such that homologous recombination (HR) between the SceCDUPRT fragment and the iCDUPRT fragment resulted in formation of a normal *CD::UPRT* gene (Fig. 16, middle and bottom rows). Fig. 18 shows a result of comparison of the survival rate between Nalm-6 cells and their HR-deficient cells (*RAD54*/*RAD54B*-double-deficient line) transfected with each constructed construct. The survival rate is expressed as a relative value calculated by taking, as 100%, the survival rate of cells not transfected with DNA (No DNA) cultured in the presence of 5-FC (100 µM). The result confirmed that, also in cases where CD::UPRT is used as a suicide gene, both the integrated-type and the divided-type selectively kill cells having normal homologous recombination activity, in the presence of 5-FC.

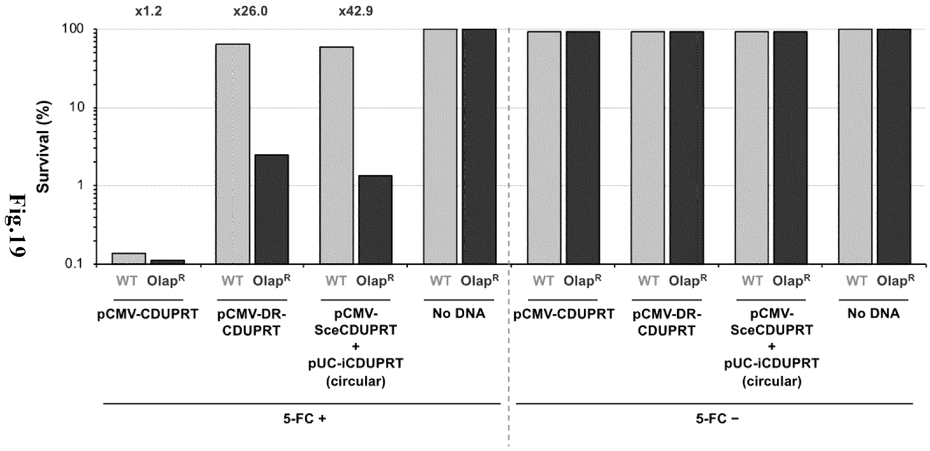

Fig. 19 shows a result of comparison of the survival rate after transfection with each construct between MDA-MB-436 cells and Olap^{R} cells. The survival rate is expressed as a relative value calculated by taking, as 100%, the survival rate of cells without transfection (No DNA) cultured in the presence of 5-FC (100 µM). When the integrated-type was transfected, the survival rate of the MDA-MB-436 cells hardly decreased, whereas, the survival rate of the Olap^{R} cells remarkably decreased (a 26-fold difference) in the presence of 5-FC. By the use of the divided type, the difference in the survival rate between the MDA-MB-436 cells and the Olap^{R} cells further increased (a 43-fold difference).

The above results confirmed that use of the *CD::UPRT* gene as a suicide gene also enables selective killing of normal cells having normal homologous recombination activity, in the presence of 5-FC. It was found that the divided-type construct has a higher selective killing effect than the integrated-type.

### Example F: Divided-Type DR-CeNL Construct (Co-transfection with SceCeNL + iCeNL)

The *CeNL* (cyan enhanced nano-lantern) gene encodes a fusion protein of the chemiluminescent protein Nluc and the cyan fluorescent protein mTurquoise2 (Goedhart J et al. Nat Commun. 2012, Mar 20; 3: 751.). CeNL protein is known to have a luminescence intensity about twice as high as that of Nluc due to forester resonance energy transfer (FRET) between Nluc and mTurquoise2 (Suzuki K et al. Nat Commun. 2016, Dec 14;7: 13718.). Therefore, use of a sequence of the *CeNL* gene for a nucleic acid molecule of the present invention enables more rapid (higher-sensitivity) measurement of homologous recombination activity than use of a chemiluminescence gene alone. Specific examples of such use are shown below.

### F-1. Preparation of Vectors

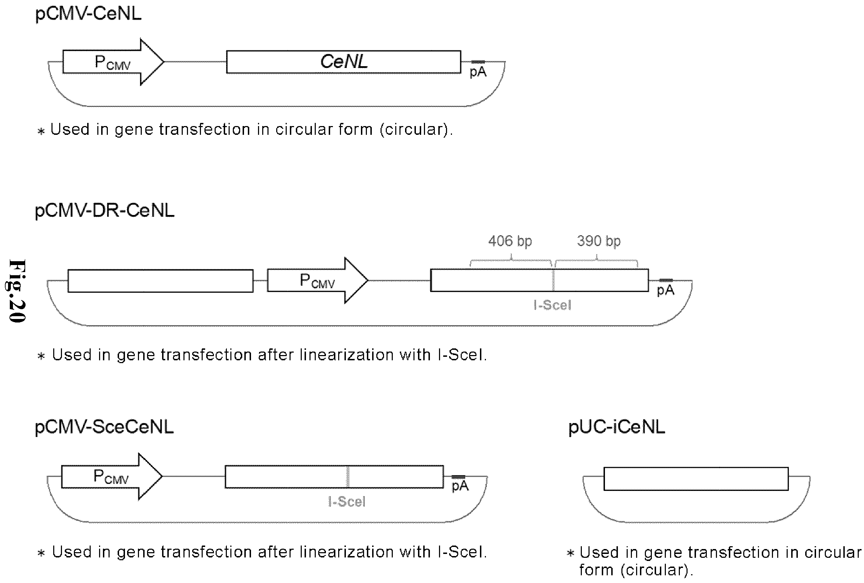

### F-1-1. CeNL Expression Vector (Fig. 20, Top Row)

In order to prepare constructs that expressed the *CeNL* gene, a partial *CeNL* gene fragment in which a recognition sequence of XhoI was linked 5'-upstream, and in which the region of positions 10 to 79 (including an EcoNI recognition sequence) in the ORF sequence of the *Nluc* gene was linked 3'-downstream, of the *mTurquoise2* gene (the region of positions 1 to 687 in the *mTurquoise2* gene sequence shown in SEQ ID NO:68) was prepared by artificial gene synthesis (pCeNL). The prepared pCeNL was digested with XhoI and EcoNI, and a fragment containing the partial *CeNL* gene fragment was recovered. Subsequently, pCMV-Nluc was digested with XhoI and EcoNI to remove the 5'-upstream untranslated region of the *Nluc* gene and positions 1 to 73 in the ORF sequence of the *Nluc* gene, and a fragment containing the CMV promoter, the poly-A addition signal, and part of the *Nluc* gene was recovered. Using a DNA Ligation Kit <Mighty Mix> (Takara Bio Inc.), the partial *CeNL* gene fragment was inserted into the recovered fragment, to obtain a vector that expressed CeNL (pCMV-CeNL), in which [CMV promoter] - [*CeNL* gene fragment] - [poly-A addition signal] were linked together. The base sequence of the *CeNL* gene, and the amino acid sequence of the CeNL protein encoded thereby, are shown in SEQ ID NOs:70 and 71, respectively. The CeNL protein prepared was a protein having a structure in which the 1st to 229th residues of mTurquoise2 protein (SEQ ID NO:69) was linked to the 4th to 171st residues of Nluc protein (SEQ ID NO:24) through LH.

### F-1-2. Divided-Type DR-CeNL Construct (Fig. 20, Bottom Row)

A 1080-bp SceCeNL fragment (SEQ ID NO:72; a DNA having a structure in which the region of positions 601 to 749 in the *CeNL* gene ORF sequence was replaced with "stop codon (TGA) + attc + I-SceI recognition sequence", having a stop codon added to the 3'-end) to be used for pCMV-SceCeNL was obtained by PCR amplification using pCMV-CeNL as a template. As a polymerase for the PCR, PrimeSTAR HS (trade name) DNA Polymerase (Takara Bio Inc.) was used. The primers used are shown below in Table 5. In the amplification of the SceCeNL fragment, two fragments were separately amplified to obtain a 5'SceCeNL fragment and a 3' SceCeNL fragment. In the amplification of the 5' SceCeNL fragment, Nluc-Fw (SEQ ID NO: 1) and Sce-5'CeNL-Rv (SEQ ID NO:78) were used to amplify a DNA fragment having a structure in which a sequence for In-Fusion reaction was added to the 5'-end of the 5'SceCeNL fragment, and in which a stop codon (TGA) was added to the 3'-end of the 5'SceCeNL fragment. In the amplification of the 3' SceCeNL fragment, Sce-3'CeNL-Fw (SEQ ID NO:79) and Nluc-Rv (SEQ ID NO:4) were used to amplify a DNA fragment having a structure in which "I-SceI recognition sequence" was added to the 5'-end of the 3'SceTK fragment, and in which a sequence for In-Fusion reaction was added to the 3'-end of the 3'SceTK fragment. Subsequently, pIRES (Takara Bio Inc., Fig. 2) was digested with NheI and XbaI to remove the IRES region, and a fragment containing the CMV promoter and the poly-A addition signal was recovered. Using an In-Fusion HD Cloning Kit (Takara Bio Inc.), the 5'SceCeNL fragment and the 3'SceCeNL fragment were inserted downstream of the CMV promoter of the recovered fragment, to obtain pCMV-SceCeNL, in which [CMV promoter] - [SceCeNL fragment] - [poly-A addition signal] were linked together. In the reading frame of the SceCeNL fragment (SEQ ID NO:72), there are the stop codon TGA introduced upstream of the I-SceI recognition sequence, and the stop codon TAA present inside the I-SceI recognition sequence. The amino acid sequences encoded by positions 1 to 603 (the region from position 1 to the first stop codon TGA), positions 604 to 624 (the region downstream of the first stop codon TGA and up to the second stop codon TAA), and positions 625 to 1080 (the region downstream of the second stop codon) of the SceCeNL fragment are shown in SEQ ID NOs: 73 to 75, respectively.

An iCeNL fragment (SEQ ID NO:76; 944 bp, the region of positions 212 to 1155 in the ORF sequence of the *CeNL* gene), homologous to pCMV-SceCeNL, was obtained by PCR amplification using pCMV-CeNL as a template. As a polymerase for the PCR, PrimeSTAR HS (trade name) DNA Polymerase (Takara Bio Inc.) was used. iCeNL-Fw (SEQ ID NO:80) and iCeNL-Rv (SEQ ID NO:81) were used as PCR primers. The 390-bp base sequence in the 5'-end side and the 406-bp base sequence in the 3'-end side of the iCeNL fragment amplified by the PCR have homologies of 100% with the 390 bp in the 3'-end side (positions 212 to 601 of SEQ ID NO:72) and the 406 bp in the 5'-end side (positions 630 to 1035 of SEQ ID NO:72), respectively, of pCMV-SceCeNL cleaved with I-SceI. Subsequently, pUC19 (Takara Bio Inc.) was digested at SmaI in the multicloning site, and the iCeNL fragment was inserted thereto using a DNA Ligation Kit <Mighty Mix> (Takara Bio Inc.), to obtain pUC-iCeNL. The amino acid sequence of the iCeNL protein encoded by positions 3 to 944 of the iCeNL fragment (SEQ ID NO:76) is shown in SEQ ID NO:77.

### F-1-3. Integrated-Type DR-CeNL Construct (Fig. 20, Middle Row)

In order to construct pCMV-DR-CeNL, which carried the Sce*CeNL* gene and the *iCeNL* gene in one vector, a DNA fragment having a structure in which sequences for In-Fusion reaction were added to both ends of a 944-bp iCeNL fragment was obtained by PCR amplification using pCMV-CeNL as a template. As a polymerase for the PCR, PrimeSTAR HS (trade name) DNA Polymerase (Takara Bio Inc.) was used. As primers, iCeNL-Fw (SEQ ID NO:80) and iCeNL-Rv (SEQ ID NO:81) were used. Subsequently, pCMV-SceCeNL was digested with BglII, and the iCeNL fragment was inserted upstream of the CMV promoter using an In-Fusion Snap Assembly Master Mix (Takara Bio Inc.), to obtain pCMV-DR-CeNL, in which [iCeNL fragment] - [CMV promoter] - [SceCeNL fragment] - [poly-A addition signal] were linked together.

The pCMV-CeNL, the pCMV-SceCeNL, the pUC-iCeNL, and the pCMV-DR-CeNL were purified using a Qiagen Plasmid Plus Midi Kit (Qiagen K.K.). The purified pCMV-CeNL and pUC-iCeNL were used in transfection as they were in the circular form. The purified pCMV-SceCeNL and pCMV-DR-CeNL were linearized by cleavage with I-SceI (New England Biolabs) and then used in transfection.

**[Table 5]**

| Primer name | Sequence (5'→3') | SEQ ID NO |
|---|---|---|
| Nluc-Fw | TAATACGACTCACTATAGGCTAG | 1 |
| Sce-5'CeNL-Rv | TTACCCTGTTATCCCTAGAAT**TC****A**GTGGTTGTCGGGCAGCAGCAC | 78 |
| Sce-3'CeNL-Fw | TCTAGGGATAACAGGGTAATGCATAGTCCTTGAACAGGGAGGTGTG | 79 |
| Nluc-Rv | CCGCCCCGACTCTAGAGTCGCGG | 4 |
| iCeNL-Fw | ACATGGCTCGACAGATCTGCTTCGCCCGCTACCCCGACC | 80 |
| iCeNL-Rv | GGCCAATATTGAAGATCTGATGGTTACTCGGAACAGCAGG | 81 |

| | | |
|---|---|---|
| Single-underline: I-SceI recognition sequence Double-underline: A site that anneals to the target gene sequence (Nluc-Fw and Nluc-Rv target vector sequences located upstream and downstream of the CeNL gene fragment, respectively.) Bold: Sequence for introduction of a stop codon (TGA) | | |

### F-2. Cells and Gene Transfection, and Luciferase Assay

### F-2-1. Cells and Gene Transfection

Same as D-2-1.

### F-2-2. Luciferase Assay

With each construct (1 µg), 2×10⁶ Nalm-6 cells (wild line) were transfected. The cells after the gene transfection were plated on a 12-well dish at 5×10⁵ cells/ml, and the luciferase activity (RLU value) was measured immediately after the gene transfection, and at 1, 2, 4, 8 and 24 hours post-transfection using the Nano-Glo Luciferase Assay System (Promega).

A luciferase assay of MDA-MB-436 cells and Olap^{R} cells was carried out as follows. On a 24-well dish, 5×10⁴ cells were plated and cultured overnight, followed by transfecting the cells with each construct (1 µg). The luciferase activity (RLU value) was measured immediately after the gene transfection, and at 1, 2, 4, 8 and 24 hours post-transfection using the Nano-Glo Luciferase Assay System (Promega).

### F-3. Results

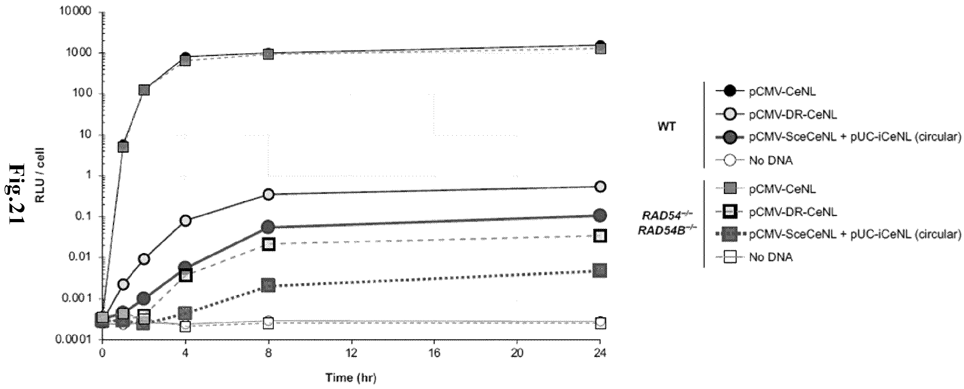

The constructs were designed such that homologous recombination (HR) between the SceCeNL fragment and the iCeNL fragment results in formation of a normal *CeNL* gene (Fig. 20). The luciferase activity in the wild line and the RAD54/RAD54B-double-deficient line (homologous recombination-deficient cells) of Nalm-6 cells after the transfection of each construct was measured over time. The results are shown in Fig. 21. pCMV-CeNL represents cells transfected with a construct that expressed the normal *CeNL* gene under the control by the CMV promoter (positive control). In the Nalm-6 cells of the wild line transfected with the integrated-type (pCMV-DR-CeNL) or divided-type (pCMV-SceCeNL + pUC-iCeNL) construct, increased luciferase activity was found within 2 hours post-transfection. On the other hand, the luciferase activity in the *RAD54*/*RAD54B-*double-deficient line was not more than 1/15 of that in the wild line in both cases of the integrated-type and the divided-type.

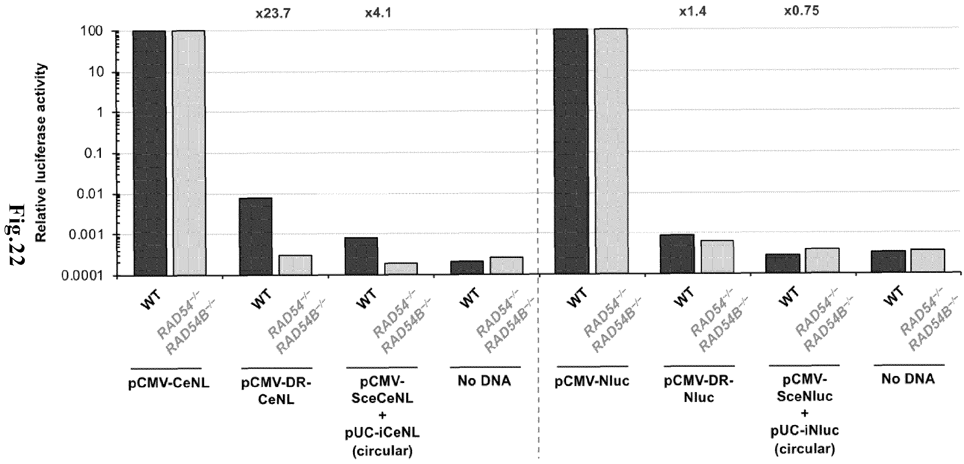

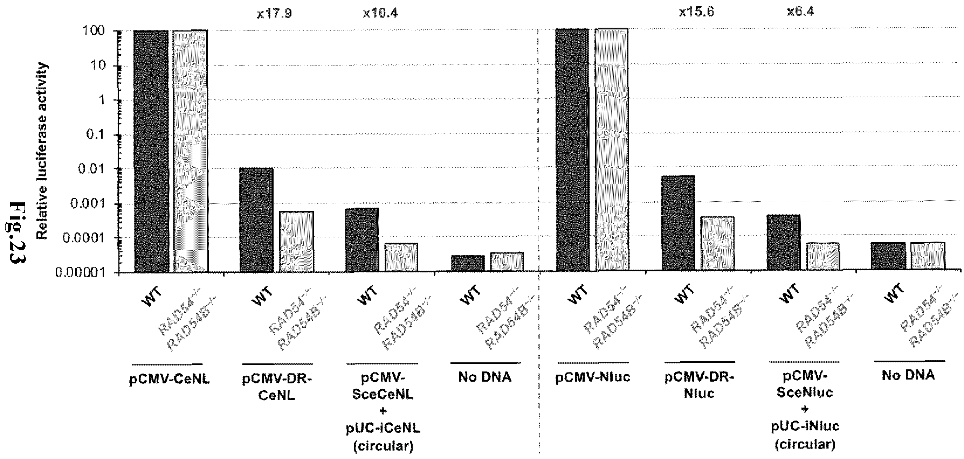

Fig. 22 and Fig. 23 show results obtained by transfecting Nalm-6 cells with the constructs of Example A, which utilize Nluc, or the constructs of the present Example F, which utilize CeNL, and performing a luciferase assay at 2 hours and 4 hours post-transfection. Each value is expressed as a relative value calculated by taking, as 100, the RLU value in the cells transfected with pCMV-CeNL or pCMV-Nluc. Since the luminescence intensity of CeNL protein is higher than that of Nluc protein, constructs using CeNL were thought to be capable of detecting the luciferase activity in a shorter time than constructs using Nluc. Actually, as shown in Fig. 22, the constructs using CeNL exhibited clear differences in the luciferase activity between the wild line and the *RAD54*/*RAD54B*-double-deficient line at 2 hours post-transfection. In the case of the constructs of Example A, which used Nluc, increase in the luciferase activity was not yet observed at 2 hours post-transfection (Fig. 22, right).

Four hours after the transfection with each construct, both the CeNL and Nluc constructs caused differences between the luciferase activity in the wild line and the luciferase activity in the *RAD54*/*RAD54B*-double-deficient line (Fig. 23). These results confirmed that constructs using CeNL also enable detection of homologous recombination activity of cells similarly to constructs using Nluc, and that constructs using CeNL are capable of detecting homologous recombination activity of cells in a shorter time than constructs using Nluc.

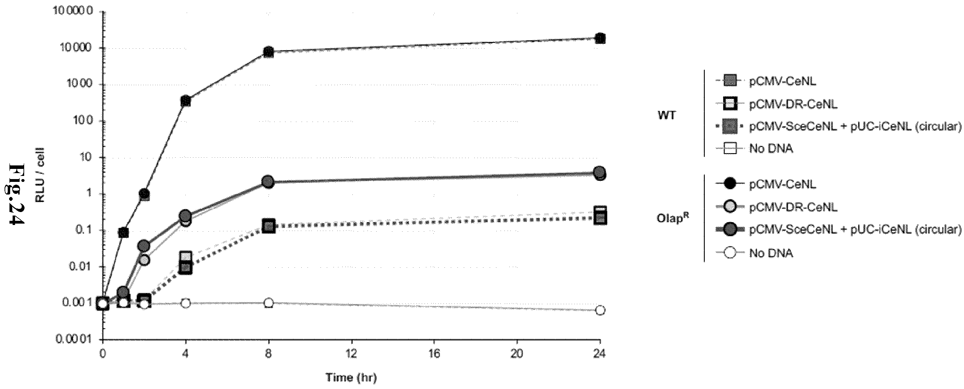

Fig. 24 shows a result of comparison of the luciferase activity between MDA-MB-436 cells (cells deficient in homologous recombination activity due to *BRCA1* deficiency) and Olap^{R} cells (MDA-MB-436 cells with restored homologous recombination activity). In the Olap^{R} cells transfected with the integrated-type (pCMV-DR-CeNL) or divided-type (pCMV-SceCeNL + pUC-iCeNL) construct, increased luciferase activity was found within 2 hours post-transfection. On the other hand, in the MDA-MB-436 cells, the luciferase activity detected after the transfection of any of the constructs was lower than that in the Olap^{R} cells, showing a difference of as large as not less than 10-fold in the case of the integrated-type, and not less than 20-fold in the case of the divided type.

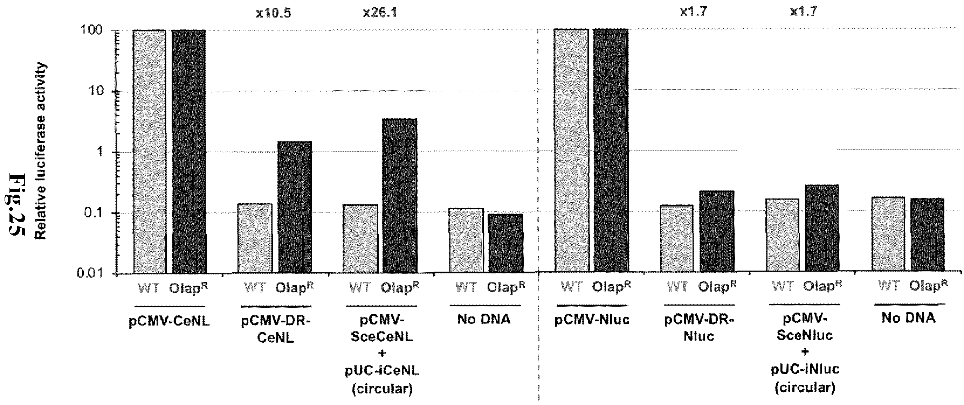

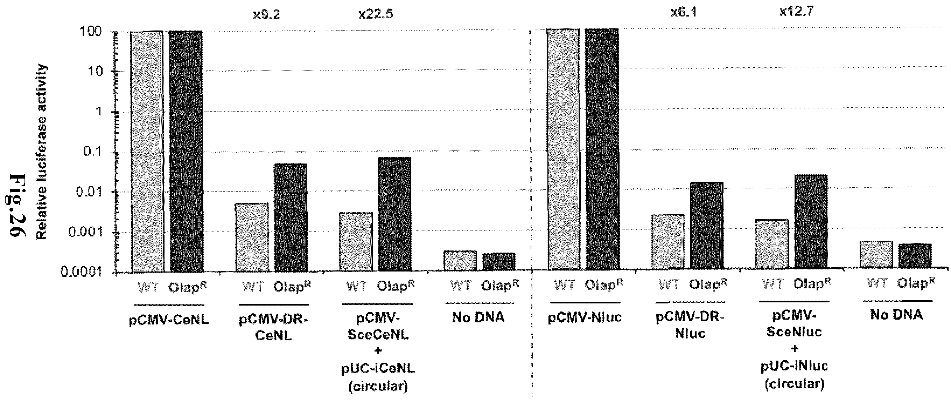

Fig. 25 and Fig. 26 show results obtained by transfecting MDA-MB-436 cells or Olap^{R} cells with a construct using Nluc or CeNL, and performing a luciferase assay at 2 hours and 4 hours post-transfection. Each value is expressed as a relative value calculated by taking, as 100, the RLU value in the cells transfected with pCMV-CeNL or pCMV-Nluc. The result confirmed that, as in the Example with Nalm-6 cells, the constructs of the present Example F, which use CeNL, are capable of detecting homologous recombination activity of the cells in a shorter time than the constructs of Example A, which use Nluc.

## Claims

1. A nucleic acid construct comprising a set of a first nucleic acid molecule and a second nucleic acid molecule,
the first nucleic acid molecule comprising: a promoter region; and a mutant gene sequence placed downstream thereof, said mutant gene sequence being constituted by a gene sequence encoding a protein and a cleavage site arranged inside thereof;
the second nucleic acid molecule comprising a complementation region capable of replacing, by homologous recombination, a partial region comprising the cleavage site in the mutant gene sequence, the complementation region being constituted by a first homologous region and a second homologous region and composed of a base sequence selected from the following (i) to (iii):
(i) a continuous partial sequence in the gene sequence encoding the protein, the continuous partial sequence containing upstream and downstream regions adjacent to the cleavage site in the mutant gene sequence;
(ii) a base sequence which is homologous to the partial sequence of (i) and encodes the same amino acid sequence as that encoded by said partial sequence; and
(iii) a continuous partial sequence in a gene sequence encoding a protein whose sequence is 80% or more identical to that of the previously-mentioned protein and which has the same activity as the previously-mentioned protein, the continuous partial sequence being a base sequence homologous to the partial sequence of (i).

2. The nucleic acid construct according to claim 1, wherein the mutant gene sequence contains a stop codon upstream of the cleavage site.

3. The nucleic acid construct according to claim 1, wherein the base sequences of (ii) and (iii) are 90% or more homologous to the partial sequence of (i).

4. The nucleic acid construct according to claim 1, wherein the second nucleic acid molecule comprises a continuous partial sequence in the gene sequence encoding the protein, the continuous partial sequence containing upstream and downstream regions adjacent to the cleavage site in the mutant gene sequence.

5. The nucleic acid construct according to claim 2, wherein the second nucleic acid molecule comprises a continuous partial sequence in the gene sequence encoding the protein, the continuous partial sequence containing upstream and downstream regions adjacent to the stop codon and the cleavage site in the mutant gene sequence.

6. The nucleic acid construct according to claim 1, wherein each of the first homologous region and the second homologous region has a strand length of at least 20 bases.

7. The nucleic acid construct according to claim 1, wherein the first nucleic acid molecule comprises a poly-A addition signal downstream of the mutant gene sequence.

8. The nucleic acid construct according to claim 1, wherein the cleavage site is a restriction enzyme recognition site.

9. The nucleic acid construct according to claim 1, wherein the second nucleic acid molecule is a circular nucleic acid molecule further containing an additional sequence of 1 to 20,000 bases linked to the complementation region, or is a linear nucleic acid molecule containing an additional sequence of 1 to 10,000 bases linked to at least one of the 5'-end and the 3'-end of the complementation region.

10. The nucleic acid construct according to claim 1, wherein the first nucleic acid molecule is a circular nucleic acid molecule, or a linear nucleic acid molecule prepared by cleaving said circular nucleic acid molecule at the cleavage site.

11. The nucleic acid construct according to claim 1, wherein the gene sequence is a gene sequence encoding a protein that acts to decrease cell survival rate or a protein whose intracellular expression is detectable.

12. The nucleic acid construct according to claim 11, wherein the gene sequence is a sequence of a suicide gene, a DNA damage-inducing gene, a DNA repair-inhibiting gene, a luminescent enzyme gene, a fluorescent protein gene, a cell surface antigen gene, a secretory protein gene, or a membrane protein gene.

13. A measurement reagent for homologous recombination activity, the measurement reagent comprising the nucleic acid construct according to any one of claims 1 to 12, wherein the gene sequence is a gene sequence encoding a protein whose intracellular expression is detectable.

14. A measurement kit for homologous recombination activity, the kit comprising the measurement reagent according to claim 13.

15. A screening system for an agent that affects homologous recombination activity, the screening system comprising the nucleic acid construct according to any one of claims 1 to 12.

16. A diagnostic agent for homologous recombination-deficient cancer, the agent comprising the nucleic acid construct according to any one of claims 1 to 12.

17. A detection agent for homologous recombination-restored cancer cells, the agent comprising the nucleic acid construct according to any one of claims 1 to 12, wherein the gene sequence is a gene sequence encoding a protein whose intracellular expression is detectable.

18. The detection agent according to claim 17, wherein the homologous recombination-restored cancer cells are PARP inhibitor-resistant cancer cells.

19. A companion diagnostic agent for predicting an effect of an anticancer drug on homologous recombination-deficient cancer, the agent comprising the nucleic acid construct according to any one of claims 1 to 12.

20. The companion diagnostic agent according to claim 19, wherein the anticancer drug is a DNA-damaging anticancer drug.

21. The companion diagnostic agent according to claim 20, wherein the DNA-damaging anticancer drug is a PARP inhibitor.

22. A therapeutic agent for homologous recombination-restored cancer, the agent comprising the nucleic acid construct according to any one of claims 1 to 12, wherein the gene sequence is a gene sequence encoding a protein that acts to decrease cell survival rate.

23. The therapeutic agent according to claim 22, wherein the homologous recombination-restored cancer is PARP inhibitor-resistant cancer.

24. A method of measuring homologous recombination activity in test cells, the method comprising:
introducing a nucleic acid construct according to any one of claims 1 to 12, wherein the gene sequence is a gene sequence encoding a protein whose intracellular expression is detectable, into test cells whose homologous recombination activity is to be measured, and into homologous recombination-proficient control cells having normal homologous recombination activity;
measuring the expression levels of the protein in the test cells and the homologous recombination-proficient control cells; and
comparing the expression level in the test cells with the expression level in the homologous recombination-proficient control cells;
wherein a protein expression in the test cells that is lower than the protein expression in the homologous recombination-proficient control cells indicates that the test cells have a lower homologous recombination activity.

25. A method of identifying a candidate of an agent that affects homologous recombination activity, the method comprising:
introducing the nucleic acid construct according to any one of claims 1 to 12 into cells having normal homologous recombination activity, and then treating the cells with each compound in a compound group; or treating cells having normal homologous recombination activity with each compound in a compound group, and then introducing the nucleic acid construct according to any one of claims 1 to 12 into the cells; and
measuring expression of the protein.

26. The method according to claim 25, wherein the protein is a protein whose intracellular expression is detectable as a signal, the method comprising selecting a compound as a candidate of an inhibitor that inhibits homologous recombination activity when the signal of the protein detected is lower in the cells treated with the compound than in the cells not treated with the compound, or selecting a compound as a candidate of an enhancer that promotes homologous recombination activity when the signal of the protein rises more rapidly or a higher signal is detected in the cells treated with the compound compared to the cells not treated with the compound.

27. The method according to claim 25, wherein the protein is a protein that acts to decrease cell survival rate, the method comprising selecting a compound as a candidate of an inhibitor that inhibits homologous recombination activity when the decrease in the cell survival rate is suppressed in the cells treated with the compound compared to the cells not treated with the compound, or selecting a compound as a candidate of an enhancer that promotes homologous recombination activity when the decrease in the cell survival rate occurs earlier in the cells treated with the compound than in the cells not treated with the compound.

28. A diagnostic method for homologous recombination-deficient cancer, the method comprising:
introducing the nucleic acid construct according to any one of claims 1 to 12 into cancer cells of a cancer patient; and
measuring expression of the protein.

29. The method according to claim 28, wherein the cancer cells are cells separated from the cancer patient, and the introduction of the nucleic acid construct into the cancer cells is carried out in vitro.

30. The method according to claim 28, wherein: the protein is a protein whose intracellular expression is detectable as a signal; the introduction of the nucleic acid construct into the cancer cells is carried out by administering the nucleic acid construct to the cancer patient; and whether or not the signal of the protein is detected from a cancer lesion is investigated.

31. The method according to claim 28, wherein: the protein is a secretory protein whose intracellular expression is detectable; the introduction of the nucleic acid construct into the cancer cells is carried out by administering the nucleic acid construct to the cancer patient; and the activity of the protein in blood separated from the patient after the administration of the nucleic acid construct is measured.

32. A detection method for homologous recombination-restored cancer cells, the method comprising:
introducing the nucleic acid construct according to any one of claims 1 to 12 into cancer cells of a cancer patient that is a patient under treatment with a PARP inhibitor or that is a patient who was once diagnosed with homologous recombination-deficient cancer; and
measuring expression of the protein.

33. The method according to claim 32, wherein the cancer cells are cells separated from the patient, and the introduction of the nucleic acid construct into the cancer cells is carried out in vitro.

34. The method according to claim 32, wherein: the protein is a protein whose intracellular expression is detectable as a signal; the introduction of the nucleic acid construct into the cancer cells is carried out by administering the nucleic acid construct to the patient; and whether or not the signal of the protein is detected from a cancer lesion is investigated.

35. A method of predicting an effect of an anticancer drug on homologous recombination-deficient cancer, the method comprising:
introducing the nucleic acid construct according to any one of claims 1 to 12 into cancer cells of a cancer patient; and
measuring expression of the protein.

36. The method according to claim 35, wherein the anticancer drug is a DNA-damaging anticancer drug.

37. The method according to claim 36, wherein the DNA-damaging anticancer drug is a PARP inhibitor.

38. The method according to claim 35, wherein the cancer cells are cells separated from the cancer patient, and the introduction of the nucleic acid construct into the cancer cells is carried out in vitro.

39. The method according to claim 35, wherein: the protein is a protein whose intracellular expression is detectable as a signal; the introduction of the nucleic acid construct into the cancer cells is carried out by administering the nucleic acid construct to the cancer patient; and whether or not the signal of the protein is detected from a cancer lesion is investigated.

40. The method according to claim 35, wherein: the protein is a secretory protein whose intracellular expression is detectable; the introduction of the nucleic acid construct into the cancer cells is carried out by administering the nucleic acid construct to the cancer patient; and activity of the protein in blood separated from the patient after the administration of the nucleic acid construct is measured.

41. A therapeutic method for homologous recombination-restored cancer, the method comprising administering a nucleic acid construct according to any one of claims 1 to 12, wherein the gene sequence is a gene sequence encoding a protein that acts to decrease cell survival rate, to a patient having the homologous recombination-restored cancer.

42. The method according to claim 41, wherein the nucleic acid construct is topically administered into a tumor or into the vicinity of a tumor of the patient.

43. The method according to claim 41, wherein the homologous recombination-restored cancer is PARP inhibitor-resistant cancer.

44. A method of predicting whether or not a gene mutation identified in a cancer patient is a pathogenic mutation that deteriorates homologous recombination activity, the method comprising:
constructing an expression vector that expresses a mutant gene having the same mutation as the previously-mentioned mutation;
providing an expression vector that expresses a wild-type gene not having the mutation;
introducing each of the mutant-gene expression vector and the wild-type-gene expression vector, and also introducing the nucleic acid construct according to any one of claims 1 to 12, into cells deficient in the gene; and
measuring expression of the protein in the cells in which each expression vector and the nucleic acid construct are introduced;
wherein the mutation is indicated to be a pathogenic mutation that deteriorates homologous recombination activity when the expression level of the protein in the cells in which the mutant-gene expression vector is introduced is lower than the expression level of the protein in the cells in which the wild-type-gene expression vector is introduced.
